# EUROPEAN PATENT APPLICATION

(11) **EP 2 141 164 A1**
(43) Date of publication of application: **06.01.2010**
(21) Application number: 08290639.7
(22) Date of filing: 01.07.2008
(51) Int. Cl.: C07D 417/12, A61K 31/53, A61P 31/00

(54) **New 1,2,4-triazine derivatives and biological applications thereof**

(71) Applicant: Mutabilis, 93230 Romainville (FR)
(72) Inventor: Oliveira, Chrystelle, 95120 Ermont (FR); Vongsouthi, Vanida, 75014 Paris (FR); Moreau, François, 91400 Orsay (FR); Denis, Alexis, 75011 Paris (FR); Escaich, Sonia, 75012 Paris (FR); Gerusz, Vincent, 75017 Paris (FR); Desroy, Nicolas, 92330 Sceaux (FR)
(74) Representative: Peaucelle, Chantal

(57) **Abstract**

The invention relates to new 1,2,4-triazine derivatives of formula (I): 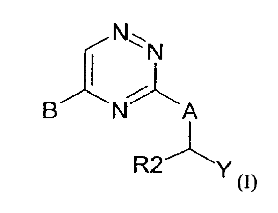 wherein A, B, R2 and Y are defined in the application,
their preparation and intermediates, their use as drugs and pharmaceutical compositions and associations containing them.

The compounds of formula (I) are capable of inhibiting bacterial heptose synthesis.

## Description

The invention relates to new 1,2,4-triazine derivatives, their preparation and intermediates, their use as drugs and pharmaceutical compositions containing them.

The invention relates to new compounds capable of inhibiting bacterial heptose synthesis.

The lipopolysaccharide (LPS) is a major component of the outer membrane of Gram-negative bacteria. It is composed of three regions: the lipid A, the core oligosaccharide and the O antigen. The core oligosaccharide is divided into the inner core and the outer core. The inner core consists in a motif of five sugars: two Kdo (Kdo: 3-deoxy-D-manno-octulosonic acid) and three successive heptoses.

The first heptose transfer is catalysed by the Heptosyltransferase I (protein WaaC) and the second heptose transfer by the Heptosyltransferase II (protein WaaF).

The natural donor substrate of these transferases is ADP heptose, which is synthesized in bacteria from sedoheptulose by the successive enzymatic steps catalyzed by the following enzymes: GmhA, RfaE, GmhB,and RfaD (WaaD) (Journal of Bacteriology, Jan.2002, p 363-369).

Heptose synthetic pathway is conserved among Gram negative bacterial species and is necessary for full LPS synthesis. It has been demonstrated that a complete LPS is necessary for pathogenesis due to the Gram negative bacteria. Bacteria lacking heptoses do have a rough phenotype because of the absence of the carbohydrate chains of the inner and outer core LPS. Bacteria having this phenotype are unable to give a productive infection in the host and in particular are very sensitive to the bactericidal effect of complement.

Gram-negative bacteria that lack heptose display the deep-rough phenotype and show a reduction in outer membrane protein content, an increased sensitivity towards detergents or hydrophobic antibiotics and are more susceptible to phago-cytosis by macrophages. In addition, deep-rough mutated bacteria have been shown to present an attenuated virulence in an infant rat model and it has been demonstrated that the inner core LPS which constitutes the outer leaflet of Gram-negative bacteria outer membrane is necessary to protect *Escherichia coli* from complement killing in vitro and for virulence in vivo but is dispensable for gut colonization.

Compounds inhibiting heptose synthesis activity are expected to prevent full LPS synthesis in Gram negative bacteria, inducing a high sensitivity to the complement and inhibiting bacterial dissemination in blood.

Therefore small molecules inhibitors of heptose synthesis could be a new way to treat bloodstream infections caused by pathogenic Gram negative bacteria.

It is known that the reactions catalyzed by RfaE are essential for heptose synthesis. As shown in WO 2006/058 796, this enzyme is essential for pathogenicity in an experimental model of infection.

The invention relates to compounds having the general formula (I) 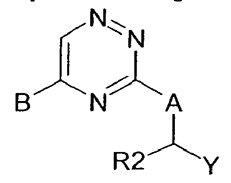 wherein,
- A is NR1, O or S;
R1 is H or (C₁-C₆) alkyl optionally substituted by one group R'1, or R1 and R2 form with N a 4 to 6 membered saturated heterocycle;
R'1 is CH₂OH, CO₂H, CO₂ (C₁-C₄) alkyl, CH₂NH₂, SO₃H or PO₃H₂;
- R2 is H or (C₁-C₆) alkyl optionally substituted by one or several identical or different R or R1 and R2 form with N a 4 to 6 membered saturated heterocycle;
- Y is a 5-10 membered mono or bicyclic, unsaturated or aromatic heterocycle containing 1-4 heteroatoms selected from N, O and S, optionally substituted by one or several identical or different groups R;
- B is (C₁-C₆) alkyl or mono or bicyclic aryl, all being optionally substituted by one or several identical or different groups R;
R is selected from the group consisting of (C₁-C₆) alkyl, (C₂-C₆) alkenyl, (C₂-C₆) alkynyl, phenyl, 4-6 membered monocyclic saturated or unsaturated heterocycle containing 1-3 heteroatoms selected from N, O and S, CO₂Rₐ, CORₐ, CONRₐR_{b}, OCORₐ, ORₐ, NRₐR_{b}, CRₐ=NOR_{b}, NRₐCOR_{b}, NRₐCOOR_{b}, OCONRₐR_{b}, NRₐCONR_{b}R, NRₐSO₂R_{b}, S(O)ₙRₐ, and SO₂NRₐR_{b}, all the above members of the group representing R being optionally substituted by one or several identical or different groups R', or R is halogen, CN or NO₂;
Rₐ, R_{b} and R_{c}, identical or different, are selected from the group consisting of H, (C₁-C₆) alkyl, (C₂-C₆) alkenyl, (C₂-C₆) alkynyl, phenyl and 4-6 membered monocyclic saturated or unsaturated heterocycle containing 1-3 heteroatoms selected from N, O and S, all the above members of the group representing Rₐ, R_{b} and R_{c} being optionally substituted by one or different groups R';
R' is selected from the group consisting of (C₁-C₆) alkyl, (C₁-C₆) alkyl substituted by hydroxy, 4-6 membered monocyclic saturated or unsaturated heterocycle containing 1-3 heteroatoms selected from N, O and S, CO₂R", COR", CONR"R"', OCOR", OR", NR"R"', NR"COR"', NR"COOR"', CONR"-(C₁-C₃)alkyl-OH, OCONR"R"', NR"CONR"'R"", NR"SO₂R"', S(O)ₙR", SO₂ NR"R"', halogen, CN, NR"-CO(C₁-C₃) alkyl-NR"'R"", NR"-CO(C₁-C₃) alkyl-heterocycle, NR"-CO(C₁-C₃) alkyl-OH, NR"-CO(C₁-C₃) alkyl-CONH₂, -CONR"-(C₁-C₃) alkyl-NR"'R"", -CONR"-(C₁-C₃) alkyl-CONH₂, -CO-NR"-(C₁-C₃) alkyl-heterocycle, the heterocyle being selected from the group consisting of piperidinyl, imidazolyl, morpholinyl, piperazinyl, pyrrolidinyl and azetidinyl;
R", R"' and R"" being identical or different are H or (C₁-C₅) alkyl or form together a 4 to 6 membered nitrogenous saturated heterocycle;
n is 0, 1 or 2;
their N-oxide derivatives,
in their racemic, enantiomeric or geometric forms,
and their addition salts thereof with acids and bases.

Among the acid salts of the products of formula (I), there may be cited, among others, those formed with mineral acids, such as hydrochloric, hydrobromic, hydro-iodic, sulfuric or phosphoric acid or with organic acids such as formic, acetic, trifluoroacetic, propionic, benzoic, maleic, fumaric, succinic, tartaric, citric, oxalic, glyoxylic, aspartic, alkanesulfonic acids, such as methanesulfonic and ethanesulfonic acids, arylsulfonic acids such as benzenesulfonic and paratoluenesulfonic acids.

Among the alkaline salts of the products of formula (I), there may be cited, among others, those formed with mineral alkalis such as, for example, sodium, potassium, lithium, calcium, magnesium or ammonium or organic bases such as, for example, methylamine, ethylamine, propylamine, trimethylamine, diethylamine, triethylamine, N,N-dimethylethanolamine, tris(hydroxymethyl)aminomethane, ethanolamine, pyridine, piperidine, piperazine, picoline, dicyclohexylamine, morpholine, benzylamine, procaine, lysine, arginine, histidine, N-methylglucamine.

In the general formula (I), as applied herein:
"C₁-C₆ alkyl" means any linear, branched or cyclic hydrocarbon groups having 1 to 6 carbon atoms, preferably methyl, ethyl, n-propyl, isopropyl, n-butyl, isobutyl and t-butyl, n-pentyl, isopentyl, neopentyl, cyclopropyl, cyclobutyl, cyclopentyl, cyclohexyl;
"C₂-C₆ alkenyl and "C₂-C₆ alkynyl" as applied herein means any linear, branched or cyclic hydrocarbon groups of 2 to 6 carbon atoms, having at least one double bond or one triple bond and preferably ethenyl, propenyl, butenyl, cyclohexenyl, ethynyl, propargyl, butynyl;
"Halogen" means F, Cl, Br, and I;

Illustrative heterocycles as mentioned in the definitions of formula I are for example those selected from the group comprising furyl, tetrahydrofuryl, benzofuryl, tetrahydrobenzofuryl, thienyl, tetrahydrothienyl, benzothienyl, tetrahydrobenzothienyl, pyrrolyl, pyrrolidinyl, indolyl, indolinyl, tetrahydroindolyl, oxazolyl, oxazolinyl, oxazolidinyl, benzoxazolyl, tetrahydrobenzoxazolyl, oxazolopyridinyl, tetrahydrooxazolopyridinyl, oxazolopyrimidinyl, tetrahydrooxazolopyrimidinyl, oxazolopyrazinyl, oxazolopyridazinyl, oxazolotriazinyl, isoxazolyl, benzoisoxazolyl, tetrahydrobenzoisoxazolyl, thiazolyl, thiazolinyl, thiazolidinyl, benzothiazolyl, tetrahydrobenzothiazolyl, thiazolopyridinyl, tetrahydrothiazolopyridinyl, thiazolopyrimidinyl, tetrahydrothiazolopyrimidinyl, thiazolopyrazinyl, thiazolopyridazinyl, thiazolotriazinyl, isothiazolyl, benzoisothiazolyl, tetrahydrobenzoisothiazolyl, imidazolyl, benzimidazolyl, tetrahydrobenzimidazolyl, pyrazolyl, indazolyl, tetrahydroindazolyl, triazolyl, oxadiazolyl, thiadiazolyl, tetrazolyl, pyranyl, dihydropyranyl, tetrahydropyranyl, benzopyranyl, dioxanyl, benzodioxanyl, dioxolanyl, benzodioxolanyl, pyridinyl, pyridonyl, piperidinyl, tetrahydropyridinyl, quinolinyl, isoquinolinyl, tetra- and perbydro-quinolinyl and isoquinolinyl, pyrimidinyl, quinazolinyl, pyrazinyl, pyrazidinyl, piperazinyl, quinoxalinyl, piridazinyl, cinnolinyl, phtalazinyl, triazinyl, purinyl, pyrazolopyridinyl, tetrahydropyrazolopyridnyl, pyrazolopyrimidinyl, pyrazolopyrazinyl, pyrazolotriazinyl, triazolopyridinyl, tetrahydrotriazolopyridinyl, triazolopyrimidinyl, triazolopyrazinyl, triazolotriazinyl, oxetanyl, azetidinyl, morpholinyl.

Illustrative nitrogenous heterocycles are for example those comprised in the above group.

As stated above, the invention includes any N-oxide derivatives of the compounds of formula (I).

According to the invention, the compounds of formula (I) can be in racemic forms, as well as in the form of pure enantiomers or non racemic (scalemic) mixture of enantiomers, including when the compounds of formula (I) have more than one stereogenic center. In case the compounds of formula (I) have unsaturated carbon carbon double bonds, both the cis (Z) and trans (E) isomers and mixtures belong to the invention.

Preferred total number of substitutions by R or R' for any substituted (C₁-C₆) alkyl, (C₂-C₆) alkenyl, (C₂-C₆) alkynyl, aryl, heterocycle or Rₐ, R_{b} and R_{c}, is from 1 to 5.

Preferred compounds according to the invention includes:
the compounds of the general formula (I) wherein B represents phenyl substituted at position 2 by a group R, or 1-naphthyl optionally substituted by R, R being as defined above;
the compounds of the general formula (I) wherein B represents phenyl substituted at positions 2,5, 2,6 and 2,4,6 by identical or different groups R, R being as defined above;
the compounds of the general formula (I) wherein B represents phenyl substituted at positions 2,6 by identical or different groups R, R being selected from the group consisting of Me, halogen, NH₂, OH and OME;
the compounds of general formula (I) wherein the heterocycle represented by Y is selected from the group consisting of oxazolyl, oxazolinyl, benzoxazolyl, tetrahydrobenzoxazolyl, oxazolopyridinyl, tetrahydrooxazolopyridinyl, oxazolopyrimidinyl, tetrahydrooxazolopyrimidinyl, oxazolopyrazinyl, oxazolopyridazinyl, oxazolotriazinyl, thiazolyl, thiazolinyl, benzothiazolyl, tetrahydrobenzothiazolyl, thiazolopyridinyl, tetrahydrothiazolopyridinyl, thiazolopyrimidinyl, tetrahydrothiazolopyrimidinyl, thiazolopyrazinyl, thiazolopyridazinyl, thiazolotriazinyl, pyrazolyl, pyrazolopyridinyl, tetrahydropyrazolopyridnyl, pyrazolopyrimidinyl, pyrazolopyrazinyl, pyrazolotriazinyl, triazolopyridinyl, tetrahydrotriazolopyridinyl, triazolopyrimidinyl, triazolopyrazinyl and triazolotriazinyl,
and in particular:
the compounds of general formula (I) wherein Y represents a benzothiazole group, a tetrahydrobenzothiazole group, or a thiazolopyridine group, optionally substituted at position 5 by a group R, R being as defined above;
the compounds of general formula (I) wherein Y represents a benzothiazole group, a tetrahydrobenzothiazole group, or a thiazolopyridine group, optionally substituted at position 5 by a group ORa, Ra being as defined above;
the compounds of general formula (1) wherein Y represents a thiazole group, optionally substituted at position 4 and/or 5 by one or two groups R, R being as defined above;
the compounds of general formula (I) wherein A is NH or N-CH₃;
the compounds of general formula (I) wherein R2 is H;
and their salts with acids or bases.

Preferred compounds according to the invention includes the compounds of examples 9, 15, 16, 17, 18, 19, 20, 21, 22, 29, 32, 33, 35, 43, 57, 64, 70, 75, 78, 90, 94, 97, 98, 99, 100, 107 and 108, the names of which are listed below:
- 5-(2-Chlorophenyl)-N-[(4,5-dimethyl-1,3-thiazol-2-yl)methyl]-1,2,4-triazin-3-amine,
- 5-(2,6-Dichlorophenyl)-N-[(5-methoxy-1,3-benzothiazol-2-yl)methyl]-1,2,4-triazin-3-amine,
- 2-{[2-({[5-(2,6-Dichlorophenyl)-1,2,4-triazin-3-yl]amino}methyl)-1,3-benzothiazol-5-yl]oxy}ethanol,
- {[2-({[5-(2,6-dichlorophenyl)-1,2,4-triazin-3-yl]amino}methyl)-1,3-benzothiazol-5-yl]oxy}acetic acid,
- 2-(2-{[5-(2,6-Dichloro-phenyl)-[1,2,4]triazin-3-ylamino]-methyl}-benzothiazol-5-yloxy)-N-(2-hydroxy-ethyl)-acetamide,
- 2-(2-{[5-(2,6-Dichloro-phenyl)-[1,2,4]triazin-3-ylamino]-methyl}-benzothiazol-5-yloxy)-N-(2-dimethylamino-ethyl)-acetamide,
- 1-{[2-({[5-(2,6-Dichlorophenyl)-1,2,4-triazin-3-yl]amino}methyl)-1,3-benzothiazol-5-yl]oxy}acetone,
- 2-{[2-({[5-(2,6-Dichlorophenyl)-1,2,4-triazin-3-yl]amino}methyl)-1,3-benzothiazol-5-yl]oxy}acetamide,
- Benzothiazol-2-ylmethyl-[5-(2-methoxy-naphthalen-1-yl)-[1,2,4]triazin-3-yl]-amine,
- N-[(5-Methoxy-1,3-benzothiazol-2-yl)methyl]-5-(2-methoxy-1-naphthyl)-1,2,4-triazin-3-amine,
- 1-{3-[(Benzothiazol-2-ylmethyl)-amino]-[1,2,4]triazin-5-yl}-naphthalen-2-ol,
- 2-{[5-(2-Hydroxy-naphthalen-1-yl)-[1,2,4]triazin-3-ylamino]-methyl}-benzothiazol-5-ol,
- 2-(3-{[(5-Methoxy-1,3-benzothiazol-2-yl)methyl]amino}-1,2,4-triazin-5-yl)-3-methylphenol,
- 3-Methyl-2-{3-[({5-[(1,3-thiazol-2-ylmethyl)amino]-1,3-benzothiazol-2-yl}methyl)amino]-1,2,4-triazin-5-yl}phenol,
- 5-(2,6-Dimethoxyphenyl)-N-(4,5,6,7-tetrahydro-1,3-benzothiazol-2-ylmethyl)-1,2,4-triazin-3-amine,
- 2-[({5-[2-(Trifluoromethyl)phenyl]-1,2,4-triazin-3-yl}amino)methyl]-1,3-benzothiazol-4-ol,
- 5-(2-Methoxy-6-methylphenyl)-N-([1,3]thiazolo[5,4-b]pyridin-2-ylmethyl)-1,2,4-triazin-3-amine,
- 2-Dimethylamino-N-(4-{3-[(5-methoxy-benzothiazol-2-ylmethyl)-amino]-[1,2,4]triazin-5-yl}-3,5-dimethyl-phenyl)-acetamide,
- N,N-Dimethyl-N'-[4-(3-{[(5-methoxy-1,3-benzothiazol-2-yl)methyl]amino}-1,2,4-triazin-5-yl)phenyl]ethane-1,2-diamine,
- {(1,3-Benzothiazol-2-ylmethyl)[5-(2,6-dimethoxyphenyl)-1,2,4-triazin-3-yl]amino}acetic acid,
- N-({5-[(2-Aminoethyl)thio]-1,3-benzothiazol-2-yl}methyl)-5-(2,6-dichlorophenyl)-1,2,4-tri azin-3-amine,
- 5-[2-Chloro-5-(1H-pyrazol-3-yl)phenyl]-N-[(5-methoxy-1,3-benzothiazol-2-yl)methyl]-1,2,4-triazin-3-amine,
- 5-[2-Chloro-5-(1H-pyrazol-4-yl)phenyl]-N-[(5-methoxy-1,3-benzothiazol-2-yl)methyl]-1,2,4-triazin-3-amine,
- 5-(2-Chloro-5-pyridin-3-ylphenyl)-N-[(5-methoxy-1,3-benzothiazol-2-yl)methyl]-1,2,4-triazin-3-amine,
- 2-[4-(4-Chloro-3-{3-[(5-methoxy-benzothiazol-2-ylmethyl)-amino]-[1,2,4]triazin-5-yl}-phenyl)-[1,2,3]triazol-1-yl]-ethanol,
- N-[(5-Methoxy-1,3-benzothiazol-2-yl)methyl]-5-(2-chloro-4-piperazin-1-ylphenyl)-1,2,4-triazin-3-amine, and
- N-[4-(3-{[5-(2-Amino-ethoxy)-benzothiazol-2-ylmethyl]-amino}-[1,2,4]triazin-5-yl)-3,5-dimethyl-phenyl]-N',N'-dimethyl-ethane-1,2-diamine,
and their salts.

The compounds of formula I and their salts may be prepared by processes known to the skilled chemist to be applicable for preparing chemically related compounds. Such processes use known starting materials or intermediates which may be obtained by standard procedures of organic chemistry. The following processes provide a variety of non-limiting routes for the production of the compounds of formula I and their intermediates. These processes constitute further features of the present invention.

The invention also relates to a process for preparing the compounds of formula (I) as defined above, which comprises:
a) reacting a compound of formula (II): 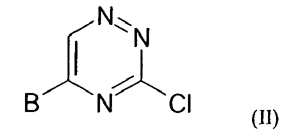 wherein B has values defined above, with a compound of formula (III):

   H-A-CH(R2)-Y (III)

   wherein A, R2 and Y have the values defined above, in the presence of a base,
b) if appropriate, the reaction being followed or preceded by one or more of the following reactions, performed in an appropriate order, to achieve the substitutions on B and/or Y and/or R1 and/or R2 defined above:
   protection of reactive functions,
   deprotection of reactive functions,
   halogenation,
   dehalogenation,
   dealkylation,
   alkylation of amine, aniline, alcohol and phenol,
   reduction of nitro, esters, cyano, aldehydes,
   transition metal-catalyzed reactions,
   acylation,
   sulfonylation/introduction of sulfonyl groups,
   saponification/hydrolysis of esters groups,
   halogen exchange,
   nucleophilic substitution with amine, thiol or alcohol,
   reductive amination,
   fixation of R groups on B and/or Y,
   N-oxidation,
   salification.

The base used in the nucleophilic substitution on the 3-chloro triazine can be an amine, preferably a tertiary amine, such as triethylamine, diisopropylethylamine pyridine or dimethylaminopyridine, an alkali metal hydride such as sodium hydride, or carbonate such as sodium or potassium carbonate.

The reaction is performed in an organic solvent which can be for example acetonitrile, tetrahydrofuran or dimethylformamide, acetonitrile being preferred, at a temperature between room temperature and the reflux of the reaction medium.

The invention also relates to a process for preparing the compounds of formula (I) as defined above, wherein A represents NH, which comprises:
a) reacting a compound of formula (IV): 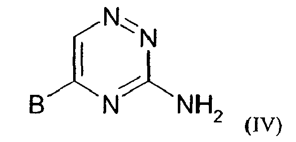 wherein B has values defined above, with a compound of formula (V):

   R2-C(O)-Y (V)

   wherein Y and R2 have the values defined above, in the presence of a reducing agent,
b) if appropriate, the reaction being followed or preceded by one or more of the reactions defined above, performed in an appropriate order, to achieve the substitutions on B and/or Y and/or R1 and/or R2 defined above.

The reducing agent used in the above reductive amination can be, for example, sodium borohydride, cyanoborohydride or triacetoxyborohydride.

The reaction can be performed in a solvent adapted to the reducing agent, this being well known to the skilled chemist.

The invention also relates to a process for preparing the compounds of formula (I) as defined above, wherein A represents NH, which comprises:
a) reacting a compound of formula (VI): 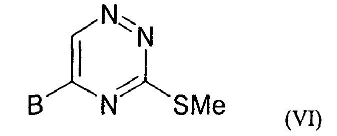 wherein B has values defined above, with a compound of formula (VII):

   H₂N-CH(R2)-Y (VII)

   wherein R2 and Y have the values defined above, in the presence of an oxidizing agent and a base,
b) if appropriate, the reaction being followed or preceded by one or more of the reactions defined above, performed in an appropriate order, to achieve the substitutions on B and/or Y and/or R1 and/or R2 defined above.

The conditions for performing the above various reactions referred to in steps b) above are all well known to the skilled and several examples are illustrated below in the experimental part.

The invention also relates to a process for preparing the compounds of formula (II) as defined above, which comprises reacting a compound of formula (IV) as defined above, with a chlorination agent.

Preferably, the chlorination agent is copper II chloride, used in the presence of tert-butyl nitrite.

The compounds of formula (II) can also be prepared by a process which comprises reacting a compound of formula (VIII):

B-CO-CHO (VIII)

wherein B has values defined above, with semicarbazide of formula (IX):

H₂N-CO-NH-NH₂ (IX)

to obtain a compound of formula (X): 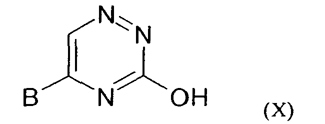 which is submitted to the action of a chlorinating agent.

In preferred conditions, the chlorination agent is phosphorous oxychloride. Phosphorous pentachloride, oxalyl chloride or thionyl chloride can also be used.

The compounds of formula (II), and intermediately (IV), can be prepared by a process which comprises reacting a compound of formula (VIII):

B-CO-CHO (VIII)

wherein B has values defined above, with aminoguanidine of formula (XI):

H₂N-C(NH)-NH-NH₂ (XI)

to obtain a compound of formula (IV): 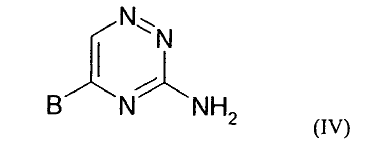 which is, if desired, either submitted to the action of an alkali metal hydroxide to obtain a compound of formula (X), which is submitted to the action of a chlorination agent as above, or directly submitted to the action of a chlorination agent as above.

The invention also relates to another process for preparing the compounds of formula (IV) as defined above, which comprises reacting a compound of formula (XI) as defined above, with a compound of formula (XII):

B-CO-C(morpholine)₂ (XII)

wherein B has values defined above, in an acidic medium.

Preferably,the reaction is performed by using acetic acid, in an alcohol like methanol or ethanol as described in the literature (Organic Letters, 2003, 2271-2274).

The compound of formula (XII) can itself be obtained by reacting a compound of formula (XIII):

B-CO-CHBr₂ (XIII)

with morpholine

The above compounds of formulae (II), (IV), (VI), (VIII) (X), (XII) and (XIII) are novel compounds and constitute a further object of the invention.

The compounds of formula (III) and (V) can be prepared starting from commercial compounds, by various processes illustrated below and in the experimental part. These processes are all described in the literature and well known to the skilled chemist.

The compounds of formula (III) wherein A is NH and R2 is hydrogen can be prepared from a compound of formula (XIV):

Y-CH₃ (XIV)

which are treated by selenium dioxide in a mixture of dioxane and water (an alcanol or tetrahydrofuran can also be used) to obtain a compound of formula (V) defined above, wherein R2 is hydrogen, which is treated by hydroxylamine in an alcohol, to obtain a compound of formula (XV):

Y-CH=NOH (XV)

which is reduced, for example by zinc in acetic acid.

The compounds of formula (III) wherein A is NR1, R1 being different from hydrogen can be prepared from a compound of formula (V) which is submitted to a reductive amination, by using an appropriate amine of formula R1-NH₂, for example in the presence of NaBH₃CN or NaBH₄.

They can also be obtained from a compound of formula (III) wherein A is NH, which is treated by a compound of formula R1-Hal.

The compounds of formula (III) wherein A is NR1 and R2 is hydrogen can also be obtained from a compound of formula(XVI):

Y-CH₂Hal (XVI)

Hal being preferably Br or Cl, which is treated by an amine NH₂R1.

The compounds of formula (III) can also be obtained from a compound of formula (XVII):

Y-H (XVII)

which is treated first by an alkyl lithium such as n-butyl lithium, and then by an amide of formula (XVIII):

R2-CONMe₂ (XVIII)

the reactive functions on R2 being eventually protected, to obtain a compound of formula (V) defined above, which is treated by hydroxylamine to obtain a compound of formula (XIX):

Y-C(R2)=NOH (XIX)

which is reduced, for example by zinc in acetic acid.

Compounds of formula (III) wherein R1 and R2 form a cycle are commercial, in particular when Y is a benzothiazole. Methods are available in the literature to prepare compounds wherein the heterocycle is different.

The compounds of formula (III) wherein A is O can be obtained from a compound of formula (V) which is reduced by using known methods, to obtain the corresponding alcohol.

The compounds of formula (III) wherein A is S can be obtained from a compound of formula (III) wherein A is O, by using a Mitsunobu reaction.

Conditions for performing the above various reactions referred to in steps b) above applied to the compounds of formula (III) are illustrated below in the experimental part, attention being drawn in particular to those used in example 108.

The compounds of formula (III) wherein A is NH, R2 is H and Y is benzothiazole possibly substituted can also be obtained by forming the thiazole ring starting from a possibly substituted 2-bromo or 2-iodo aniline which is treated by a compound of formula (XX):

H₂N-C(S)-CH₂-NH-COOalkyl (XX)

in the presence of a palladium complex and calcium (II) oxide, in acetonitrile, to obtain the corresponding compound of formula (XXI):

Y-CH₂-NH-COOalkyl (XXI)

Y being a possibly substituted benzothiazole, which is treated in acidic conditions. Illustration is given below in the experimental part.

The above process can also be used to prepare compounds of formula (III) wherein Y is different from benzothiazole, by using instead of an aniline an α bromo or iodo amino-heteroaromatic compound. The heteroaromatic ring is preferably a pyridine ring.

The above compounds of formula (III) wherein Y is substituted by one or several identical or different groups R are novel compounds and constitute a further object of the invention.

As said above, the new compounds of formula (I) are capable of inhibiting bacterial heptose synthesis which makes them useful as drugs for preventing or treating bacterial infections and another object of the invention is the use of the compounds of formula (I) as drugs.

The drugs of the invention are especially useful for the prevention and therapeutical treatment of severe infections due to Gram negative bacteria able to dissiminate in blood such as the following species (spp.): *Escherichia coli, Enterobacter, Salmonella, Shigella, Pseudomonas, Acinetobacter, Neisseria, Klebsiella, Serratia, Citrobacter, Proteus, Yersinia, Haemophilus, Legionella, Moraxella* and *Helicobacter pylori.*

A further object of the present invention is therefore the use, as drugs and in particular antibiotic drugs, of the compounds of formula (I) as defined above, as well as their salts with pharmaceutically acceptable acids and bases.

More particularly, an object of the invention is the use, as drugs, of the preferred compounds of formula (I) mentioned above, and in particular of the compounds listed below:
-5-(2-Chlorophenyl)-N-[(4,5-dimethyl-1,3-thiazol-2-yl)methyl]-1,2,4-triazin-3-amine,
-5-(2,6-Dichlorophenyl)-N-[(5-methoxy-1,3-benzothiazol-2-yl)methyl]-1,2,4-triazin-3-amine,
-2-{[2-({[5-(2,6-Dichlorophenyl)-1,2,4-triazin-3-yl]amino}methyl)-1,3-benzothiazol-5-yl]oxy}ethanol,
-{[2-({[5-(2,6-dichlorophenyl)-1,2,4-triazin-3-yl]amino}methyl)-1,3-benzothiazol-5-yl]oxy}acetic acid,
-2-(2-{[5-(2,6-Dichloro-phenyl)-[1,2,4]triazin-3-ylamino]-methyl}-benzothiazol-5-yloxy)-N-(2-hydroxy-ethyl)-acetamide,
-2-(2-{[5-(2,6-Dichloro-phenyl)-[1,2,4]triazin-3-ylamino]-methyl}-benzothiazol-5-yloxy)-N-(2-dimethylamino-ethyl)-acetamide,
-1-{[2-({[5-(2,6-Dichlorophenyl)-1,2,4-triazin-3-yl]amino}methyl)-1,3-benzothiazol-5-yl]oxy}acetone,
-2-{[2-({[5-(2,6-Dichlorophenyl)-1,2,4-triazin-3-yl]amino}methyl)-1,3-benzothiazol-5-yl]oxy}acetamide,
-Benzothiazol-2-ylmethyl-[5-(2-methoxy-naphthalen-1-yl)-[1,2,4]triazin-3-yl]-amine,
-N-[(5-Methoxy-1,3-benzothiazol-2-yl)methyl]-5-(2-methoxy-1-naphthyl)-1,2,4-triazin-3-amine,
-1-{3-[(Benzothiazol-2-ylmethyl)-amino]-[1,2,4]triazin-5-yl}-naphthalen-2-ol,
-2-{[5-(2-Hydroxy-naphthalen-1-yl)-[1,2,4]triazin-3-ylamino]-methy}benzothiazol-5-ol,
-2-(3-{[(5-Methoxy-1,3-benzothiazol-2-yl)methyl]amino}-1,2,4-triazin-5-yl)-3-methylphenol,
-3-Methyl-2-{3-[({5-[(1,3-thiazol-2-ylmethyl)amino]-1,3-benzothiazol-2-yl}methyl)amino]-1,2,4-triazin-5-yl}phenol,
-5-(2,6-Dimethoxyphenyl)-N-(4,5,6,7-tetrahydro-1,3-benzothiazol-2-ylmethyl)-1,2,4-triazin-3-amine,
-2-[({5-[2-(Trifluoromethyl)phenyl]-1,2,4-triazin-3-yl}amino)methyl]-1,3-benzothiazol-4-ol,
-5-(2-Methoxy-6-methylphenyl)-N-([1,3]thiazolo[5,4-b]pyridin-2-ylmethyl)-1,2,4-triazin-3-amine,
-2-Dimethylamino-N-(4-{3-[(5-methoxy-benzothiazol-2-ylmethyl)-amino]-[1,2,4]triazin-5-yl}-3,5-dimethyl-phenyl)-acetamide,
-N,N-Dimethyl-N'-[4-(3-{[(5-methoxy-1,3-benzothiazol-2-yl)methyl]amino}-1,2,4-triazin-5-yl)phenyl]ethane-1,2-diamine,
-{(1,3-Benzothiazol-2-ylmethyl)[5-(2,6-dimethoxyphenyl)-1,2,4-triazin-3-yl]amino}acetic acid,
-N-({5-[(2-Aminoethyl)thio]-1,3-benzothiazol-2-yl}methyl)-5-(2,6-dichlorophenyl)-1,2,4-triazin-3-amine,
-5-[2-Chloro-5-(1H-pyrazol-3-yl)phenyl]-N-[(5-methoxy-1,3-benzothiazol-2-yl)methyl]-1,2,4-triazin-3-amine,
-5-[2-Chloro-5-(1H-pyrazol-4-yl)phenyl]-N-[(5-methoxy-1,3-benzothiazol-2-yl)methyl]-1,2,4-triazin-3-amine,
-5-(2-Chloro-5-pyridin-3-ylphenyl)-N-[(5-methoxy-1,3-benzothiazol-2-yl)methyl]-1,2,4-triazin-3-amine,
-2-[4-(4-Chloro-3-{3-[(5-methoxy-benzothiazol-2-ylmethyl)-amino]-[1,2,4]triazin-5-yl}-phenyl)-[1,2,3]triazol-1-yl]-ethanol,
-N-[(5-Methoxy-1,3-benzothiazol-2-yl)methyl]-5-(2-chloro-4-piperazin-1-ylphenyl)-1,2,4-triazin-3-amine, and
-N-[4-(3-{[5-(2-Amino-ethoxy)-benzothiazol-2-ylmethyl]-amino}-[1,2,4]triazin-5-yl)-3,5-dimethyl-phenyl]-N',N'-dimethyl-ethane-1,2-diamine,
and their pharmaceutically acceptable salts with acids and bases.

The invention also relates to pharmaceutical compositions comprising an effective amount of at least one compound of formula I such as above defined, in association with a pharmaceutically acceptable carrier.

Said pharmaceutical compositions are advantageously formulated to be administered under oral, parental, and preferably injectable routes, with individual doses appropriate for the patient to be treated.

The compositions according to the invention can be solid or liquid and be present in the pharmaceutical forms commonly used in human medicine, such as for example, plain or sugar-coated tablets, gelatin capsules, granules, suppositories, injectable preparations, ointments, creams, gels; they are prepared according to the customary methods. The active ingredient/s can be incorporated in same, using excipients which are customarily used in these pharmaceutical compositions, such as talc, gum arabic, lactose, starch, magnesium stearate, cocoa butter, aqueous or nonaqueous vehicles, fatty substances of animal or vegetable origin, paraffin derivatives, glycols, various wetting agents, dispersants or emulsifiers, preservatives.

These compositions can in particular be present in the form of a powder intended to be dissolved extemporaneously in an appropriate vehicle, for example, non-pyrogenic sterile water.

The dose administered varies according to the condition treated, the patient in question, the administration route and the product envisaged. It can, for example, be comprised between 0.1 g and 10 g per day, by oral route in humans, with the product described in Example 1 or also comprised between 0.1 g and 10 g per day by intramuscular or intravenous route.

The drugs according to the invention can be advantageously associated/combined with other antibacterials.

A further object of the invention is therefore the associations of the compounds of formula (I) with antimicrobial peptides or natural, hemisynthetic or synthetic antibacterial molecules as well as pharmaceutical compositions containing them.

### Experimental part

The following examples illustrate the invention.

Proton nuclear magnetic resonance (¹H NMR) spectra were recorded on a 400 MHz Brüker instrument, and chemical shifts are reported in parts per million downfield from the internal standard tetramethylsilane (TMS). Abbreviations for NMR data are as follows: s=singlet, d=doublet, t=triplet, q=quadruplet, m=multiplet, dd=doublet of doublets, dt=doublet of triplets, br=broad. J indicates the NMR coupling constant measured in Hertz. CDCl₃ is deuteriochloroform, DMSO-d₆ is hexadeuteriodimethylsulfoxide, and CD₃OD is tetradeuteriomethanol. Mass spectra were obtained using electrospray (ES) ionization techniques on an Agilent 1100 Series LCMS. HPLC (analytical and preparative) were performed on an Agilent 1100 HPLC with DAD (Diode Array Detection). Preparative HPLC were performed at 0.7mL/min on a Thermo Electron, Hypersil BDS C-18 column (250 x 4.6mm, 5µm) using a gradient of acetonitrile and water with 0.1% TFA (50% in acetonitrile to 100% and then back to 50%). Analtech Silica Gel GF and E. Merck Silica Gel 60 F-254 thin layer plates were used for thin layer chromatography. Flash chromatography was carried out on Flashsmart Pack cartridge, irregular silica 40-60µm or spherical silica 20-40µm.

The meaning of certain abbreviations is given herein. ESI refers to electrospray ionization, HPLC refers to high pressure liquid chromatography, LCMS refers to liquid chromatography coupled with a mass spectrometer, M in the context of mass spectrometry refers to the molecular peak, MS refers to mass spectrometer, NMR refers to nuclear magnetic resonance, pH refers to potential of hydrogen, TFA refers to trifluoroacetic acid, Xantphos refers to 9,9-dimethyl-4,5-bis(diphenylphosphino)xanthene, TEA refers to triethylamine, DIPEA refers to *N*,*N-*diisopropylethylamine, dppf refers to 1,1'-bis(diphenylphosphino)ferrocene, THF refers to tetrahydrofuran, DMF refers to *N,N*-dimethylformamide, EDAC refers *N-*(3-dimethylaminopropyl)-N'-ethylcarbodiimide hydrochloride, 4-DMAP refers to 4-(dimethylamino)pyridine, TLC refers to thin layer chromatography.

The starting materials are commercially available unless indicated otherwise.

### Example 1:

### 5-(2,6-Dimethoxyphenyl)-N-[(4,5-dimethyl-1,3-thiazol-2-yl)methyl]-1,2,4-triazin-3-amine

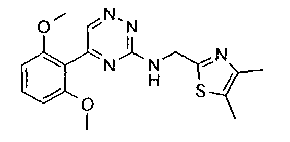

a)
   To a solution of 1-(2,6-dimethoxyphenyl)ethanone (736 mg, 4 mmol) in anhydrous THF (4 mL), was added portionwise phenyl trimethyl ammonium tribromide (3.18 g, 8 mmol) over a period of one hour. The solution was stirred under argon overnight. Cold water (5 mL) was added and the resulting mixture was filtered. The solid was rinsed with cold water (5 mL) twice. The solid was collected, dissolved in dichloromethane and the resulting solution dried over sodium sulfate, filtered and evaporated. 2,2-dibromo-1-(2,6-dimethoxyphenyl)ethanone (1.17 g, 86%) was obtained as a white solid and was used in the next step without purification.
   ESI-MS m/z 337, 339 and 341 (M+H)⁺.
b)
   Under argon, morpholine (333 µL, 3.8 mmol) was added all at once to a solution of 2,2-dibromo-1-(2,6-dimethoxyphenyl)ethanone (308 mg, 0.91 mmol) in anhydrous THF (0.3 mL). The resulting mixture was thoroughly degassed and then heated slowly over one hour to 67°C. The solution was kept stirring at this temperature for 2 days after which it was cooled to room temperature. The white solid was filtered off and the residue was concentrated.
   Under argon, the crude residue was next dissolved in methanol (1.5 mL), aminoguanidine bicarbonate (128 mg, 0.94 mmol) was added followed by acetic acid (156 µL, 2.7 mmol). The resulting mixture was briefly degassed and stirred at room temperature for 1.5 hours, then heated at 67°C for 2 days. The reaction mixture was then cooled to room temperature, concentrated and purified by flash chromatography (silica gel, dichloromethane/methanol, 1/0 to 95/5) to afford 5-(2,6-dimethoxyphenyl)-1,2,4-triazin-3-amine (66.3 mg, 31%) as a white solid.
   ESI-MS m/z 233 (M+H)⁺.
   (4,5-Dimethyl-1,3-thiazol-2-yl)methylamine could be prepared according to literature procedure (Justus Liebigs Ann. Chem. 1964, 676, 141) or using the following procedure:
c)
   Under argon, to a solution of 4,5-dimethylthiazole (600 µL, 5.5 mmol) in anhydrous THF (40 mL) at -78°C, *n*-butyllithium (2.3 M in hexanes, 3.6 mL, 8.28 mmol) was slowly added and the reaction mixture was stirred at -78°C for one hour. Then a solution of anhydrous DMF (1.1 mL, 14.2 mmol) in anhydrous THF (10 mL) was added. The resulting mixture was stirred for 2.5 hours, allowing the temperature to raise to -60°C. Acetic acid (0.5 mL) and an aqueous solution of ammonium chloride were added, and the temperature let to raise to room temperature. The resulting solution was extracted with diethyl ether and ethyl acetate. The combined organic extracts were dried over sodium sulfate, filtered and evaporated. The crude product was purified by flash chromatography (silica gel, cyclohexane/ethyl acetate, 1/0 to 7/3) to afford 4,5-dimethyl-1,3-thiazole-2-carbaldehyde (724 mg, 93%), as a yellow oil which turns into a white solid after storage at -18°C.
   ESI-MS m/z 160 (M+H₂O+H)⁺.
d) Representative procedure for the conversion of thiazole-2-carbaldehyde derivatives into (1,3-thiazol-2-yl)methylamine derivatives.
   Under argon, a solution of 4,5-dimethyl-1,3-thiazole-2-carbaldehyde (644.9 mg, 4.57 mmol), hydroxylamine hydrochloride (648.1 mg, 9.143 mmol) and sodium methoxide (493.7 mg, 9.14 mmol) in ethanol (9 mL) was stirred at room temperature overnight. The white solid was filtered off, rinsed with methanol and the solution was evaporated. The crude oxime was dissolved in acetic acid (25 mL), zinc dust (1.8 g, 27.5 mmol) was added portionwise at room temperature and the reaction mixture was stirred at room temperature for 3 hours. The mixture was then filtered over a pad of celite and rinsed with methanol. Toluene was added and the filtrate was concentrated. An aqueous solution of ammonium chloride was added, the solution was acidified to pH = 2 with hydrochloric acid 1N and extracted with diethyl ether and ethyl acetate. These organic layers were discarded. The aqueous layer was basified with aqueous sodium hydroxide to pH = 10, and then extracted with diethyl ether and ethyl acetate. Combined organic extracts were dried over sodium sulfate, filtered and evaporated to give crude (4,5-dimethyl-1,3-thiazol-2-yl)methylamine (321.7 mg, 49%) as a pale yellow oil which was used in the next steps without purification.
   ESI-MS m/z 143 (M+ H)⁺.
e)
   Under argon, a solution of tert-butyl nitrite (90%, 85 µL, 0.64 mmol) in anhydrous acetonitrile (0.5 mL) was added to a solution of 5-(2,6-dimethoxyphenyl)-1,2,4-triazin-3-amine (100 mg, 0.43 mmol) and copper(II) chloride (70 mg, 0.52 mmol) in anhydrous acetonitrile (2.5 mL). The resulting mixture was stirred at 65°C for 2 hours. After cooling to room temperature, cold 3N aqueous hydrochloric acid was added and the mixture was extracted with diethyl ether and ethyl acetate. The combined organic extracts were washed with brine, then dried over sodium sulfate, filtered and evaporated to give crude 3-chloro-5-(2,6-dimethoxyphenyl)-1,2,4-triazine as a brown solid. The solid was dissolved in anhydrous acetonitrile (2 mL), then (4,5-dimethyl-1,3-thiazol-2-yl)methylamine (61.1 mg, 0.43 mmol) and triethylamine (60 µL, 0.43 mmol) were added and the resulting mixture was stirred at 80°C overnight. The reaction mixture was concentrated. Purification by preparative TLC (silica gel, dichloromethane/methanol 95/5) afforded 5-(2,6-dimethoxyphenyl)-N-[(4,5-dimethyl-1,3-thiazol-2-yl)methyl]-1,2,4-triazin-3-amine (25.6 mg, 17%) as a beige solid.
   ESI-MS m/z 358 (M+H)⁺.
   ¹H NMR (CDCl₃), δ (ppm): 8.59 (s, 1H), 7.38 (t, J = 8.4 Hz, 1H), 6.63 (d, J = 8.4 Hz, 2H), 4.99 (d, J = 5.2 Hz, 2H), 3.75 (s, 6H), 2.31 (s, 6H).

### Example 2:

### 5-(3-Bromo-2,6-dimethoxyphenyl)-N-[(4,5-dimethyl-1,3-thiazol-2-yl)methyl]-1,2,4-triazin-3-amine

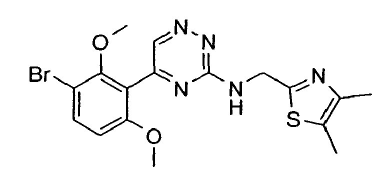

Under argon, N-bromosuccinimide (3.8 mg, 0.02 mmol) was added to a solution of 5-(2,6-dimethoxyphenyl)-N-[(4,5-dimethyl-1,3-thiazol-2-yl)methyl]-1,2,4-triazin-3-amine (7.7 mg, 0.02 mmol) in dichloromethane (0.3 mL). The reaction mixture as stirred at room temperature overnight. The reaction mixture was concentrated. Purification by preparative TLC (silica gel, dichloromethane/methanol 95/5) afforded 5-(3-bromo-2,6-dimethoxyphenyl)-N-[(4,5-dimethyl-1,3-thiazol-2-yl)methyl]-1,2,4-triazin-3-amine (4.6 mg, 49%) as a brown solid.

ESI-MS m/z 436 and 438 (M+H)⁺.

¹H NMR (CDCl₃), δ (ppm): 8.63 (s, 1H), 7.62 (d, J = 8.8 Hz, 1H), 6.70 (d, J = 8.8 Hz, 1H), 5.17 (br s, 2H), 3.77 (s, 3H), 3.72 (s, 3H), 2.42 (s, 3H), 2.37 (s, 3H).

### Example 3:

### 2-(3-{[(4,5-Dimethyl-1,3-thiazol-2-yl)methyl]amino}-1,2,4-triazin-5-yl)-3-methoxyphenol

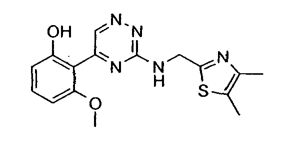

A solution of 5-(2,6-dimethoxyphenyl)-N-[(4,5-dimethyl-1,3-thiazol-2-yl)methyl]-1,2,4-triazin-3-amine (600 mg, 1.67 mmol) in anhydrous dichloromethane (6 mL) under nitrogen atmosphere was cooled to -78°C. Boron tribromide (0.32ml, 3.3mmol) was added dropwise while maintaining the temperature of the reaction mixture below -40°C. The reaction was then allowed to warm up slowly to 0°C at which temperature the reaction was complete on TLC. The reaction mixture was quenched by addition of a saturated aqueous ammonium chloride solution and neutralized with 10% sodium bicarbonate aqueous solution. The resulting mixture was extracted with dichloromethane, the combined organic extracts were washed with water, brine, dried over anhydrous sodium sulfate and concentrated. Purification by flash chromatography (silica gel, dichloromethane/methanol, 98.5/1.5) afforded 2-(3-{[(4,5-dimethyl-1,3-thiazol-2-yl)methyl]amino}-1,2,4-triazin-5-yl)-3-methoxyphenol (150 mg, 26%) as a yellow solid.

ESI-MS m/z 344 (M+H)⁺.

¹H NMR (CDCl₃), δ (ppm): 9.23 (s, 1H), 7.27-7.24 (m, 1H), 6.74 (dd, J = 8.3 Hz and 1.0 Hz, 1H), 6.52 (dd, J = 8.3 Hz and 1.0 Hz, 1H), 6.1 (br s, 1H), 4.94 (d, J = 6.1 Hz, 2H), 3.91 (s, 3H), 2.33 (s, 6H).

### Example 4:

### N-(1,3-Benzothiazol-2-ylmethyl)-5-(2,6-dimethoxyphenyl)-1,2,4-triazin-3-amine

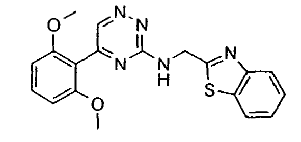

Under argon, a solution of 5-(2,6-dimethoxyphenyl)-1,2,4-triazin-3-amine (30 mg, 0.13 mmol) and 1,3-benzothiazole-2-carbaldehyde (42 mg, 0.26 mmol) in 1,2-dichloroethane (0.7 mL) and THF (0.3 mL) with 4Å molecular sieves was stirred overnight at 35°C. Sodium borohydride (15 mg, 0.4 mmol) and methanol (0.2 mL) were then added and the reaction mixture was stirred for 4 hours at 35°C. The solution was filtered and the filtrate was concentrated. Dichloromethane and water were added, the resulting mixture was extracted with dichloromethane. The combined organic extracts were dried over sodium sulfate, filtered and evaporated. Purification by preparative TLC (silica gel, dichloromethane/methanol 95/5) afforded N-(1,3-benzothiazol-2-ylmethyl)-5-(2,6-dimethoxyphenyl)-1,2,4-triazin-3-amine (2.5 mg, 5%) as a white solid.

ESI-MS m/z 380 (M+H)⁺.

¹H NMR (CDCl₃), δ (ppm): 8.61 (s, 1H), 7.98 (d, J = 8.0 Hz, 1H), 7.84 (d, J = 8.0 Hz, 1H), 7.48-7.45 (m, 1H), 7.40-7.35 (m, 2H), 6.62 (d, J = 8.0 Hz, 2H), 5.20 (d, J = 5.2 Hz, 2H), 3.70 (s, 6H).

### Example 5:

### N-[(4,5-Dimethyl-1,3-thiazol-2-yl)methyl]-5-(2-nitrophenyl)-1,2,4-triazin-3-amine

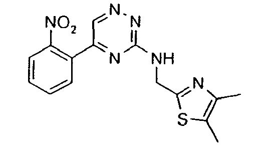

Representative procedure for the conversion of acetophenone derivatives into N-substituted-5-phenyl-1,2,4-triazin-3-amine compounds via 5-aryl-1,2,4-triazin-3-amine intermediates.
a)
   To a solution of 1-(2-nitrophenyl)ethanone (15 g, 91 mmol) in 1,4-dioxane (300 mL) and water (50 mL) was added selenium dioxide (50.4 g, 450 mmol). The reaction mixture was refluxed for 12 hours until the reaction was complete on TLC. The reaction mixture was cooled, diluted with water and extracted with dichloromethane. The combined organic extracts were washed with brine, dried over anhydrous sodium sulfate and concentrated to give crude (2-nitrophenyl)(oxo)acetaldehyde as a brown oil which was used in the next step without purification.
b)
   The crude (2-nitrophenyl)(oxo)acetaldehyde was dissolved in methanol (100 mL) and aminoguanidine hydrochloride (9.24 g, 84 mmol) was added followed by triethylamine (11.6 mL, 84 mmol). The resulting mixture was degassed and stirred at room temperature for 1.5 hours and then refluxed for 2 days. The mixture was cooled to room temperature and concentrated. Purification by flash chromatography (silica gel, chloroform/methanol gradient) led to 5-(2-nitrophenyl)-1,2,4-triazin-3-amine (6.0 g, 30%) as a yellow solid.
   ESI-MS m/z 218 (M+H)⁺.
c)
   To a solution of copper(II) chloride (3.56 g, 26.5 mmol) and tert-butyl nitrite (3.93 mL, 33 mmol) in anhydrous acetonitrile at 65°C was slowly added 5-(2-nitrophenyl)-1,2,4-triazin-3-amine (4.8 g, 22.1 mmol) and the resulting mixture was stirred for 3 hours. The reaction mixture was cooled, and poured into 1.5N hydrochloric acid cooled to 0°C. The mixture was extracted with diethylether and the combined ether layers were washed with brine, dried over anhydrous sodium sulfate and concentrated. The crude product was purified by flash chromatography (silica gel, chloroform/methanol, 99/1) to give 3-chloro-5-(2-nitrophenyl)-1,2,4-triazine (1.6 g, 31 %).
   ESI-MS m/z 237 and 239 (M+H)⁺.
d)
   To a solution of 3-chloro-5-(2-nitrophenyl)-1,2,4-triazine (1.2 g, 5.07 mmol) in anhydrous acetonitrile (7 mL) was added (4,5-dimethyl-1,3-thiazol-2-yl)methylamine (720 mg, 5.07 mmol, prepared as in example 1), anhydrous potassium carbonate (3.5 g, 25.3 mmol) and the resulting mixture was stirred at room temperature for 12 hours. The reaction mixture was concentrated, diluted with water and extracted with ethyl acetate. The combined organic extracts were washed with brine, dried over anhydrous sodium sulfate and concentrated. The crude product obtained was purified by flash chromatography (silica gel, chloroform/methanol, 99/1) to afford N-[(4,5-dimethyl-1,3-thiazol-2-yl)methyl]-5-(2-nitrophenyl)-1,2,4-triazin-3-amine (700 mg, 40%) as a brown solid.
   ESI-MS m/z 343 (M+H)⁺.
   ¹H NMR (CDCl₃), δ (ppm): 8.8 (s, 1H), 8.03 (d, J = 7.8 Hz, 1H), 8.02-7.63 (m, 3H), 4.90 (br s, 2H), 2.3 (s, 6H).

### Example 6:

### 5-(2-Aminophenyl)-N-[(4,5-dimethyl-1,3-thiazol-2-yl)methyl]-1,2,4-triazin-3-amine

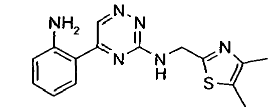

To a solution of N-[(4,5-dimethyl-1,3-thiazol-2-yl)methyl]-5-(2-nitrophenyl)-1,2,4-triazin-3-amine (180 mg, 0.53 mmol) in methanol (4 mL) was added iron powder (350 mg, 6.3 mmol), followed by ammonium chloride (450 mg, 8.4 mmol) in water (4 mL). The resulting mixture was refluxed for 12 hours. After cooling and filtration through a pad of celite, the filtrate was concentrated and then diluted with water. The aqueous solution was extracted with dichloromethane. Combined organic layers were washed with brine, dried over anhydrous sodium sulfate and concentrated. The crude residue was purified by flash chromatography (silica gel, chloroform/methanol, 99/1) to afford 5-(2-aminophenyl)-N-[(4,5-dimethyl-1,3-thiazol-2-yl)methyl]-1,2,4-triazin-3-amine (100 mg, 60%) as a yellow solid.

ESI-MS m/z 313 (M+H)⁺.

¹H NMR (CDCl₃), δ (ppm): 9.08 (s, 1H), 7.72 (d, J = 8.1 Hz, 1H), 7.30-7.26 (m, 1H), 6.79-6.72 (m, 2H), 6.05 (br s, 2H), 4.92 (br s, 2H), 2.32 (s, 6H).

### Example 7:

### N-[2-(3-{[(4,5-Dimethyl-1,3-thiazol-2-yl)methyl]amino}-1,2,4-triazin-5-yl)phenyl]acetamide

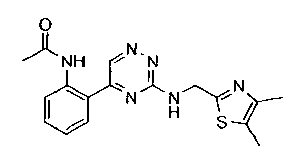

To a solution of 5-(2-aminophenyl)-N-[(4,5-dimethyl-1,3-thiazol-2-yl)methyl]-1,2,4-triazin-3-amine (40 mg, 0.13 mmol) in anhydrous dichloromethane (1 mL) cooled to 0°C, was added acetic anhydride (13 µL, 0.14 mmol) and the resulting mixture was stirred at room temperature for 12h. The reaction mixture was concentrated and the crude residue was purified by flash chromatography (silica gel, chloroform/methanol, 99/1) to yield N-[2-(3-{[(4,5-dimethyl-1,3-thiazol-2-yl)methyl]amino}-1,2,4-triazin-5-yl)phenyl]acetamide (9 mg, 20%) as a yellow solid.

ESI-MS m/z 355 (M+H)⁺.

¹H NMR (CDCl₃), δ (ppm): 9.09 (s, 1H), 8.57 (d, J = 8.2 Hz, 1H), 7.76 (d, J = 8.2 Hz, 1H), 7.57-7.52 (m, 1H), 7.25-7.21 (m, 1H), 4.99 (br s, 2H), 2.32 (s, 6H), 2.15 (s, 3H).

### Example 8:

### N-[2-(3-{[(4,5-Dimethyl-1,3-thiazol-2-yl)methyl]amino}-1,2,4-triazin-5-yl)phenyl]lmethanesulfonamide

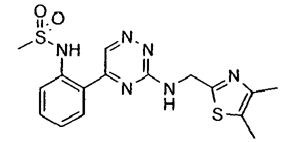

To a solution of 5-(2-aminophenyl)-N-[(4,5-dimethyl-1,3-thiazol-2-yl)methyl]-1,2,4-triazin-3-amine (70 mg, 0.22 mmol) in anhydrous dichloromethane (2 mL) cooled to 0°C, was added pyridine (90 µL, 1.11 mmol) followed by methanesulfonyl chloride (17 µL, 0.22 mmol). The reaction mixture was stirred at room temperature overnight. The reaction mixture was basified with an aqueous sodium bicarbonate solution and extracted with dichloromethane. Combined organic extracts were washed with water, brine, dried over anhydrous sodium sulfate and concentrated. The crude residue was purified by preparative HPLC (reverse phase) to obtain N-[2-(3-{[(4,5-dimethyl-1,3-thiazol-2-yl)methyl]amino}-1,2,4-triazin-5-yl)phenyl]methanesulfonamide (10 mg, 11%) as a brown solid.

ESI-MS m/z 391 (M+H)⁺.

¹H NMR (CDCl₃), δ (ppm): 9.17 (s, 1H), 7.92 (d, J = 7.8 Hz, 1H), 7.78 (d, J = 8.1 Hz, 1H), 7.59-7.56 (m, 1H), 7.27-7.24 (m, 1H), 4.97 (br s, 2H), 3.08 (s, 3H), 2.33 (s, 6H).

### Example 9:

### 5-(2-Chlorophenyl)-N-[(4,5-dimethyl-1,3-thiazol-2-yl)methyl]-1,2,4-triazin-3-amine

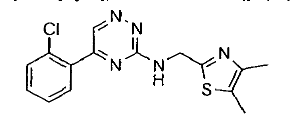

A solution of 5-(2-aminophenyl)-N-[(4,5-dimethyl-1,3-thiazol-2-yl)methyl]-1,2,4-triazin-3-amine (100 mg, 0.32 mmol) in water (1 mL) and hydrochloric acid (37%, 1 mL) was cooled to 0°C. Sodium nitrite (30 mg, 0.43 mmol) in water (0.5 mL) was added dropwise, maintaining the temperature below 5°C. The reaction mixture was stirred at 0°C for 30 min. To this mixture was added a freshly prepared solution of copper(I) chloride (47 mg, 0.48 mmol) in water (1 mL) and hydrochloric acid (37%, 1 mL). The resulting mixture was stirred at room temperature for 30 minutes. The reaction mixture was basified with an aqueous sodium bicarbonate solution and extracted with dichloromethane. Combined organic extracts were washed with water, brine, dried over anhydrous sodium sulfate and concentrated. The crude residue was purified by flash chromatography (silica gel, chloroform/methanol, 99/1) to yield 5-(2-chlorophenyl)-N-[(4,5-dimethyl-1,3-thiazol-2-yl)methyl]-1,2,4-triazin-3-amine (30 mg, 28%) as a yellow solid.

ESI-MS m/z 332 and 334 (M+H)⁺.

¹H NMR (CDCl₃), δ (ppm): 9.09 (s, 1H), 7.69-7.67 (m, 1H), 7.53-7.51 (m, 1H), 7.48-7.42 (m, 2H), 5.01 (br s, 2H), 2.34 (s, 6H).

In the following examples (example 10 to example 12), the title compounds are prepared from acetophenone derivatives which are commercially available starting materials, following the representative procedure described in example 5 for the synthesis of N-substituted-5-phenyl-1,2,4-triazin-3-amine compounds via 5-aryl-1,2,4-triazin-3-amine intermediates.

### Example 10:

### 5-(2,5-Dimethoxyphenyl)-N-[(4,5-dimethyl-1,3-thiazol-2-yl)methyl]-1,2,4-triazin-3-amine

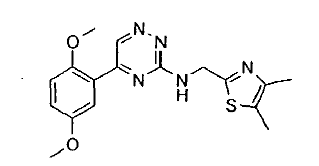

ESI-MS m/z 358 (M+H)⁺.

¹H NMR (CDCl₃), δ (ppm): 8.84 (s, 1H), 7.56 (d, J = 2.2 Hz, 1H), 6.97-6.96 (m, 2H), 4.98 (d, J = 5.7Hz, 2H), 3.84 (s, 6H), 2.33 (s, 6H).

### Example 11:

### 5-(2,4-Dimethoxyphenyl)-N-[(4,5-dimethyl-1,3-thiazol-2-yl)methyl]-1,2,4-triazin-3-amine

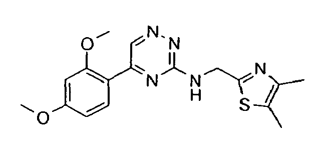

ESI-MS m/z 358 (M+H)⁺.

¹H NMR (CDCl₃), δ (ppm): 8.76 (s, 1H), 7.89 (d, J = 8.6 Hz, 1H), 6.65 (dd, J = 8.5 Hz and 2.3 Hz, 1H), 6.56 (d, J = 2.3 Hz, 1H), 4.96 (d, J = 4.7 Hz, 2H), 3.87 (s, 3H), 3.86 (s, 3H), 2.32 (s, 6H).

### Example 12:

### N-[(4,5-Dimethyl-1,3-thiazol-2-yl)methyl]-5-(1-naphthyl)-1,2,4-triazin-3-amine

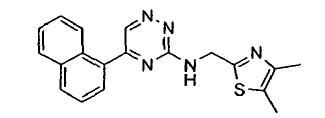

ESI-MS m/z 348 (M+H)⁺.

¹H NMR (CDCl₃), δ (ppm): 8.98 (s, 1H), 8.24 (d, J = 8.2 Hz, 1H), 8.02 (d, J = 8.0 Hz, 1H), 7.95 (d, J = 8.2 Hz, 1H), 7.75-7.73 (m, 1H), 7.62-7.53 (m, 3H), 5.0 (br s, 2H), 2.33 (s, 6H).

### Example 13:

### N-(1,3-Benzothiazol-2-ylmethyl)-5-[2-(trifluoromethyl)phenyl]-1,2,4-triazin-3-amine

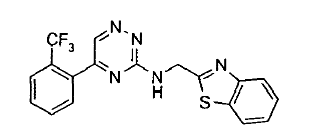

a)
   To a solution of 2-(trifluoromethyl)phenylglyoxal hydrate (5.98 g, 27.1 mmol) and aminoguanidine hydrochloride (3.34 g, 29.6 mmol) in ethanol (90 mL) under nitrogen atmosphere, was added triethylamine (4.13 mL, 29.6 mmol). The reaction mixture was stirred for 2 hours at room temperature, then refluxed for 2 days. The solvent was evaporated to give an orange solid, which was dissolved in acetone (100 mL), then water (300 mL) was added and a solid was filtered off, washed with water, to give 5-[2-(trifluoromethyl)phenyl]-1,2,4-triazin-3-amine (3.32 g, 51%) as a brown solid which was used in the next step without purification.
   ESI-MS m/z 241 (M+H)⁺.
b)
   5-[2-(trifluoromethyl)phenyl]-1,2,4-triazin-3-amine (1.0 g, 4.16 mmol) was added to a solution of potassium hydroxide (0.51 g, 9.1 mmol) in water (5 mL) and ethanol (5 mL). The resulting mixture was heated for 16 hours at 96°C. The solvents were evaporated, the residue was dissolved in water and the pH adjusted to 7 with aqueous hydrochloric acid (1N, 1mL). The solvent was evaporated to give 5-(2-methylphenyl)-1,2,4-triazin-3(2H)-one as a green solid. The latter compound was dissolved in anhydrous chloroform (10 mL), phosphorus oxychloride (3.65 mL, 39 mmol) was added followed by a few drops of anhydrous DMF. The reaction mixture was stirred at 60°C for 2 hours. The solution was then poured into an ice-water mixture (30 mL), and dichloromethane (30 mL) was added. The resulting mixture was stirred for 2 hours at room temperature. The aqueous phase was extracted with dichloromethane, combined organic extracts were dried over anhydrous sodium sulfate, filtered and evaporated. The residue was purified by flash chromatography (silica gel, cyclohexane/dichloromethane, 1/0 to 0/1) to give 3-chloro-5-[2-(trifluoromethyl)phenyl]-1,2,4-triazine as a light brown oil (726 mg, 67%).
   ESI-MS m/z 260 and 262 (M+H)⁺.
   ¹H NMR (CDCl₃), δ (ppm): 9.42 (s, 1H), 7.91-7.89 (m, 1H), 7.80-7.73 (m, 2H), 7.65-7.64 (m, 1H).
c)
   To a solution of 3-chloro-5-[2-(trifluoromethyl)phenyl]-1,2,4-triazine (31.5 mg, 0.12 mmol) and 1,3-benzothiazol-2-ylmethylamine hydrochloride (48 mg, 0.24 mmol) in anhydrous acetonitrile (0.5 mL), was added potassium carbonate (50 mg, 0.36 mmol). The reaction mixture was stirred at 50°C for 30 minutes then at 85°C overnight. The reaction mixture was concentrated. Purification by preparative TLC (silica gel, dichloromethane/methanol 94/6) afforded N-(1,3-benzothiazol-2-ylmethyl)-5-[2-(trifluoromethyl)phenyl]-1 ,2,4-triazin-3-amine (14.8 mg, 31%) as a yellow solid.
   ESI-MS m/z 388 (M+H)⁺.
   ¹H NMR (CDCl₃), δ (ppm): 8.81 (s, 1H), 8.00 (d, J = 8.0 Hz, 1H), 7.86-7.82 (m, 2H), 7.70-7.63 (m, 2H), 7.53-7.46 (m, 2H), 7.41-7.37 (m, 1H), 5.20 (br s, 2H).

### Example 14:

### N-(1,3-Benzothiazol-2-ylmethyl)-5-[2-chloro-5-(trifluoromethyl)phenyl]-1,2,4-triazin-3-amine

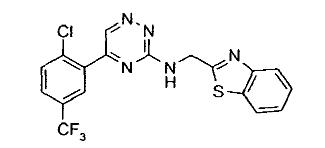

Under argon, to a solution of 3-chloro-5-[2-chloro-5-(trifluoromethyl)phenyl]-1,2,4-triazine (625 mg, 2.12 mmol, prepared from 1-[2-chloro-5-(trifluoromethyl)phenyl]ethanone according to the representative procedure described in example 5) in anhydrous acetonitrile (5 mL) were added 1,3-benzothiazol-2-ylmethylamine hydrochloride (425 mg, 2.12 mmol) and potassium carbonate (4.38 g, 31.7 mmol). The reaction mixture was stirred at room temperature for 12 hours. The reaction mixture was concentrated, diluted with water and extracted with ethyl acetate. The combined organic extracts were washed with brine, dried over anhydrous sodium sulfate and concentrated. The crude product obtained was purified by flash chromatography (silica gel, 0.25% methanol in chloroform) to afford N-[(4,5-dimethyl-1,3-thiazol-2-yl)methyl]-5-(2-nitrophenyl)-1,2,4-triazin-3-amine (200 mg, 22%) as a brown solid.

ESI-MS m/z 422 and 424 (M+H)⁺.

¹H NMR (CDCl₃), δ (ppm): 8.74 (s, 1H), 8.06 (d, J = 8.2 Hz, 1H), 8.02 (s, 1H), 7.89 (d, J = 8.0 Hz, 1H), 7.68-7.64 (m, 2H), 7.55-7.51 (m, 1H), 7.45-7.42 (m, 1H), 5.28 (br s, 2H).

### Example 15:

### 5-(2,6-Dichlorophenyl)-N-[(5-methoxy-1,3-benzothiazol-2-yl)methyl]-1,2,4-triazin-3-amine

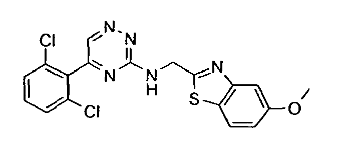

a)
   Under nitrogen atmosphere, to a solution of 1-(2,6-dichloropbenyl)ethanone (510 mg, 2.7 mmol) in ethanol (5 mL) and water (0.5 mL), selenium dioxide (330 mg, 3.0 mmol) was added. The reaction mixture was stirred at 105 °C for 48 hours. The solution was filtered on a pad of celite and rinsed with ethanol. The filtrate was concentred to give crude (2,6-dichlorophenyl)(oxo)acetaldehyde which was used without further purification in the next step.
b)
   Under nitrogen atmosphere, to a solution of crude (2,6-dichlorophenyl)(oxo)acetaldehyde in ethanol (5 mL) and water (5 mL), semicarbazide hydrochloride (248 mg, 2.2 mmol) and potassium carbonate (304 mg, 2.2 mmol) were added, and the reaction mixture was stirred at room temperature for 2 hours and then refluxed for 20 hours. The solution was filtered on a pad of celite and rinsed with ethanol. Ethanol in the filtrate was concentrated, and the aqueous solution was acidified with aqueous hydrochloric acid 6N. The solution was extracted with ethyl acetate, combined organic extracts were washed with water, dried over sodium sulfate, filtered and concentrated to give crude 5-(2,6-dichlorophenyl)-1,2,4-triazin-3(2H)-one as a brown oil which was used in the next step without purification.
c)
   Under nitrogen atmosphere, to a solution of crude 5-(2,6-dichlorophenyl)-1,2,4-triazin-3(2H)-one 5-(2,6-dichlorophenyl)-1,2,4-triazin-3(2H)-one in anhydrous chloroform (2 mL) at room temperature, phosphorus oxychloride (0.75 mL, 8.2 mmol) was added dropwise, followed by a few drops of DMF. The reaction mixture was refluxed for 2 hours. Completion of the reaction was followed by TLC. The reaction mixture was cooled to room temperature and poured into an ice-water mixture. The solution was stirred for 5 minutes, then aqueous sodium hydroxide 2N (5 mL) was slowly added over 10 minutes. The solution was extracted with dichloromethane, combined organic extracts were washed with water, dried over sodium sulfate, filtered and concentrated. Purification by flash chromatography (silica gel, cyclohexane/ethyl acetate, 95/5) afforded 3-chloro-5-(2,6-dichlorophenyl)-1,2,4-triazine (85 mg, 12%) as a yellow oil.
   ¹H NMR (CDCl₃, 300 MHz), δ (ppm): 9.29 (s, 1H), 7.51-7.41 (m, 3H).
d) Representative procedure for the synthesis of (1,3-benzothiazol-2-yl)methylamine compounds from methyl-1,3-benzothiazole derivatives
   Under argon, selenium dioxide (371 mg, 3.34 mmol) was added to a solution of 5-methoxy-2-methyl-1,3-benzothiazole (400 mg, 2.23 mmol) in 1,4-dioxane (6 mL). The reaction mixture was stirred at 105°C for 3 hours, then filtered over a pad of celite and rinsed with hot 1,4-dioxane. The filtrate was concentrated to give crude 5-methoxy-1,3-benzothiazole-2-carbaldehyde. The latter compound was dissolved in ethanol (4.7 mL), hydroxylamine hydrochloride (306 mg, 4.45 mmol) and sodium methylate (285 mg, 5.28 mmol) were successively added and the reaction mixture was stirred at room temperature overnight. The solution was filtered and concentrated to give crude 5-methoxy-1,3-benzothiazole-2-carbaldehyde oxime. The latter compound was dissolved in acetic acid (12 mL), and zinc dust (875 mg, 13.38 mmol) was added portionwise. The solution was stirred at room temperature for 3 hours. The mixture was then filtered over a pad of celite and rinsed with methanol. Toluene was added and the filtrate was concentrated. Water was added, then the solution was acidified to pH = 2 with hydrochloric acid 1N and extracted with ethyl acetate. These organic layers were discarded. The aqueous layer was basified with aqueous sodium hydroxide to pH = 12, and then extracted with ethyl acetate. Combined organic extracts were dried over sodium sulfate, filtered and evaporated to give (5-methoxy-1,3-benzothiazol-2-yl)methylamine (180 mg, 42%). The latter compound could be isolated as its hydrochloride salt by stirring in a solution of hydrogen chloride in methanol and ethyl acetate for 2 hours and then filtration. The solid was rinsed with ethyl acetate to afford (5-methoxy-1,3-benzothiazol-2-yl)methylamine hydrogen chloride.
   ESI-MS m/z 195 (M+H)⁺.
   ¹H NMR (DMSO-d₆, 300 MHz), δ (ppm): 8.84 (br s, 3H), 8.05 (d, J = 9.0 Hz, 1H), 7.53 (d, J = 2.4 Hz, 1H), 7.15 (dd, J = 9.0 Hz and 2.4Hz, 1H), 4.59-4.54 (m, 2H), 3.86 (s, 3H).
e)
   Under nitrogen atmosphere, (5-methoxy-1,3-benzothiazol-2-yl)methylamine (70 mg, 0.36 mmol) and DIPEA (63 µL, 0.36 mmol) were added to a solution of 3-chloro-5-(2,6-dichlorophenyl)-1,2,4-triazine (85 mg, 0.33 mmol) in anhydrous acetonitrile (1 mL), and the reaction mixture was refluxed overnight. Water was added and the solution was extracted with ethyl acetate. Combined organic extracts were washed with water, dried over sodium sulfate, filtered and concentrated to give a brown solid. The crude product was washed with methylene chloride to give 5-(2,6-dichlorophenyl)-N-[(5-methoxy-1,3-benzothiazol-2-yl)methyl]-1,2,4-triazin-3-amine (75 mg, 55%) as a beige solid.
   ESI-MS m/z 418 and 420 (M+H)⁺.
   ¹H NMR (CDCl₃), δ (ppm): 8.69 (s, 1H), 7.68 (d, J = 8.9 Hz, 1H), 7.47 (d, J = 2.0 Hz, 1H), 7.42-7.32 (m, 3H), 7.02 (dd, J = 8.9 Hz and J = 2.0 Hz, 1H), 5.16 (s, 2H), 3.38 (s, 3H).

### Example 16:

### 2-{[2-({[5-(2,6-Dichlorophenyl)-1,2,4-triazin-3-yl]amino}methyl)-1,3-benzothiazol-5-y]oxy}ethanol

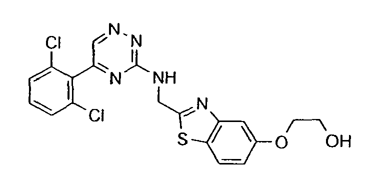

a)
   Under argon, a solution of boron tribromide in dichloromethane (1M, 6.4 mL, 6.4 mmol) was slowly added to a solution of 5-(2,6-dichlorophenyl)-N-[(5-methoxy-1,3-benzothiazol-2-yl)methyl]-1,2,4-triazin-3-amine (267 mg, 0.64 mmol) in anhydrous dichloromethane (6.4 mL) at -78°C. The reaction mixture was stirred overnight allowing the temperature to rise to room temperature. Then the solution was cooled to 0°C and methanol was added. The resulting mixture was stirred at 50°C for 30 minutes then concentrated. Ethyl acetate and water were added to the residue. The solution was extracted with ethyl acetate; combined organic extracts were dried over sodium sulfate, filtered and evaporated. Purification by preparative TLC (silica gel, dichloromethane/methanol 95/5) afforded 2-({[5-(2,6-dichlorophenyl)-1,2,4-triazin-3-yl]amino}methyl)-1,3-benzothiazol-5-ol (226 mg, 88%) as a yellow solid.
   ESI-MS m/z 404 and 406 (M+H)⁺.
b)
   Under argon, a solution of 2-({[5-(2,6-dichlorophenyl)-1,2,4-triazin-3-yl]amino}methyl)-1,3-benzothiazol-5-ol (101 mg, 0.25 mmol) and potassium carbonate (69 mg, 0.5 mmol) in acetone (2 mL) was stirred at room temperature for 30 minutes. Ethyl bromoacetate (55 µL, 0.5 mmol) was added and the reaction mixture was stirred at 60°C overnight. The mixture was filtered, rinsed with methanol and dichloromethane, and the filtrate was evaporated. Purication by preparative TLC (silica gel, dichloromethane/methanol/ 95/5) afforded ethyl {[2-({[5-(2,6-dichlorophenyl)-1,2,4-triazin-3-yl]amino}methyl)-1,3-benzothiazol-5-yl]oxy}acetate (53.4 mg, 43 %) as a yellow solid.
   ESI-MS m/z 490 and 492 (M+H)⁺.
c)
   Under argon, a solution of diisobutylaluminium hydride in toluene (1M, 420 µL, 0.42 mmol) was slowly added to a solution of ethyl {[2-({[5-(2,6-dichlorophenyl)-1,2,4-tn'azin-3-yl]amino}methyl)-1,3-benzothiazol-5-yl]oxy}acetate (40 mg, 0.081 mmol) in anhydrous dichloromethane (1 mL) stirred at -78°C. The reaction mixture kept stirring for 3 hours, allowing the temperature to raise slowly to -30°C. Then methanol was added, followed by an aqueous solution of sodium potassium tartrate was added and the temperature let to rise. Brine was added and the mixture was extracted with dichloromethane, combined organic extracts were dried over sodium sulfate, filtered and evaporated. The crude product was dissolved in methanol, then a few milligrams of NaBH₄ were added and the resulting mixture was stirred for 2 hours under argon at room temperature. A few drops of hydrochloric acid 2N were added and the solution was concentrated. Purication by preparative TLC (silica gel, dichloromethane/methanol/ 95/5) afforded 2-{[2-({[5-(2,6-dichlorophenyl)-1,2,4-triazin-3-yl]amino}methyl)-1,3-benzothiazol-5-yl]oxy}ethanol (18.9 mg, 52 %) as a white solid.
   ESI-MS m/z 448 and 450 (M+H)⁺.
   ¹H NMR (DMSO-d₆), δ (ppm): 8.87 (s, 1H), 7.89 (d, J = 8.4 Hz, 1H), 7.69-7.54 (m, 3H), 7.48 (br s, 1H), 7.05 (d, J = 8.4 Hz, 1H), 5.05 (br s, 2H), 4.07-4.02 (m, 2H), 3.77-3.73 (m, 2H).

### Example 17:

### {[2-({[5-(2,6-dichlorophenyl)-1,2,4-triazin-3-yl]amino}methyl)-1,3-benzothiazol-5-yl]oxy}acetic acid

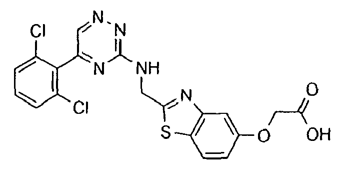

Lithium hydroxide (5.8 mg, 0.24 mmol) was added to a solution of ethyl {[2-({[5-(2,6-dichlorophenyl)-1,2,4-triazin-3-yl]amino}methyl)-1,3-benzothiazol-5-yl]oxy}acetate (40 mg, 0.081 mmol, prepared as in example 16) in THF (1 mL) and water (0.5 mL). The reaction was stirred vigorously under argon at room temperature for 1.5 hours. Hydrochloric acid 1N was added and the solution was extracted with diethyl ether and ethyl acetate. The combined organic extracts were dried over sodium sulfate, filtered and evaporated. The product was recrystallised in a mixture of cyclohexane and dichloromethane to give {[2-({[5-(2,6-dichlorophenyl)-1,2,4-triazin-3-yl]amino}methyl)-1,3-benzothiazol-5-yl]oxy}acetic acid (29 mg, 77%) as a beige solid.

ESI-MS m/z 462 and 464 (M+H)⁺.

¹H NMR (DMSO-d₆), δ (ppm): 8.87 (s, 1H), 7.90 (d, J = 8.8 Hz, 1H), 7.69-7.54 (m, 3H), 7.42 (br s, 1H), 7.05 (d, J = 8.8 Hz, 1H), 5.05 (br s, 2H), 4.72 (s, 2H).

### Example 18:

### 2-(2-{[5-(2,6-Dichloro-phenyl)-[1,2,4]triazin-3-ylamino]-methyl}-benzothiazol-5-yloxy)-N-(2-hydroxy-ethyl)-acetamide

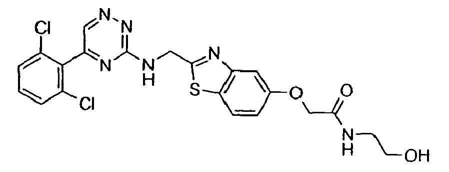

Under argon, ethanolamine (5 µL, 0.083 mmol) was added at room temperature to a solution of {[2-({[5-(2,6-dichlorophenyl)-1,2,4-triazin-3-yl]amino}methyl)-1,3-benzothiazol-5-yl]oxy}acetic acid (20 mg, 0.043 mmol, prepared as in example 17), N-(3-dimethylaminopropyl)-N'-ethylcarbodiimide hydrochloride (16.5 mg, 0.086 mmol) and 4-dimethylaminopyridine (15.8 mg, 0.129 mmol) in anhydrous THF (0.2 mL) and dichloromethane (0.1 mL). The reaction mixture was stirred at 50°C for 6 hours. Then a dilute solution of hydrochloric acid was added and the mixture was extracted with diethyl ether and ethyl acetate. Combined organic extracts were dried over sodium sulfate, filtered and evaporated. Purification by preparative TLC (silica gel, dichloromethane/methanol/aqueous ammonium hydroxide 33%, 90/10/1) gave 2-(2-{[5-(2,6-dichloro-phenyl)-[1,2,4]triazin-3-ylamino]-methyl}-benzothiazol-5-yloxy)-N-(2-hydroxy-ethyl)-acetamide (6.7 mg, 31 %) as a white solid.

ESI-MS m/z 505 and 507 (M+H)⁺.

¹H NMR (CD₃OD), δ (ppm): 8.70 (s, 1H), 7.83 (d, J = 8.7 Hz, 1H), 7.49-7.45 (m, 4H), 7.17 (d, J = 8.7 Hz, 1H), 5.07 (br s, 2H), 4.63 (s, 2H), 3.66 (t, J = 5.7 Hz, 2H), 3.44 (t, J = 5.7 Hz, 2H).

### Example 19:

### 2-(2-{[5-(2,6-Dichloro-phenyl)-[1,2,4]triazin-3-ylamino]-methyl}-benzothiazol-5-yloxy)-N-(2-dimethylamino-ethyl)-acetamide

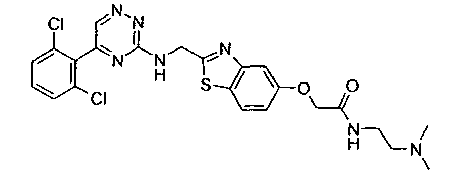

Under argon, N,N-dimethylethane-1,2-diamine (9.4 µL, 0.083 mmol) was added at room temperature to a solution of {[2-({[5-(2,6-dichlorophenyl)-1,2,4-triazin-3-yl]amino}methyl)-1,3-benzothiazol-5-yl]oxy}acetic acid (20 mg, 0.043 mmol, prepared as in example 17), N-(3-dimethylaminopropyl)-N'-ethylcarbodiimide hydrochloride (16.5 mg, 0.086 mmol) and 4-dimethylaminopyridine (15.8 mg, 0.129 mmol) in anhydrous THF (0.2 mL) and dichloromethane (0.1 mL). The reaction mixture was stirred at 50°C for 4 hours. Then a dilute aqueous solution of ammonium hydroxide was added and the mixture was extracted with diethyl ether and ethyl acetate. Combined organic extracts were dried over sodium sulfate, filtered and evaporated. Purification by preparative TLC (silica gel, dichloromethane/methanol/aqueous ammonium hydroxide 33%, 90/10/1) gave 2-(2-{[5-(2,6-Dichlorophenyl)-[1,2,4]triazin-3-ylamino]-methyl}-benzothiazol-5-yloxy)-N-(2-dimethylamino-ethyl)-acetamide (16.5 mg, 72%) as a white solid.

ESI-MS m/z 532 and 534 (M+H)⁺.

¹H NMR (CD₃OD), δ (ppm): 8.69 (s, 1H), 7.82 (d, J = 8.8 Hz, 1H), 7.49-7.45 (m, 4H), 7.16 (dd, J = 8.8 Hz and 1.4 Hz, 1H), 5.06 (br s, 2H), 4.61 (s, 2H), 3.46 (t, J = 6.6 Hz, 2H), 2.55 (t, J = 6.6 Hz, 2H), 2.31 (s, 6H).

### Example 20:

### 1-{[2-({[5-(2,6-Dichlorophenyl)-1,2,4-triazin-3-yl]amino}methyl)-1,3-benzothiazol-5-yl]oxy}acetone

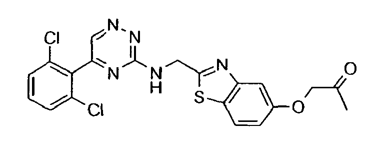

Under argon, a solution of 2-({[5-(2,6-dichlorophenyl)-1,2,4-triazin-3-yl]amino}methyl)-1,3-benzothiazol-5-ol (30 mg, 0.074 mmol, prepared as in example 16) and potassium carbonate (20.5 mg, 0.149 mmol) in anhydrous THF (0.3 mL) was stirred at 50°C for 1 hour. Then 1-chloroacetone (12 µL, 0.15 mmol) was added and the reaction mixture was stirred at 50°C overnight. The mixture was then concentrated and the residue was purified by preparative TLC (silica gel, dichloromethane/methanol 95/5) to afford 1-{[2-({[5-(2,6-dichlorophenyl)-1,2,4-triazin-3-yl]amino}methyl)-1,3-benzothiazol-5-yl]oxy}acetone (5.8 mg, 17%) as a yellow solid.

ESI-MS m/z 460 and 462 (M+H)⁺.

¹H NMR (CDCl₃), δ (ppm): 8.70 (s, 1H), 7.73 (d, J = 8.9 Hz, 1H), 7.43-7.33 (m, 4H), 7.08 (dd, J = 8.9 Hz and J = 2.7 Hz, 1H), 5.17 (br s, 2H), 4.63 (s, 2H), 2.31 (s, 3H).

### Example 21:

### 2-{[2-({[5-(2,6-Dichlorophenyl)-1,2,4-triazin-3-yl]amino}methyl)-1,3-benzothiazol-5-yl]oxy}acetamide

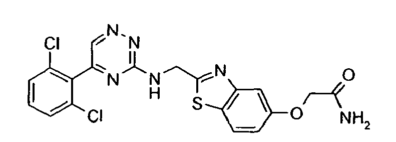

Under argon, a solution of 2-({[5-(2,6-dichlorophenyl)-1,2,4-triazin-3-yl]amino}methyl)-1,3-benzothiazol-5-ol (30 mg, 0.074 mmol, prepared as in example 16) and potassium carbonate (20.5 mg, 0.149 mmol) in anhydrous THF (0.3 mL) was stirred at 50°C for I hour. Then iodoacetamide (27.5 mg, 0.15 mmol) was added and the reaction mixture was stirred at 50°C overnight. The mixture was then concentrated and the residue was purified by preparative TLC (silica gel, dichloromethane/methanol 95/5) to afford 2-{[2-({[5-(2,6-dichlorophenyl)-1,2,4-triazin-3-yl]amino}methyl)-1,3-benzothiazol-5-yl]oxy}acetamide (13.6 mg, 40%) as a yellow solid.

ESI-MS m/z 461 and 463 (M+H)⁺.

¹H NMR (CDCl₃), δ (ppm): 8.70 (s, 1H), 7.74 (d, J = 8.7 Hz, 1H), 7.50 (d, J = 2.4 Hz, 1H), 7.42-7.33 (m, 3H), 7.06 (dd, J = 8.7 Hz and J = 2.4 Hz, 1H), 6.60 (br s, 2H), 5.87 (br s, 1H), 5.17 (br s, 2H), 4.57 (s, 2H).

### Example 22:

### Benzothiazol-2-ylmethyl-[5-(2-methoxy-naphthalen-1-yl)-[1,2,4]triazin-3-yl]-amine

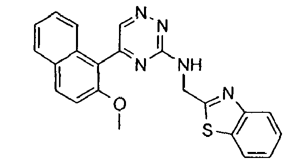

a) Representative procedure for the conversion of aryl methyl ketones into 5-aryl-3-chloro-[1,2,4]-triazine derivatives via 5-aryl-1,2,4-triazin-3(2H)-one intermediates:
   A solution of 1-(2-methoxy-naphthalen-1-yl)-ethanone (1 g, 5 mmol, prepared as in J. Org. Chem. 1995, 60, 3427), selenium dioxide (610 mg, 5.5 mmol) in dioxane (4 mL) and water (0.8 mL) was heated to 105°C for 4 h. The reaction mixture was cooled to room temperature, filtered over a pad of celite and evaporated. The resulting orange oil was dissolved in ethanol (4 mL) and water (4 mL). Semicarbazide hydrochloride (390 mg, 3.5 mmol) and potassium carbonate (690 mg, 5 mmol) were added, and the solution was stirred at room temperature for 1.5 h, then at 105°C overnight. The reaction mixture was filtered on a pad of celite while hot, the reaction vessel being rinsed with hot ethanol. The resulting solution was evaporated. Purification by flash chromatography (silica gel, dichloromethane/methanol 1/0 to 95/5) led to 5-(2-methoxy-1-naphthyl)-1,2,4-triazin-3(2H)-one (377 mg, 30%) as a yellow solid.
   ESI-MS m/z 254 (M+H)⁺.
   Under argon, to a suspension of 5-(2-methoxy-1-naphthyl)-1,2,4-triazin-3(2H)-one (377 mg, 1.5 mmol) in anhydrous chloroform at room temperature were successiveley added phosphorus oxychloride (1.4 mL, 15 mmol) and anhydrous DMF (2 drops). The reaction mixture was heated to 50°C for 1.5 h, then cooled to room temperature and added to a dilute aqueous hydrochloric acid solution cooled to 0°C. The resulting solution was extracted with dichloromethane, The combined organic extracts were dried over sodium sulfate, filtered and evaporated. The crude product was purified by flash chromatography (silica gel, cyclohexane/dichloromethane 1/0 to 1/1 then cyclohexane/ethyl acetate 7/3) to afford 3-chloro-5-(2-methoxynapbthalen-1-yl)-[1,2,4]triazine (162 mg, 40%) as an orange oil.
   ESI-MS m/z 272 and 274 (M+H)⁺.
b) Representative procedure for the synthesis of N-substituted-5-aryl-1,2,4-triazin-3-amine compounds by reaction of 5-aryl-3-chloro-[1,2,4]-triazine derivatives with amines:
   A solution of 3-chloro-5-(2-methoxy-naphthalen-l-yl)-[l,2,4]triazine (108.7 mg, 0.4 mmol), benzothiazol-2-yl-methylamine hydrochloride (120 mg, 0.6 mmol), diisopropylethylamine (175 µL, 1 mmol) in anhydrous acetonitrile (2 mL) was stirred at room temperature for 2 h, then at 85°C over 2 days. An aqueous solution of ammonium chloride was added and the reaction mixture was extracted with ethyl acetate. The combined organic extracts were dried over sodium sulfate, filtered and evaporated. Purification by preparative TLC (silica gel, dichloromethane/methanol 95/5) afforded benzothiazol-2-ylmethyl-[5-(2-methoxy-naphthalen-1-yl)-[1,2,4]triazin-3-yl]-amine (67.1 mg, 42%) as a yellow solid.
   ESI-MS m/z 400 (M+H)⁺.
   ¹H NMR (CDCl₃), δ (ppm): 8.83 (s, 1H), 7.99-7.95 (m, 2H), 7.87 (d, J = 7.6 Hz, 1H), 7.80 (d, J = 8.0 Hz, 1H), 7.58-7.56 (m, 1H), 7.50-7.46 (m, 1H), 7.41-7.29 (m, 4H), 5.19 (br s, 2H), 3.89 (s, 3H).
   In the following examples (example 23 to example 31), the title compounds are prepared according to the procedures described in example 22.
   Titles compounds are prepared by reaction of 5-aryl-3-chloro-[1,2,4]-triazine intermediates with amines according to the representative procedure for the synthesis of N-substituted-5-aryl-1,2,4-triazin-3-amine compounds by reaction of 5-aryl-3-chloro-[1,2,4]-triazine derivatives with amines as described in example 22. The corresponding 5-aryl-3-chloro-[1,2,4]-triazine intermediates are prepared from acetophenone derivatives following the representative procedure described in example 22 for the conversion of aryl methyl ketones into 5-aryl-3-chloro-[1,2,4]-triazine derivatives via 5-aryl-1,2,4-triazin-3(2H)-one intermediates. Acetophenone derivatives are commercially available starting materials or readily synthesized according to literature procedures. Amines are prepared as described above (example 1 and example 15) or commercially available starting materials, or prepared according to literature procedures.

### Example 23:

### N-(1,3-Benzothiazol-2-ylmethyl)-5-(3-methoxy-2-methylphenyl)-1,2,4-triazin-3-amine

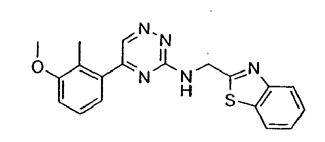

ESI-MS m/z 364 (M+H)⁺.

¹H NMR (CDCl₃), δ (ppm): 8.80 (s, 1H), 7.99 (d, J = 8.0 Hz, 1H), 7.84 (d, J = 8.0 Hz, 1H), 7.47 (t, J = 8.0 Hz, 1H), 7.37 (t, J = 8.0 Hz, 1H), 7.28-7.26 (m, 1H), 7.05 (d, J = 8.0 Hz, 1H), 6.97 (d, J = 8.0 Hz, 1H), 5.18 (s, 2H), 3.86 (s, 3H), 2.22 (s, 3H).

### Example 24:

### N-(1,3-Benzothiazol-2-ylmethyl)-5-(4-methoxy-2-methylphenyl)-1,2,4-triazin-3-amine

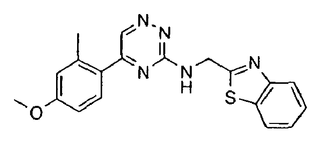

ESI-MS m/z 364 (M+H)⁺.

¹H NMR (CDCl₃), δ (ppm): 8.84 (s, 1H), 8.00 (d, J = 8.0 Hz, 1H), 7.84 (d, J = 8.0 Hz, 1H), 7.52 (d, J = 8.4 Hz, 1H), 7.49-7.45 (m, 1H), 7.39-7.35 (m, 1H), 6.85-6.82 (m, 1H), 6.80 (s, 1H), 5.18 (br s, 2H), 3.84 (s, 3H), 2.43 (s, 3H).

### Example 25:

### N-(1,3-Benzothiazol-2-ylmethyl)-5-(2-methoxy-6-methylphenyl)-1,2,4-triazin-3-amine

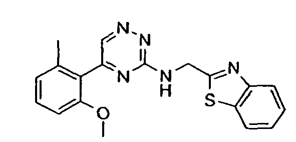

ESI-MS m/z 364 (M+H)⁺.

¹H NMR (CDCl₃), δ (ppm): 8.51 (s, 1H), 7.97 (d, J = 8.0 Hz, 1H), 7.85 (d, J = 8.0 Hz, 1H), 7.50-7.47 (m, 1H), 7.42-7.38 (m, 2H), 6.88-6.86 (m, 2H), 5.21 (d, J = 5.9 Hz, 2H), 3.83 (s, 3H), 2.04 (s, 3H).

### Example 26:

### N-(1,3-Benzothiazol-2-ylmethyl)-5-(2-chloro-6-fluorophenyl)-1,2,4-triazin-3-amine

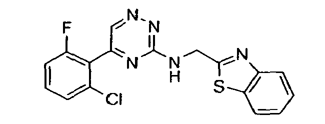

ESI-MS m/z 372 and 374 (M+H)⁺.

¹H NMR (CDCl₃), δ (ppm): 8.79 (d, J = 1.3 Hz, 1H), 8.00 (d, J = 8.0 Hz, 1H), 7.85 (d, J = 8.0 Hz, 1H), 7.48 (td, J = 8.0 Hz and J = 1.3 Hz, 1H), 7.43-7.36 (m, 2H), 7.33-7.31 (m, 1H), 7.13 (t, J = 9.1 Hz, 1H), 6.43 (br s, 1H), 5.20 (s, 2H).

### Example 27:

### 5-(2-Bromophenyl)-N-[(5-methoxy-1,3-benzothiazol-2-yl)methyl]-1,2,4-triazin-3-amine

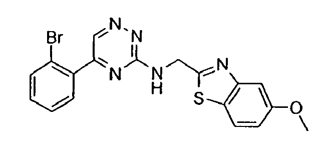

ESI-MS m/z 428 and 430(M+H)⁺.

¹H NMR (CDCl₃), δ (ppm): 9.04 (s, 1H), 7.73-7.67 (m, 2H), 7.57-7.53 (m, 1H), 7.48 (d, J = 2.5 Hz, 1H), 7.43 (t, J = 7.8 Hz, 1H), 7.35 (td, J= 7.8 Hz and J = 1.5 Hz, 1H), 7.02 (dd, J = 8.8 Hz and J = 2.5 Hz, 1H), 5.18 (s, 2H), 3.87 (s, 3H).

### Example 28:

### 5-(4-Bromo-2,6-dimethylphenyl)-N-[(5-methoxy-1,3-benzothiazol-2-yl)methyl]-1,2,4-triazin-3-amine

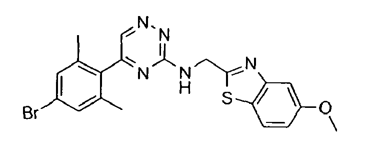

ESI-MS m/z 456 and 458 (M+H)⁺.

¹H NMR (CDCl₃), δ (ppm): 8.59 (s, 1H), 7.68 (d, J = 8.8 Hz, 1H), 7.46 (d, J = 2.4 Hz, 1H), 7.25 (s, 2H), 7.02 (dd, J = 8.8 Hz and 2.4 Hz, 1H), 5.15 (br s, 2H), 3.88 (s, 3H), 2.06 (s, 6H).

### Example 29:

### N-[(5-Methoxy-1,3-benzothiazol-2-yl)methyl]-5(2-methoxy-1-naphthyl)-1,2,4-triazin-3-amine

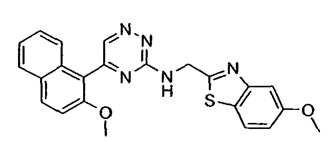

ESI-MS m/z 430 (M+H)⁺.

¹H NMR (CDCl₃), δ (ppm): 8.83 (s, 1H), 7.97 (d, J = 9.2 Hz, 1H), 7.80 (d, J = 8.4 Hz, 1H), 7.70 (d, J = 8.8 Hz, 1H), 7.60-7.55 (m 1H), 7.46 (d, J = 2.4 Hz, 1H), 7.34 (d, J = 8.8 Hz, 1H), 7.34-7.30 (m, 1H), 7.03 (dd, J = 8.8 Hz and 2.4 Hz, 1H), 6.36 (br s, 1H), 5.15 (br s = 2H), 3.89 (s, 3H), 3.88 (s, 3H).

### Example 30:

### 5-(2-Bromo-4-fluorophenyl)-N-[(5-methoxy-1,3-benzothiazol-2-yl)methyl]-1,2,4-triazin-3-amine

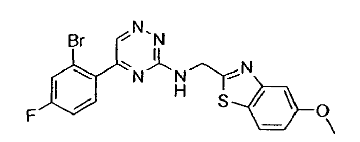

ESI-MS m/z 446 and 448 (M+H)⁺.

¹H NMR (CDCl₃), δ (ppm): 9.09 (s, 1H), 7.73 (d, J = 8.8 Hz, 1H), 7.62 (dd, J = 8.4 Hz and 5.6 Hz, 1H), 7.52 (d, J = 2.4 Hz, 1H), 7.49 (dd, J = 8.0 Hz and 2.4 Hz, 1H), 7.22-7.18 (m, 1H), 7.07 (dd, J = 8.8 Hz and 2.4 Hz, 1H), 5.21 (br s, 2H), 3.92 (s, 3H).

### Example 31:

### 5-(5-Bromo-2-chlorophenyl)-N-[(5-methoxy-1,3-benzothiazol-2-yl)methyl]-1,2,4-triazin-3-amine

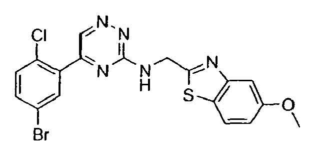

ESI-MS m/z 462, 464 and 466 (M+H)⁺.

¹H NMR (CDCl₃, 300 MHz), δ (ppm): 9.05 (s, 1H), 7.88 (d, J = 8.7 Hz, 1H), 7.85-7.70 (m, 2H), 7.65-7.55 (m, 1H), 7.50 (d, J=2.7 Hz, 1H), 7.05 (dd, J = 8.7 Hz and 2.7 Hz, I H), 5.10-4.90 (br s, 2H), 3.83 (s, 3H).

### Example 32:

### 1-{3-[(Benzothiazol-2-ylmethyl)-amino]-[1,2,4]triazin-5-yl}-naphthalen-2-ol

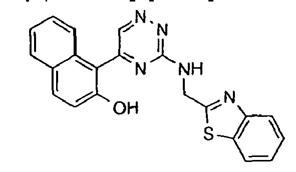

Under argon, to a solution of benzothiazol-2-ylmethyl-[5-(2-methoxy-naphthalen-1-yl)-[1,2,4]triazin-3-yl]-amine (49.5 mg, 0.12 mmol, prepared as in example 22) in anhydrous dichloromethane (0.5 mL) at -78°C was added dropwise a solution of boron tribromide in dichloromethane (1M, 1.25 mL, 1.25 mmol). The reaction mixture was stirred overnight allowing the temperature to raise to room temperature. The solution was cooled to -78°C, then methanol (2 mL) was added and the temperature let to rise. Solvents were evaporated, dichloromethane and an aqueous solution of ammonium chloride were added, and the resulting solution was extracted with dichloromethane. The combined organic extracts were dried over sodium sulfate, filtered and evaporated. The crude product was purified by preparative TLC (silica gel, dichloromethane/methanol 94/6) followed by recristallization in dichloromethane and cyclohexane to afford 1-{3-[(benzothiazol-2-ylmethyl)-amino]-[1,2,4]triazin-5-yl}-naphthalen-2-ol (9.6 mg, 20%) as an orange solid.

ESI-MS m/z 386 (M+H)⁺.

¹H NMR (DMSO-d₆), δ (ppm): 8.90 (s, 1H), 8.09 (d, J = 8.8 Hz, 1H), 7.92-7.74 (m, 3H), 7.62-7.10 (m, 6H), 4.92 (br s, 2H).

### Example 33:

### 2-{[5-(2-Hydroxy-naphthalen-1-yl)-[1,2,4]triazin-3-ylamino]-methyl}-benzothiazol-5-ol

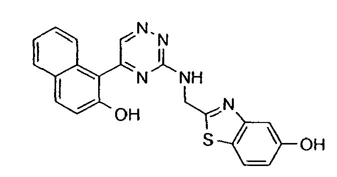

Under argon, to a solution of (5-methoxy-benzothiazol-2-ylmethyl)-[5-(2-methoxy-naphthalen-1-yl)-[1,2,4]triazin-3-yl]-amine (40.7 mg, 0.095 mmol, prepared as in example 29) in anhydrous dichloromethane (1 mL) at -78°C was added dropwise a solution of boron tribromide in dichloromethane (1M, 2 mL, 2 mmol). The reaction mixture was stirred overnight allowing the temperature to rise to room temperature. The solution was cooled to -78°C, then methanol (2 mL) was added and the temperature let to rise. Solvents were evaporated, ethyl acetate and an aqueous solution of ammonium chloride were added, and the resulting solution was extracted with ethyl acetate. The combined organic extracts were dried over sodium sulfate, filtered and evaporated. The crude product was purified by preparative TLC (silica gel, dichloromethane/methanol 95/5) to afford 2-{[5-(2-hydroxy-naphthalen-1-yl)-[1,2,4]triazin-3-ylamino]-methyl}-benzothiazol-5-ol (24.6 mg, 65%) as a yellow solid.

ESI-MS m/z 402 (M+H)⁺.

¹H NMR (DMSO-d₆), δ (ppm): 9.65 (s, 1H), 8.83 (s, 1H), 7.94-7.87 (m, 1H), 7.84-7.80 (m, 2H), 7.30-7.21 (m, 4H), 6.93 (d, J = 8.4 Hz, 1H), 4.92 (br s, 2H).

### Example 34:

### 2-{3-[(1,3-Benzothiazol-2-ylmethyl)amino]-1,2,4-triazin-5-yl}-3-methylphenol

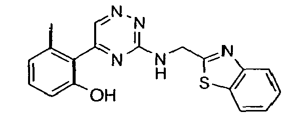

Under argon, to a solution of N-(1,3-benzothiazol-2-ylmethyl)-5-(2-methoxy-6-methylphenyl)-1,2,4-triazin-3-amine (50 mg, 0.14 mmol, prepared as in example 25) in anhydrous dichloromethane (0.2 mL) at -78°C was added dropwise a solution of boron tribromide in dichloromethane (1M, 0.55 mL, 0.55 mmol). The reaction mixture was stirred for 2 hours allowing the temperature to rise to room temperature. The solution was cooled to -78°C, and boron tribromide in dichloromethane (1M, 0.55 mL, 0.55 mmol) was added again. The reaction mixture was stirred overnight allowing the temperature to rise to room temperature. Next the solution was cooled to 0°C, methanol and water were added and the temperature let to rise. The resulting solution was extracted with ethyl acetate. The combined organic extracts were dried over sodium sulfate, filtered and evaporated. The crude product was purified by preparative TLC (silica gel, dichloromethane/methanol 95/5) to afford 2-{3-[(1,3-benzothiazol-2-ylmethyl)amino]-1,2,4-triazin-5-yl}-3-methylphenol (16.9 mg, 35%) as an yellow solid.

ESI-MS m/z 350 (M+H)⁺.

¹H NMR (CDCl₃), δ (ppm): 8.94 (s, 1H), 8.02 (d, J = 8.0 Hz, 1H), 7.86 (d, J = 8.0 Hz, 1H), 7.49 (td, J = 8.1 Hz and J = 1.1 Hz, 1H), 7.39 (td, J = 8.1 Hz and J = 1.1 Hz, 1H), 7.28-7.24 (m, 1H), 6.88-6.84 (m, 2H), 5.17 (br s, 2H), 2.50 (s, 3H).

### Example 35:

### 2-(3-{[(5-Methoxy-1,3-benzothiazol-2-yl)methyl]amino}-1,2,4-triazin-5-yl)-3-methylphenol

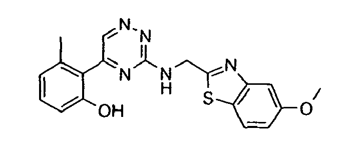

a)
   Under argon, a solution of 3-chloro-5-(2-methoxy-6-methylphenyl)-1,2,4-triazine (50 mg, 0.21 mmol, prepared as in example 25) in anhydrous dichloromethane (1 mL) was cooled to -78°C, and boron tribromide (2mL, 20 mmol) was added dropwise. The reaction mixture was stirred allowing the temperature to raise to room temperature, completion of the reaction was followed by TLC. Water (5 mL) was added, and the mixture was extracted with dichloromethane. Combined organic extracts were washed with an aqueous solution of sodium hydrogencarbonate, then dried over sodium sulfate, filtered and evaporated to give 2-(3-chloro-1,2,4-triazin-5-yl)-3-methylphenol.
b)
   A solution of 2-(3-chloro-1,2,4-triazin-5-yl)-3-methylphenol (16.3 mg, 0.074 mmol) and (5-methoxy-1,3-benzothiazol-2-yl)mcthylamine (28.6 mg, 0.147 mmol, prepared as in example 15) in anhydrous acetonitrile (0.2 mL) was stirred at 85°C for 2 days. The solution was then concentrated. Aqueous hydrochloric acid 1N was added and the solution was extracted with ethyl acetate. Combined organic extracts were dried over sodium sulfate, filtered and evaporated. The residue was purified by preparative TLC (silica gel, dichloromethane/methanol 96/4) to afford 2-(3-{[(5-methoxy-1,3-benzothiazol-2-yl)methyl]amino}-1,2,4-triazin-5-yl)-3-methylphenol (10.5 mg, 38%) as a yellow solid.
   ESI-MS m/z 380 (M+H)⁺.
   ¹H NMR (CDCl₃), δ (ppm): 8.93 (s, 1H), 7.70 (d, J = 8.6 Hz, 1H), 7.50 (d, J = 2.4 Hz, 1H), 7.28-7.24 (m, 1H), 7.04 (dd, J = 8.6 Hz and J = 2.4 Hz, 1H), 6.88-6.83 (m, 2H), 5.14 (br s, 1H), 3.88 (s, 3H), 2.49 (s, 3H).

### Example 36:

### 4-{3-[(1,3-Benzothiazol-2-ylmethyl)amino]-1,2,4-triazin-5-yl}-3-methylphenol

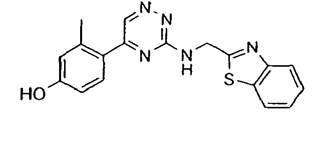

a)
   Under argon, a solution of boron tribromide in dichloromethane (1M, 8 mL, 8 mmol) was added to 3-chloro-5-(4-methoxy-2-methylphenyl)-1,2,4-triazine (162 mg, 0.69 mmol, prepared as in example 24) cooled to -10°C. The reaction mixture was stirred overnight allowing the temperature to raise to room temperature. The mixture was poured in an ice-water bath, and dichloromethane was added. The resulting solution was extracted with dichloromethane, combined organic extracts were dried over sodium sulfate, filtered and evaporated to give crude 4-(3-chloro-1,2,4-triazin-5-yl)-3-methylphenol.
b)
   A solution of 4-(3-chloro-1,2,4-triazin-5-yl)-3-methylphenol (60 mg, 0.27 mmol), benzothiazol-2-yl-methylamine (89 mg, 0.54 mmol), pyridine (28 µL, 0.32 mmol) in anhydrous acetonitrile (0.4 mL) was stirred at 85°C overnight. The solution was then concentrated and the residue was purified by preparative TLC (silica gel, dichloromethane/methanol 92/8) to give 4-{3-[(1,3-benzothiazol-2-ylmethyl)amino]-1,2,4-triazin-5-yl}-3-methylphenol (33.8 mg, 36%) as a yellow oil.
   ESI-MS m/z 350 (M+H)⁺.
   ¹H NMR (CDCl₃), δ (ppm): 8.78 (s, 1H), 7.97 (d, J = 8.1 Hz, 1H), 7.83 (d, J = 8.1 Hz, 1H), 7.48 (t, J = 7.5 Hz, 1H), 7.39-7.31 (m, 2H), 6.77-6.75 (m, 2H), 6.56 (br s, 1H), 5.16 (s, 2H), 2.30 (s, 3H).

### Example 37:

### 1-(4-{3-[(1,3-Benzothiazol-2-ylmethyl)aminol]-1,2,4-triazin-5-yl}-3-methylphenoxy)propan-2-ol

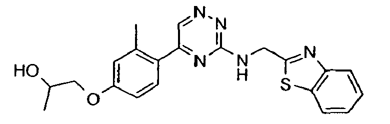

Under argon, a solution of 4-{3-[(1,3-benzothiazol-2-ylmethyl)amino]-1,2,4-triazin-5-yl}-3-methylphenol (15 mg, 0.043 mmol, prepared as in example 36) and potassium carbonate (6 mg, 0.043 mmol), in anhydrous DMF (0.2 mL) was stirred at 50°C for 1 hour. Then propylene oxide (6 µL, 0.086 mmol) was added and the solution was stirred at 50°C overnight. The mixture was then concentrated and the residue was purified by preparative TLC (silica gel, cyclohexane/ethyl acetate/methanol 50/50/2) to afford 1-(4-{3-[(1,3-benzothiazol-2-ylmethyl)amino]-1,2,4-triazin-5-yl}-3-methylphenoxy)propan-2-ol (1.2 mg, 7%).

ESI-MS m/z 408 (M+H)⁺.

¹H NMR (CDCl₃), δ (ppm): 8.84 (s, 1H), 8.00 (d, J = 8.0 Hz, 1H), 7.84 (d, J = 7.5 Hz, 1H), 7.53-7.46 (m, 2H) 7.38 (t, J = 7.5 Hz, 1H), 6.86-6.84 (m, 2H), 5.18 (br s, 2H), 4.25-4.18 (m, 1H), 3.97 (dd, J = 9.1 Hz and J = 3.2 Hz, 1H), 3.87-3.82 (m, 1H), 2.40 (s, 3H), 1.30 (d, J = 6.4 Hz, 3H).

### Example 38:

### 4-(3-{[(5-Methoxy-1,3-benzothiazol-2-yl)methyl]amino}-1,2,4-triazin-5-yl)-3-methylphenol

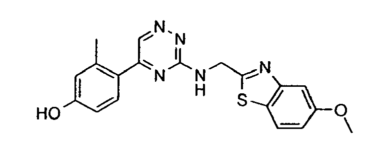

A solution of 4-(3-chloro-1,2,4-triazin-5-yl)-3-methylphenol (73 mg, 0.33 mmol, prepared as in example 36) and (5-methoxy-1,3-benzothiazol-2-yl)methylamine (134 mg, 0.69 mmol, prepared as in example 15) in anhydrous acetonitrile (0.45 mL) was stirred at 85°C overnight. The solution was then concentrated and the residue was purified by preparative TLC (silica gel, dichloromethane/methanol 97/3) to give 4-(3-{[(5-methoxy-1,3-benzothiazol-2-yl)methyl]amino}-1,2,4-triazin-5-yl)-3-methylphenol (4.6 mg, 4%) as a yellow solid.

ESI-MS m/z 380 (M+H)⁺.

¹H NMR (CD₃OD), δ (ppm): 8.75 (s, 1H), 7.77 (d, J = 8.7 Hz, 1H), 7.44 (d, J = 2.4 Hz, 1H), 7.13-7.07 (m, 1H), 7.04 (dd, J = 8.7 Hz and J = 2.4 Hz, 1H), 6.96-6.87 (m, 2H), 5.06 (s, 2H), 3.87 (s, 3H), 2.06 (s, 3H).

### Example 39:

### 3-{3-[(1,3-Benzothiazol-2-yl)amino]-1,2,4-triazin-5-yl}-2-methylphenol

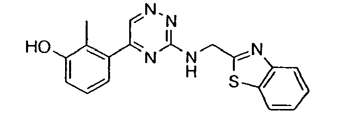

a)
   Under argon a solution of boron tribromide in dichloromethane (1M, 8 mL, 8 mmol) was added 3-chloro-5-(3-methoxy-2-methylphenyl)-1,2,4-triazine (132 mg, 0.56 mmol, prepared as in example 23) cooled to 0°C. The reaction mixture was stirred for 5 hours at 0°C. Dichloromethane was added and the reaction mixture was quenched with water. The solution was extracted with dichloromethane, combined organic extracts were dried over sodium sulfate, filtered and evaporated to give 3-(3-chloro-1,2,4-triazin-5-yl)-2-methylphenol.
b)
   A solution of 3-(3-chloro-1,2,4-triazin-5-yl)-3-methylphenol (20 mg, 0.09 mmol), benzothiazol-2-yl-methylamine (29.6 mg, 0.18 mmol), pyridine (9.3 µL, 0.11 mmol) in anhydrous acetonitrile (0.2 mL) was stirred at 85°C for 3 days. The solution was then concentrated and the residue was purified by preparative TLC (silica gel, dichloromethane/methanol 92/8) to give 3-{3-[(1,3-benzothiazol-2-ylmethyl)amino]-1,2,4-triazin-5-yl}-3-methylphenol (7.7 mg, 24%) as a yellow oil.
   ESI-MS m/z 350 (M+H)⁺.
   ¹H NMR (CDCl₃), δ (ppm): 8.79 (s, 1H), 7.99 (d, J = 7.5 Hz, 1H), 7.84 (d, J = 7.5 Hz, 1H), 7.47 (t, J = 7.5 Hz, 1H), 7.37 (t, J = 7.5 Hz, 1H), 7.14 (t, J = 7.8 Hz, 1H), 7.00 (d, J = 7.8 Hz, 1H), 6.94 (d, J = 7.8 Hz, 1H), 5.18 (s, 2H), 2.22 (s, 3H).

### Example 40:

### N-(1,3-Benzothiazol-2-ylmethyl)-5-(3-ethoxy-2-methylphenyl)-1,2,4-triazin-3-amine

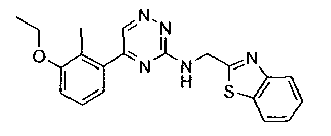

Under argon, a solution of 3-{3-[(1,3-benzothiazol-2-ylmethyl)amino]-1,2,4-triazin-5-yl}-3-methylphenol (20 mg, 0.057 mmol, prepared as in example 39), potassium carbonate (7.9 mg, 0.057 mmol) and tetrabutylammonium iodide (3.7 mg, 0.01 mmol) in anhydrous THF (0.25 mL) was stirred at 50°C for 1 hour. Then ethyl iodide (5.5 µL, 0.068 mL) was added and the reaction mixture was stirred overnight at 50°C. The mixture was then concentrated and the residue was purified by preparative TLC (silica gel, dichloromethane/methanol 95/5) to afford N-(1,3-benzothiazol-2-ylmethyl)-5-(3-ethoxy-2-methylphenyl)-1,2,4-triazin-3-amine (4.4 mg, 20%) as an off-white solid.

ESI-MS m/z 378 (M+H)⁺.

¹H NMR (CDCl₃), δ (ppm): 8.80 (s, 1H), 7.99 (d, J = 7.8 Hz, 1H), 7.85 (d, J = 7.8 Hz, 1H), 7.48 (t, J = 7.8 Hz, 1H), 7.38 (t, J = 7.8 Hz, 1H), 7.27-7.23 (m, 1H), 7.03 (d, J = 7.8 Hz, 1H), 6.96 (d, J = 7.8 Hz, 1H), 5.19 (d, J = 4.3 Hz, 2H), 4.06 (q, J = 6.9 Hz, 2H), 2.24 (s, 3H), 1.44 (t, J = 6.9 Hz, 3H).

In the following examples (example 41 and example 42), 2-(aminomethyl)-1,3-benzothiazol-5-amine was prepared from 2-methyl-5-nitro-1,3-benzothiazole according to the representative procedure for the synthesis of (1,3-benzothiazol-2-yl)methylamine compounds from methyl-1,3-benzothiazole derivatives described in example 15.

### Example 41:

### 2-({[5-(2,6-Dimethoxyphenyl)-1,2,4-triazin-3-yl]amino}methyl)-1,3-benzothiazol-5-amine

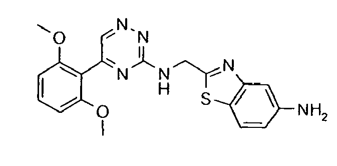

a)
   Under argon, a solution of tert-butyl nitrite (90%, 155 µL, 1.17 mmol) and copper(II) chloride (116 mg, 0.86 mmol) in anhydrous acetonitrile (1 mL) was heated at 65°C, and a solution of 5-(2,6-dimethoxyphenyl)-1,2,4-triazin-3-amine (181 mg, 0.78 mmol, prepared as in example 1) in anhydrous acetonitrile (3 mL) and dichloromethane (2 mL) was added. The reaction mixture was stirred at 65°C for 1.5 hours. The solution was then poured into aqueous hydrochloric acid 6N cooled to 0°C. The mixture was extracted with diethyl ether, combined organic extracts were washed with brine, then dried over sodium sulfate, filtered and evaporated. The residue was purified by flash chromatography (silica gel, cyclohexane/ethyl acetate, 8/2) to afford 3-chloro-5-(2,6-dimethoxyphenyl)-1,2,4-triazine (58 mg, 29%) as a yellow solid.
b)
   Under argon, a solution of 3-chloro-5-(2,6-dimethoxyphenyl)-1,2,4-triazine (60 mg, 0.24 mmol) and 2-(aminomethyl)-1,3-benzothiazol-5-amine (150 mg, 0.72 mmol) in anhydrous acetonitrile (0.5 mL) was stirred at 85°C for 3 days. The mixture was then concentrated and the residue was purified by preparative TLC (silica gel, dichloromethane/methanol 95/5) to afford 2-({[5-(2,6-dimethoxyphenyl)-1,2,4-triazin-3-yl]amino}methyl)-1,3-benzothiazol-5-amine (43.7 mg, 46%) as an orange solid.
   ESI-MS m/z 395 (M+H)⁺.
   ¹H NMR (CDCl₃), δ (ppm): 8.64 (s, 1H), 7.57 (d, J = 8.6 Hz, 1H), 7.36 (t, J = 8.6 Hz, 1H), 7.27-7.25 (m, 1H), 6.70 (dd, J = 8.6 Hz and J = 2.1 Hz, 1H), 6.61 (d, J = 8.6 Hz, 2H), 6.47 (br s, 1H), 5.11 (d, J = 5.4 Hz, 2H), 3.70 (s, 6H).

### Example 42:

### 2-(3-{[(5-Amino-1,3-benzothiazol-2-yl)methyl]amino}-1,2,4-triazin-5-yl)-3-methylphenol

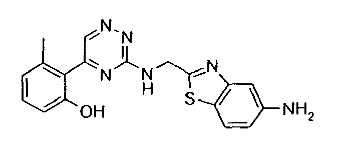

Under argon, a solution of 2-(3-chloro-1,2,4-triazin-5-yl)-3-methylphenol (142.2 mg, 0.64 mmol, prepared as in example 35), 2-(aminomethyl)-1,3-benzothiazol-5-amine (150 mg, 0.84 mmol) and DIPEA (112 µL, 0.64 mmol) in anhydrous acetonitrile (0.8 mL) was stirred at 85°C overnight. The solution was concentrated and the residue was purified by flash chromatography (silica gel, dichloromethane/methanol, 95/5) and preparative TLC (silica gel, dichloromethane/methanol, 95/5) to afford 2-(3-{[(5-amino-1,3-benzothiazol-2-yl)methyl]amino}-1,2,4-triazin-5-yl)-3-methylphenol (110 mg, 47%) as a brown solid.

ESI-MS m/z 365 (M+H)⁺.

¹H NMR (CD₃OD), δ (ppm): 8.67 (s, 1H), 7.58 (d, J = 8.4 Hz, 1H), 7.22 (d, J = 2.1 Hz, 1H), 7.14 (t, J = 8.0 Hz, 1H), 6.85 (dd, J = 8.4 Hz and J = 2.1 Hz, 1H), 6.75-6.70 (m, 2H), 5.00 (br s, 2H), 1.94 (br s, 3H).

### Example 43:

### 3-Methyl-2-{3-[({5-[(1,3-thiazol-2-ylmethyl)amino]-1,3-benzothiazol-2-yl}methyl)amino]-1,2,4-triazin-5-yl}phenol

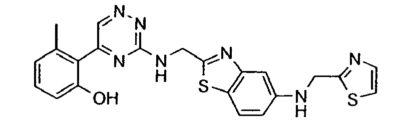

Under argon, a solution of -(3-{[(5-amino-1,3-benzothiazol-2-yl)methyl]amino}-1,2,4-triazin-5-yl)-3-methylphenol (20 mg, 0.055 mmol, prepared as in example 42), 1,3-thiazole-2-carbaldehyde (18.7 mg, 0.165 mmol) and titanium(IV) isopropoxide (48 µL, 0.164 mol) in anhydrous THF (0.25 mL) with 4Å molecular sieves was stirred overnight at room temperature. Sodium borohydride (2.5 mg, 0.066 mmol) was added and the reaction mixture was stirred at room temperature for 4 hours. The solution was filtered over a pad of celite. Ethyl acetate and aqueous ammonium chloride were added to the filtrate. The mixture was extracted with ethyl acetate, combined organic extracts were dried over sodium sulfate, filtered and evaporated. Purification by preparative TLC (silica gel, dichloromethane/methanol, 95/5) led to 3-methyl-2-{3-[({5-[(1,3-thiazol-2-ylmethyl)amino]-1,3-benzothiazol-2-yl}methyl)amino]-1,2,4-triazin-5-yl}phenol (1.8 mg, 7%) as a yellow solid.

ESI-MS m/z 462 (M+H)⁺.

¹H NMR (CD₃OD), δ (ppm): 8.66 (s, 1H), 7.75-7.73 (m, 2H), 7.61 (d = J = 8.8 Hz, 1H), 7.47-7.45 (m, 1H), 7.15 (t, J = 8.0 Hz, 1H), 7.05 (d, J = 2.2 Hz, 1H), 6.85 (dd, J = 8.8 Hz and 2.2 Hz, 1H), 6.73 (d, J = 8.0 Hz, 2H), 4.98 (s, 2H), 4.72 (s, 2H), 2.00 (br s, 3H).

7.58 (d, J = 8.4 Hz, 1H), 7.22 (d, J = 2.1 Hz, 1H), 7.14 (t, J = 8.0 Hz, 1H), 6.85 (dd, J = 8.4 Hz and J = 2.1 Hz, 1H), 6.75-6.70 (m, 2H), 5.00 (br s, 2H), 1.94 (br s, 3H).

### Example 44:

### N-[2-({[5-(2,6-Dimethoxyphenyl)-1,2,4-triazin-3-yl]amino}methyl)-1,3-benzothiazol-5-yl]acetamide

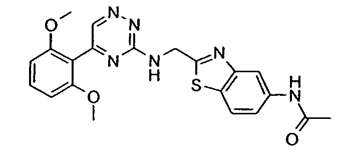

Under argon, a solution of 2-({[5-(2,6-dimethoxyphenyl)-1,2,4-triazin-3-yl]amino}methyl)-1,3-benzothiazol-5-amine (19.4 mg, 0.049 mmol, prepared as in example 41) and pyridine (5 µL, 0.058 mmol) in anhydrous dichloromethane (0.1 mL) was cooled to 0°C. Acetyl chloride (3.5 µL, 0.049 mmol) was added and the reaction mixture was stirred for 5 hours allowing the temperature to raise to room temperature. Then acetyl chloride (3.5 µL, 0.049 mmol) was added again and the reaction mixture was stirred overnight at room temperature. The solution was concentrated, then ethyl acetate and water were added. The mixture was extracted with ethyl acetate, combined organic extracts were dried over sodium sulfate, filtered and evaporated. Purification by preparative TLC (silica gel, dichloromethane/methanol, 95/5) led to N-[2-({[5-(2,6-dimethoxyphenyl)-1,2,4-triazin-3-yl]amino}methyl)-1,3-benzothiazol-5-yl]acetamide (6.8 mg, 32%) as a yellow oil.

ESI-MS m/z 437 (M+H)⁺.

¹H NMR (CDCl₃), δ (ppm): 8.65 (s, 1H), 8.06 (s, 1H), 7.74 (d, J = 8.7 Hz, 1H), 7.64 (d, J = 8.7 Hz, 1H), 7.49 (s, 1H), 7.37 (t, J = 8.6 Hz, 1H), 6.62 (d, J = 8.6 Hz, 2H), 6.35 (br s, 1H), 5.16 (d, J = 5.1 Hz, 2H), 3.71 (s, 6H), 2.22 (s, 3H).

### Example 45:

### N-[2-({[5-(2,6-Dimethoxyphenyl)-1,2,4-triazin-3-yl]amino}methyl)-1,3-benzothiazol-5-yl]methanesulfonamide

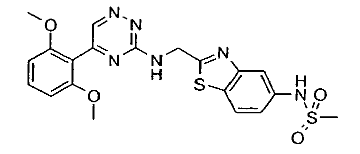

Under argon, a solution of 2-({[5-(2,6-dimethoxyphenyl)-1,2,4-triazin-3-yl]amino}methyl)-1,3-benzothiazol-5-amine (17.4 mg, 0.044 mmol, prepared as in example 41) and pyridine (4 µL, 0.046 mmol) in anhydrous dichloromethane (0.1 mL) was cooled to 0°C. Methanesulfonyl chloride (3.5 µL, 0.045 mmol) was added and the reaction mixture was stirred overnight allowing the temperature to raise to room temperature. The solution was concentrated, then ethyl acetate and water were added. The mixture was extracted with ethyl acetate, combined organic extracts were dried over sodium sulfate, filtered and evaporated. Purification by preparative TLC (silica gel, dichloromethane/methanol, 95/5) led to N-[2-({[5-(2,6-dimethoxyphenyl)-1,2,4-triazin-3-yl]amino}methyl)-1,3-benzothiazol-5-yl]methanesulfonamide (5.2 mg, 25%) as a yellow solid.

ESI-MS m/z 473 (M+H)⁺.

¹H NMR (CDCl₃), δ (ppm): 8.66 (s, 1H), 7.83 (d, J = 2.0 Hz, 1H), 7.78 (d, J = 8.6 Hz, 1H), 7.37 (t, J = 8.3 Hz, 1H), 7.32 (dd, J = 8.6 Hz and J = 2.0 Hz, 1H), 7.13 (s, 1H), 6.63 (d, J = 8.3 Hz, 2H), 6.31 (br s, 1H), 5.17 (d, J = 5.6 Hz, 2H), 3.72 (s, 6H), 3.02 (s, 3H).

### Example 46:

### 5-(2,6-Dimethoxyphenyl)-N-[(5-methoxy-1,3-benzothiazol-2-yl)methyl]-1,2,4-triazin-3-amine

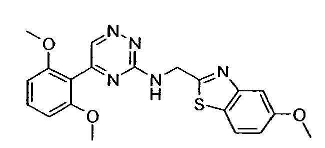

Under argon, a solution of 3-chloro-5-(2,6-dimethoxyphenyl)-1,2,4-triazine (60 mg, 0.24 mmol, prepared as in example 41) and (5-methoxy-1,3-benzothiazol-2-yl)methylamine (139.3 mg, 0.72 mmol, prepared as in example 15) in anhydrous acetonitrile (0.5 mL) was stirred at 85°C for 3 days. The solution was then concentrated and the residue was purified by preparative TLC (silica gel, dichloromethane/methanol 95/5) to give 5-(2,6-dimethoxyphenyl)-N-[(5-methoxy-1,3-benzothiazol-2-yl)methyl]-1,2,4-triazin-3-amine (70 mg, 72%) as a yellow solid.

ESI-MS m/z 410 (M+H)⁺.

¹H NMR (CDCl₃), δ (ppm): 8.65 (s, 1H), 7.69 (d, J = 8.7 Hz, 1H), 7.48 (d, J = 2.4 Hz, 1H), 7.37 (t, J = 8.3 Hz, 1H), 7.02 (dd, J = 8.7 Hz and J = 2.4 Hz, 1H), 6.63 (d, J = 8.3 Hz, 2H), 6.13 (br s, 1H), 5.17 (d, J = 5.6 Hz, 2H), 3.89 (s, 3H), 3.72 (s, 6H).

### Example 47:

### 2-({[5-(2,6-Dimethoxyphenyl)-1,2,4-triazin-3-yl]amino}methyl)-N,N-dimethyl-1,3-benzothiazol-5-amine

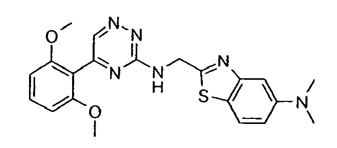

a)
   Under argon, methyl iodide (0.57 mL, 9.2 mmol) was added to a solution of 2-methyl-1,3-benzothiazol-5-amine (1.0 g, 6.1 mmol) and potassium hydroxide (608 mg, 10.8 mmol) in acetone (100 mL) and water (5 mL). The reaction mixture was stirred at room temperature for 2 hours, then methyl iodide (0.57 mL, 9.2 mmol) and potassium hydroxide (343 mg, 6.1 mmol) were added and the reaction mixture was stirred for 3 days at room temperature. Ethyl acetate was added and acetone was evaporated. Water was added and the mixture was extracted with ethyl acetate. The combined organic extracts were dried over sodium sulfate, filtered and evaporated. The residue was purified by flash chromatography (silica gel, dichloromethane/methanol 1/0 to 97/3) and preparative TLC (silica gel, cyclohexane/ethyl acetate 7/3) to furnish N,N,2-trimethyl-1,3-benzothiazol-5-amine (260 mg, 22%) as an off-white solid.
   ESI-MS m/z 193 (M+H)⁺.
b)
   2-(aminomethyl)-N,N-dimethyl-1,3-benzothiazol-5-amine was obtained from N,N,2-trimethyl-1,3-benzothiazol-5-amine according to the representative procedure for the synthesis of (1,3-benzothiazol-2-yl)methylamine compounds from methyl-1,3-benzothiazole derivatives described in example 15.
c)
   Under argon, a solution of 3-chloro-5-(2,6-dimethoxyphenyl)-1,2,4-triazine (22 mg, 0.087 mmol, prepared as in example 41) and 2-(aminomethyl)-N,N-dimethyl-1,3-benzothiazol-5-amine (38 mg, 0.18 mmol) in anhydrous acetonitrile (0.6 mL) was stirred at 85°C overnight. The solution was then concentrated and the residue was purified by preparative TLC (silica gel, dichloromethane/methanol 95/5) to give 2-({[5-(2,6-dimethoxyphenyl)-1,2,4-triazin-3-yl]amino}methyl)-N,N-dimethyl-1,3-benzothiazol-5-amine (8.2 mg, 22%) as a yellow solid.
   ESI-MS m/z 423 (M+H)⁺.
   ¹H NMR (CDCl₃), δ (ppm): 8.60 (s, 1H), 7.63 (d, J = 8.9 Hz, 1H), 7.36 (t, J = 8.4 Hz, 1H), 7.29 (d, J = 2.5 Hz, 1H), 6.90 (dd, J = 8.9 Hz and J = 2.5 Hz, 1H), 6.62 (d, J = 8.4 Hz, 2H), 6.17 (br s, 1H), 5.13 (d, J = 5.6 Hz, 2H), 3.72 (s, 6H), 3.00 (s, 6H).

### Example 48:

### 5-(2,6-Dimethoxyphenyl)-N-[(5,6-dimethyl-1,3-benzothiazol-2-yl)methyl]-1,2,4-triazin-3-amine

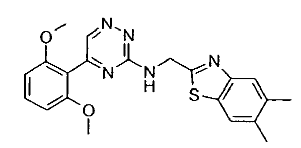

Under argon, a solution of 3-chloro-5-(2,6-dimethoxyphenyl)-1,2,4-triazine (30 mg, 0.12 mmol, prepared as in example 41) and (5,6-dimethyl-1,3-benzothiazol-2-yl)methylamine (69 mg, 0.36 mmol, prepared from 2,5,6-trimethyl-1,3-benzothiazole according to the representative procedure for the synthesis of (1,3-benzothiazol-2-yl)methylamine compounds from methyl-1,3-benzothiazole derivatives described in example 15) in anhydrous acetonitrile (0.25 mL) was stirred at 85°C for 3 days. The solution was then concentrated and the residue was purified by preparative TLC (silica gel, dichloromethane/methanol 95/5) to give 5-(2,6-dimethoxyphenyl)-N-[(5,6-dimethyl-1,3-benzothiazol-2-yl)methyl]-1,2,4-triazin-3-amine (23 mg, 47%) as a yellow solid.

ESI-MS m/z 408 (M+H)⁺.

¹H NMR (CDCl₃), δ (ppm): 8.63 (s, 1H), 7.73 (s, 1H), 7.57 (s, 1H), 7.35 (t, J = 8.4 Hz, 1H), 6.61 (d, J = 8.4 Hz, 2H), 6.35 (br s, 1H), 5.13 (d, J = 5.6 Hz, 2H), 3.70 (s, 6H), 2.38 (s, 3H), 2.36 (s, 3H).

### Example 49:

### 5-(2,6-Dimethoxyphenyl)-N-[(5-methyl-1,3-benzothiazol-2-yl)methyl]-1,2,4-triazin-3-amine

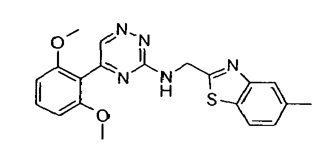

Under argon, a solution of 3-chloro-5-(2,6-dimethoxyphenyl)-1,2,4-triazine (30 mg, 0.12 mmol, prepared as in example 41) and (5-methyl-1,3-benzothiazol-2-yl)methylamine (64 mg, 0.36 mmol, prepared from 2,5-dimethyl-1,3-benzothiazole according to the representative procedure for the synthesis of (1,3-benzothiazol-2-yl)methylamine compounds from methyl-1,3-benzothiazole derivatives described in example 15) in anhydrous acetonitrile (0.3 mL) was stirred at 85°C for 6 hours and then at 50°C for 3 days. The solution was then concentrated and the residue was purified by preparative TLC (silica gel, dichloromethane/methanol 95/5) to give 5-(2,6-dimethoxyphenyl)-N-[(5-methyl-1,3-benzothiazol-2-yl)methyl]-1,2,4-triazin-3-amine (32.4 mg, 69%) as a white solid.

ESI-MS m/z 394 (M+H)⁺.

¹H NMR (CDCl₃), δ (ppm): 8.66 (s, 1H), 7.77 (s, 1H), 7.70 (d, J = 8.3 Hz, 1H), 7.35 (t, J = 8.6 Hz), 7.18 (d, J = 8.3 Hz, 1H), 6.61 (d, J = 8.6 Hz, 2H), 6.45 (br s, 1H), 5.10 (s, 2H), 3.69 (s, 6H), 2.49 (s, 3H).

### Example 50:

### 2-({[5-(2,6-Dimethoxyphenyl)-1,2,4-triazin-3-yl]amino}methyl)-1,3-benzothiazol-6-amine

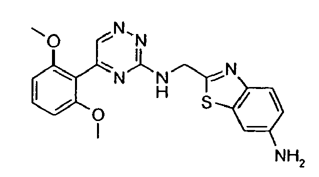

Under argon, a solution of 3-chloro-5-(2,6-dimethoxyphenyl)-1,2,4-triazine (32.3 mg, 0.13 mmol, prepared as in example 41), 2-(aminomethyl)-1,3-benzothiazol-6-amine (30 mg, 0.17 mmol, prepared from 2-methyl-6-nitro-1,3-benzothiazole according to the representative procedure for the synthesis of (1,3-benzothiazol-2-yl)methylamine compounds from methyl-1,3-benzothiazole derivatives described in example 15) and DIPEA (23 µL, 0.13 mmol) in anhydrous acetonitrile (0.6 mL) was stirred at 85°C for 2 days. The solution was then concentrated and the residue was purified by preparative TLC (silica gel, dichloromethane/methanol 95/5) to give 2-({[5-(2,6-dimethoxyphenyl)-1,2,4-triazin-3-yl]amino}methyl)-1,3-benzothiazol-6-amine (32 mg, 63%) as a yellow oil.

ESI-MS m/z 395 (M+H)⁺.

¹H NMR (CDCl₃), δ (ppm): 8.63 (s, 1H), 7.71 (d, J = 8.8 Hz, 1H), 7.35 (t, J = 8.3 Hz, 1H), 7.04 (d, J = 2.3 Hz, 1H), 6.79 (dd, J = 8.8 Hz and J = 2.3 Hz, 1H), 6.61 (d, J = 8.3 Hz, 2H), 6.39 (br s, 1H), 5.07 (d, J = 5.9 Hz, 2H), 3.70 (s, 6H).

### Example 51:

### N-[2-({[5-(2,6-Dimethoxyphenyl)-1,2,4-triazin-3-yl]amino}methyl)-1,3-benzothiazol-6-yl]acetamide

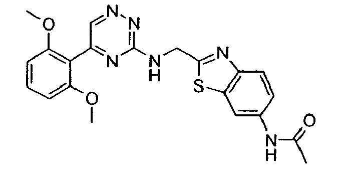

Under argon, a solution of 2-({[5-(2,6-dimethoxyphenyl)-1,2,4-triazin-3-yl]amino}methyl)-1,3-benzothiazol-6-amine (8.0 mg, 0.020 mmol, prepared as in example 50) and pyridine (2.5 µL, 0.029 mmol) in anhydrous dichloromethane (0.5 mL) was cooled to 0°C. Acetyl chloride (1.8 µL, 0.025 mmol) was added and the reaction mixture was stirred overnight at room temperature. The solution was concentrated, and then ethyl acetate and water were added. The mixture was extracted with ethyl acetate, combined organic extracts were dried over sodium sulfate, filtered and evaporated. Purification by preparative TLC (silica gel, dichloromethane/methanol, 95/5) led to N-[2-({(S-(2,6-dimethoxyphenyl)-1,2,4-triazin-3-yl]amino}methyl)-1,3-benzothiazol-6-yl]acetamide (2.6 mg, 29%) as a pale yellow oil.

ESI-MS m/z 437 (M+H)⁺.

¹H NMR (CDCl₃), δ (ppm): 8.65 (s, 1H), 8.38 (d, J = 1.9 Hz, 1H), 7.87 (d, J = 8.6 Hz, 1H), 7.39-7.35 (m, 2H), 6.63 (d, J = 8.6 Hz, 2H,), 6.21 (br s, 1H), 5.15 (d, J = 5.9 Hz, 2H), 3.72 (s, 6H), 2.21 (s, 3H).

### Example 52:

### N-[2-({[5-(2,6-Dimethoxyphenyl)-1,2,4-triazin-3-yl]amino}methyl)-1,3-benzothiazol-6-yl]methanesulfonamide

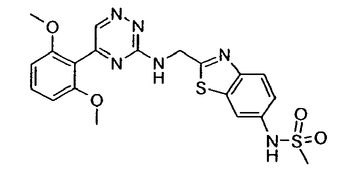

Under argon, a solution of 2-({[5-(2,6-dimethoxyphenyl)-1,2,4-triazin-3-yl]amino}methyl)-1,3-benzothiazol-6-amine (18 mg, 0.046 mmol, prepared as in example 50) and pyridine (4 µL, 0.046 mmol) in anhydrous dichloromethane (0.1 mL) was cooled to 0°C. Methanesulfonyl chloride (3.5 µL, 0.045 mmol) was added and the reaction mixture was stirred overnight allowing the temperature to raise to room temperature. The solution was concentrated, and then ethyl acetate and water were added. The mixture was extracted with ethyl acetate, combined organic extracts were dried over sodium sulfate, filtered and evaporated. Purification by preparative TLC (silica gel, dichloromethane/methanol, 95/5) led to N-[2-({[5-(2,6-dimethoxyphenyl)-1,2,4-triazin-3-yl]amino}methyl)-1,3-benzothiazol-6-yl]methanesulfonamide (3.0 mg, 14%) as a yellow oil.

ESI-MS m/z 473 (M+H)⁺.

¹H NMR (CDCl₃), δ (ppm): 8.67 (s, 1H), 7.90 (d, J = 8.9 Hz, 1H), 7.79 (d, J = 2.2 Hz, 1H), 7.41-7.35 (m, 2H), 6.89 (br s, 1H), 6.64 (d, J = 8.5 Hz, 2H), 6.20 (br s, 1H), 5.18 (d, J = 5.6 Hz, 2H), 3.73 (s, 6H), 3.01 (s, 3H).

### Example 53:

### 2-({[5-(2,6-Dimethoxyphenyl)-1,2,4-triazin-3-yl]amino}methyl)-1,3-benzothiazol-6-ol

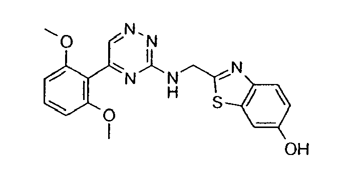

a)
   Under argon, a solution of diisobutylaluminium hydride in toluene (1M, 6.3 mL, 6.3 mmol) was added to a solution of 6-methoxy-1,3-benzothiazole-2-carbonitrile (601 mg, 3.13 mmol) in anhydrous THF (25 mL) cooled to -78°C. The reaction mixture was stirred at -78°C for 2 hours, then diisobutylaluminium hydride in toluene (1M, 6.3 mL, 6.3 mmol) was again added dropwise. The reaction mixture was stirred for 2 hours allowing the temperature to raise to -20°C. An aqueous solution of sodium potassium tartrate was added and the temperature let to rise. The mixture was extracted with diethyl ether and ethyl acetate, combined organic extracts were dried over sodium sulfate, filtered and evaporated. The crude product was dissolved in ethanol (6 mL), hydroxylamine hydrochloride (417 mg, 6 mmol) and sodium methoxide (324 mg, 6 mmol) were added and the reaction mixture was stirred at room temperature overnight. The white solid was filtered off, rinsed with methanol and the filtrate was evaporated. The crude oxime was dissolved in acetic acid (15 mL), zinc dust (1.18 g, 18 mmol) was added portionwise and the reaction mixture was stirred at room temperature for 2 hours. The mixture was then filtered over a pad of celite and rinsed with methanol. Toluene was added and the filtrate was concentrated. Ethyl acetate and water were added. The aqueous layer was acidified to pH = 2 with hydrochloric acid 1N and extracted with diethyl ether and ethyl acetate. These organic layers were discarded. The aqueous layer was basified with aqueous sodium hydroxide to pH = 10, and then extracted with diethyl ether and ethyl acetate. Purification by flash chromatography (silica gel, dichloromethane/methanol, 1/0 to 9/1) led to (6-methoxy-1,3-benzothiazol-2-yl)methylamine (300 mg, 49%) as a light brown oil.
   ESI-MS m/z 195 (M+H)⁺.
b)
   To a solution of (6-methoxy-1,3-benzothiazol-2-yl)methylamine (300 mg, 1.54 mmol) in anhydrous dichloromethane (6 mL) stirred at -78°C under argon, a solution of boron tribromide in heptane (1M, 6.2 mL, 6.2 mmol) was added dropwise. The reaction mixture was stirred for 6 hours allowing the temperature to raise slowly to 0°C. The solution was then cooled to -35°C, methanol was added and the temperature let to rise. The mixture was concentrated, then dichloromethane and water were added. The aqueous layer was basified with an aqueous solution of sodium hydroxide to pH = 12, and the solution was extracted with dichloromethane. These organic layers were discarded. The pH of the aqueous layer was adjusted to 8 with aqueous ammonium chloride. The aqueous layer was extracted with dichloromethane, combined organic extracts were dried over sodium sulfate, filtered and evaporated. Purification by flash chromatography (silica gel, dichloromethane/methanol, 1/0 to 9/1) afforded 2-(aminomethyl)-1,3-benzothiazol-6-ol (40.8 mg, 15%) as an off-white solid.
   ESI-MS m/z 181 (M+H)⁺.
c)
   Under argon, a solution of 3-chloro-5-(2,6-dimethoxyphenyl)-1,2,4-triazine (34.2 mg, 0.136 mmol, prepared as in example 41), 2-(aminomethyl)-1,3-benzothiazol-6-ol (36.8 mg, 0.20 mmol) and pyridine (16.5 µL, 0.20 mmol) in anhydrous acetonitrile (0.75 mL) was stirred at 85°C overnight. The solution was then concentrated and the residue was purified by preparative TLC (silica gel, dichloromethane/methanol 95/5) to give 2-({[5-(2,6-dimethoxyphenyl)-1,2,4-triazin-3-yl]amino}methyl)-1,3-benzothiazol-6-ol (9.4 mg, 17%) as an omage solid.
   ESI-MS m/z 396 (M+H)⁺.
   ¹H NMR (CD₃OD), δ (ppm): 8.52 (s, 1H), 7.72 (d, J = 8.4 Hz, 1H), 7.39 (t, J = 8.4 Hz, 1H), 7.26 (d, J = 2.4 Hz, 1H), 6.97 (dd, J = 8.4 Hz and 2.4 Hz, 1H), 6.71 (d, J = 8.4 Hz, 2H), 4.98 (br s, 2H), 3.64 (br s, 6H).

### Example 54:

### N-[(5-Chloro-1,3-benzothiazol-2-yl)methyl]-5-(2,6-dimethoxyphenyl)-1,2,4-triazin-3-amine

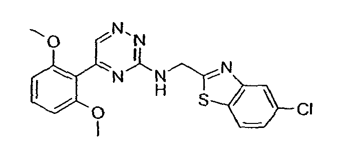

a)
   Under argon, chloroacetyl chloride (160 µL, 2 mmol) was added to a solution of 2-amino-4-chlorobenzenethiol (160 mg, 1 mmol) in anhydrous toluene (3 mL) at room temperature. Triethylamine (140 µL, 1 mmol) was added dropwise and the reaction mixture was stirred at room temperature for 20 minutes, then at 105°C for 2.5 hours. The solution was cooled to room temeprature and then poured into an aqueous solution of sodium hydrogencarbonate. The mixture was extracted with diethyl ether, combined organic extracts were dried over sodium sulfate, filtered and evaporated to give crude 5-chloro-2-(chloromethyl)-1,3-benzothiazole. The latter compound was placed in a screwed-cap tube, and dissolved in THF (0.5 mL). An aqueous ammonium hydroxide solution (33%, 3 mL) and a few milligrams of tetrabutylammonium iodide were added and the reaction mixture was stirred overnight at 60°C. Ethyl acetate was added and ammonia was evaporated. The aqueous layer was acidified to pH = 1 with cold 1N aqueous hydrochloric acid. Extraction with diethyl ether and ethyl acetate gave organic extracts which were discarded. The aqueous layer was basified with aqueous sodium hydroxide to pH = 10, then extracted with diethyl ether and ethyl acetate. Combined organic extracts were dried over sodium sulfate, filtered and evaporated to give crude (5-chloro-1,3-benzothiazol-2-yl)methylamine (32.7 mg, 16%) as a brown solid.
   ESI-MS m/z 199 and 201 (M+H)⁺.
b)
   Under argon, a solution of 3-chloro-5-(2,6-dimethoxyphenyl)-1,2,4-triazine (20 mg, 0.08 mmol, prepared as in example 41), (5-chloro-1,3-benzothiazol-2-yl)methylamine (32.7 mg, 0.16 mmol) and DIPEA (14 µL, 0.08 mmol) in anhydrous acetonitrile (0.5 mL) was stirred at 85°C overnight. An aqueous ammonium chloride solution was added and the mixture was extracted with ethyl acetate. The combined organic extracts were dried over sodium sulfate, filtered and evaporated. Purification by preparative TLC (silica gel, dichloromethane/methanol 95/5) afforded N-[(5-chloro-1,3-benzothiazol-2-yl)methyl]-5-(2,6-dimethoxyphenyl)-1,2,4-triazin-3-amine (15.4 mg, 47%) as a brown solid.
   ESI-MS m/z 414 and 416 (M+H)⁺.
   ¹H NMR (CDCl₃), δ (ppm); 8.59 (s, 1H), 7.96 (d, J = 2.0 Hz, 1H), 7.75 (d, J = 8.4 Hz, 1H), 7.39 (t, J = 8.4 Hz, 1H), 7.34 (dd, J = 8.4 Hz and 2.0 Hz, 1H), 6.62 (d, J = 8.4 Hz, 2H), 5.18 (d, J = 6.0 Hz, 2H), 3.69 (s, 6H).

### Example 55:

### 5-(2,6-Dimethoxyphenyl)-N-{[5-(trifluoromethyl)-1,3-benzothiazol-2-yl]methyl}-1,2,4-triazin-3-amine

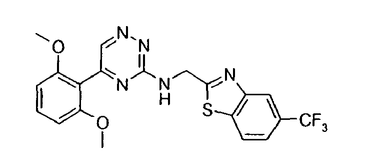

a)
   [5-(trifluoromethyl)-1,3-benzothiazol-2-yl]methylamine was prepared from 2-amino-4-(trifluoromethyl)benzenethiol following the same procedure as in example 54 for the preparation of (5-chloro-1,3-benzothiazol-2-yl)methylamine from 2-amino-4-chlorobenzenethiol.
   ESI-MS m/z 233 (M+H)⁺.
b)
   Under argon, a solution of 3-chloro-5-(2,6-dimethoxyphenyl)-1,2,4-triazine (16.2 mg, 0.064 mmol, prepared as in example 41) and [5-(trifluoromethyl)-1,3-benzothiazol-2-yl]methylamine (60 mg, 0.26 mmol) in anhydrous acetonitrile (0.75 mL) was stirred at 85°C overnight. The solution was then concentrated and the residue was purified by preparative TLC (silica gel, dichloromethane/methanol 95/5) to afford 5-(2,6-dimethoxyphenyl)-N-{[5-(trifluoromethyl)-1,3-benzothiazol-2-yl]methyl}-1,2,4-triazin-3-amine (21.1 mg, 73%)as a yellow solid.
   ESI-MS m/z 448 (M+H)⁺.
   ¹H NMR (CDCl₃), δ (ppm): 8.65 (s, 1H), 8.24 (s, 1H), 7.95 (d, J = 8.4 Hz, 1H), 7.60 (d, J = 8.4 Hz, 1H), 7.37 (t, J = 8.4 Hz, 1H), 6.61 (d, J = 8.4 Hz, 2H), 5.19 (d, J = 5.6 Hz, 2H), 3.69 (s, 6H).

### Example 56:

### N-(1,3-Benzoxazol-2-ylmethyl)-5-(2,6-dimethoxyphenyl)-1,2,4-triazin-3-amine

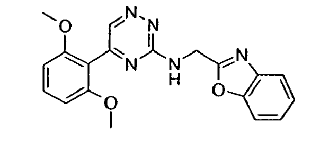

a)
   Under argon, chloroacetyl chloride (122 µL, 1.5 mmol) was added to a solution of 2-aminophenol (111 mg, 1 mmol) in xylenes (4 mL). Triethylamine (153 µL, 1.1 mmol) was next added dropwise. The resulting mixture was stirred for 2 hours at room temperature then at 145°C overnight. An aqueous solution of ammonium chloride was added, and the mixture was extracted with ethyl acetate. The combined organic extracts were washed with brine, then dried over sodium sulfate, filtered and evaporated to give crude 2-(chloromethyl)-1,3-benzoxazole. The latter compound was placed in a screwed-cap tube, and dissolved in THF (0.5 mL). An aqueous ammonium hydroxide solution (33%, 4 mL) was added. The reaction mixture was stirred 30 minutes at room temperature then 22 hours at 60°C. Ethyl acetate was added and ammonia was evaporated. The aqueous layer was acidified to pH = I with cold 1N aqueous hydrochloric acid. Extraction with diethyl ether and ethyl acetate gave organic extracts which were discarded.. The aqueous layer was basified with aqueous sodium hydroxide to pH = 10, then extracted with diethyl ether and ethyl acetate. Combined organic extracts were dried over sodium sulfate, filtered and evaporated to give crude 1,3-benzoxazol-2-ylmethylamine (19.4 mg, 13%) as a brown solid.
   ESI-MS m/z 149 (M+H)⁺.
b)
   Under argon, a solution of 3-chloro-5-(2,6-dimethoxyphenyl)-1,2,4-triazine (16.5 mg, 0.066 mmol, prepared as in example 41), 1,3-benzoxazol-2-ylmethylamine (19.4 mg, 0.13 mmol) and DIPEA (11 µL, 0.063 mmol) in anhydrous acetonitrile (0.3 mL) was stirred at 85°C for 4 hours then at 50°C for 3 days. An aqueous ammonium chloride solution was added and the mixture was extracted with ethyl acetate. The combined organic extracts were dried over sodium sulfate, filtered and evaporated. Purification by preparative TLC (silica gel, dichloromethane/methanol 95/5) afforded N-(1,3-benzoxazol-2-ylmethyl)-5-(2,6-dimethoxyphenyl)-1,2,4-triazin-3-amine (6.1 mg, 26%) as a white solid.
   ESI-MS m/z 364 (M+H)⁺.
   ¹H NMR (CDCl₃), δ (ppm): 8.58 (s, 1H), 7.70-7.68 (m, 1H), 7.52-7.50 (m, 1H), 7.39 (t, J = 8.4 Hz, 1H), 7.34-7.31 (m, 2H), 6.62 (d, J = 8.4 Hz, 2H), 5.07 (d, J = 5.6 Hz, 2H), 3.68 (s, 6H).

### Example 57:

### 5-(2,6-Dimethoxyphenyl)-N-(4,5,6,7-tetrahydro-1,3-benzothiazol-2-ylmethyl)-1,2,4-triazin-3-amine

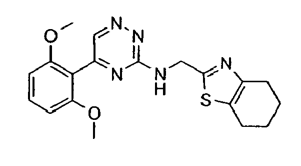

a)
   A solution of 2-chlorocyclohexanone (58 µL, 0.5 mmol), tert-butyl-2-amino-2-thioxoethylcarbamate (105.7 mg, 0.5 mmol) in ethanol (2 mL) was stirred at room temperature for 1.5 hours, then at 80°C for 40 hours. The reaction was then concentrated. Ethyl acetate and a diluted hydrochloric acid solution were added to the crude product. The solution was extracted with diethyl ether and ethyl acetate. These organic layers were discarded. The aqueous layer was basified with aqueous sodium hydroxide to pH = 10, and then extracted with diethyl ether and ethyl acetate. Combined organic extracts were dried over sodium sulfate, filtered and evaporated to give crude 1-(4,5,6,7-tetrahydro-1,3-benzothiazol-2-yl)methanamine (30.9 mg, 36%) as a brown oil which was used in the next steps without purification.
   ESI-MS m/z 169 (M+H)⁺.
b)
   Under argon, a solution of 3-chloro-5-(2,6-dimethoxyphenyl)-1,2,4-triazine (20 mg, 0.08 mmol, prepared as in example 41), 1-(4,5,6,7-tetrahydro-1,3-benzothiazol-2-yl)methanamine (30.9 mg, 0.18 mmol) and triethylamine (11 µL, 0.08 mmol) in anhydrous acetonitrile (0.5 mL) was stirred at 85°C overnight. An aqueous solution of diluted hydrochloric acid was added and the mixture was extracted with ethyl acetate. The combined organic extracts were dried over sodium sulfate, filtered and evaporated. Purification by preparative TLC (silica gel, dichloromethane/methanol 95/5) afforded 5-(2,6-dimethoxyphenyl)-N-(4,5,6,7-tetrahydro-1,3-benzothiazol-2-ylmethyl)-1,2,4-triazin-3-amine (6.6 mg, 22%) as a brown oil.
   ESI-MS m/z 384 (M+H)⁺.
   ¹H NMR (CDCl₃), δ (ppm): 8.50 (s, 1H), 7.46 (t, J = 8.4 Hz, 1H), 6.65 (d, J = 8.4 Hz, 2H), 5.19 (br s, 2H), 3.79 (s, 6H), 2.84 (br s, 2H), 2.74 (br s, 2H), 1.86 (br s, 4H).

### Example 58:

### 5-(2,6-Dimethoxyphenyl)-3-[(4,5-dimethyl-1,3-thiazol-2-yl)methoxy]-1,2,4-triazine

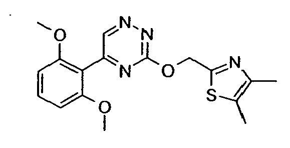

a)
   (4,5-dimethyl-1,3-thiazol-2-yl)methanol was prepared by reduction of 4,5-dimethyl-1,3-thiazole-2-carbaldehyde (prepared as in example 1) with sodium borohydride and purified by flash chromatography.
b)
   Under argon, sodium hydride (60%, 3.1 mg, 0.08 mmol) was added to a solution of (4,5-dimethyl-1,3-thiazol-2-yl)methanol (16.2 mg, 0.11 mmol) in anhydrous THF (1 mL). The reaction mixture was stirred at room temperature for 20 minutes, then 3-chloro-5-(2,6-dimethoxyphenyl)-1,2,4-triazine (10 mg, 0.04 mmol, prepared as in example 41) was added and the resulting mixture was stirred overnight at 70°C. An aqueous ammonium chloride solution was added and the mixture was extracted with diethyl ether and ethyl acetate. The combined organic extracts were dried over sodium sulfate, filtered and evaporated. Purification by preparative TLC (silica gel, dichloromethane/methanol 95/5) afforded 5-(2,6-dimethoxyphenyl)-3-[(4,5-dimethyl-1,3-thiazol-2-yl)methoxy]-1,2,4-triazine (5.4 mg, 38%) as a pale yellow oil.
   ESI-MS m/z 359 (M+H)⁺.
   ¹H NMR (CDCl₃), δ (ppm): 8.99 (s, 1H), 7.40 (t, J = 8.4 Hz, 1H), 6.64 (d, J = 8.4 Hz, 2H), 5.80 (s, 2H), 3.76 (s, 6H), 2.36 (s, 6H).

### Example 59:

### 5-(2,6-Dimethoxyphenyl)-N-[(1,5-dimethyl-1H-pyrazol-3-yl)methyl]-1,2,4-triazin-3-amine

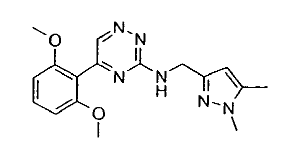

Under argon, a solution of 3-chloro-5-(2,6-dimethoxyphenyl)-1,2,4-triazine (6.85 mg, 0.027 mmol, prepared as in example 41), (1,5-dimethyl-1H-pyrazol-3-yl)methylamine (8 mg, 0.064 mmol) and potassium carbonate (3.8 mg, 0.028 mmol) in anhydrous acetonitrile (0.7 mL) was stirred at 85°C overnight. The solution was evaporated. The residue was purified by preparative TLC (silica gel, dichloromethane/methanol 95/5) to afford 5-(2,6-dimethoxyphenyl)-N-[(1,5-dimethyl-1H-pyrazol-3-yl)methyl]-1,2,4-triazin-3-amine (5.5 mg, 59%) as an off-white solid.

ESI-MS m/z 341 (M+H)⁺.

¹H NMR (CDCl₃), δ (ppm): 8.45 (s, 1H), 7.40 (t, J = 8.4 Hz, 1H), 6.64 (d, J = 8.4 Hz, 2H), 6.06 (s, 1H), 4.70 (d, J = 5.2 Hz, 2H), 3.77 (s, 6H), 3.73 (s, 3H), 2.23 (s, 3H).

### Example 60:

### 5-(2,6-Dimethoxyphenyl)-N-[1-(4,5-dimethyl-1,3-thiazol-2-yl)ethyl]-1,2,4-triazin-3-amine

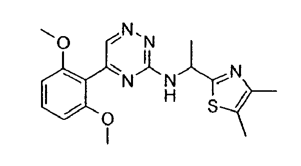

a)
   Under argon, to a solution of 4,5-dimethylthiazole (580 µL, 5.32 mmol) in anhydrous THF (35 mL) at -78°C, *n*-butyllithium (2.3 M in hexane, 3.3 mL, 7.59 mmol) in anhydrous THF (5 mL) was slowly added and the reaction mixture was stirred at -78°C for one hour. Then a solution of anhydrous N,N-dimethylacetamide (1.16 mL, 12.48 mmol) in anhydrous THF (10 mL) was added. The resulting mixture was stirred for 3 hours, allowing the temperature to raise to -40°C. Methanol and an aqueous solution of ammonium chloride were added, and the temperature let to raise to room temperature. The resulting solution was extracted with diethyl ether and ethyl acetate. The combined organic extracts were dried over sodium sulfate, filtered and evaporated. The crude product was purified by flash chromatography (silica gel, cyclohexane/ethyl acetate, 1/0 to 9/1) to afford 1-(4,5-dimethyl-1,3-thiazol-2-yl)ethanone (814 mg, 98%) as a white solid.
   ESI-MS m/z 156 (M+H)⁺.
b)
   Under argon, a solution of 1-(4,5-dimethyl-1,3-thiazol-2-yl)ethanone (155 mg, 1 mmol), hydroxylamine hydrochloride (142 mg, 2 mmol) and sodium methoxide (108 mg, 2 mmol) in ethanol (2 mL) was stirred at room temperature overnight. The white solid was filtered off, rinsed with methanol and the solution was evaporated. The crude oxime was dissolved in acetic acid (6 mL), zinc dust (392 mg, 6 mmol) was added portionwise at room temperature and the reaction mixture was stirred at room temperature for 3.5 hours. The mixture was then filtered over a pad of celite and rinsed with methanol. Toluene was added and the filtrate was concentrated. An aqueous solution of ammonium chloride was added, the solution was acidified to pH = 1 with hydrochloric acid 1N and extracted with diethyl ether and ethyl acetate. These organic layers were discarded. The aqueous layer was basified with aqueous sodium hydroxide to pH = 10, and then extracted with diethyl ether and ethyl acetate. Combined organic extracts were dried over sodium sulfate, filtered and evaporated to give crude 1-(4,5-dimethyl-1,3-thiazol-2-yl)ethanamine (67.2 mg, 43%) as a pale yellow oil which was used in the next steps without purification.
   ESI-MS m/z 157 (M+ H)⁺.
c)
   Under argon, a solution of 3-chloro-5-(2,6-dimethoxyphenyl)-1,2,4-triazine (8.2 mg, 0.033 mmol, prepared as in example 41), 1-(4,5-dimethyl-1,3-thiazol-2-yl)ethanamine (10 mg, 0.064 mmol) and potassium carbonate (5.3 mg, 0.038 mmol) in anhydrous acetonitrile (0.7 mL) was stirred at 85°C overnight. The solution was evaporated. The residue was purified by preparative TLC (silica gel, dichloromethane/methanol 95/5) to give 5-(2,6-dimethoxyphenyl)-N-[1-(4,5-dimethyl-1,3-thiazol-2-yl)ethyl]-1,2,4-triazin-3-amine (3.6 mg, 30%) as a pale yellow oil.
   ESI-MS m/z 372 (M+H)⁺.
   ¹H NMR (CDCl₃), δ (ppm): 8.51 (s, 1H), 7.41 (t, J = 8.4 Hz, 1H), 6.64 (d, J = 8.4 Hz, 2H), 5.63-5.58 (m, 1H), 3.76 (s, 6H), 2.32 (s, 6H), 1.77 (d, J = 6.8 Hz, 3H).

### Example 61:

### 2-{1-[5-(2,6-Dimethoxyphenyl)-1,2,4-triazin-3-yl]piperidin-2-yl}-1,3-benzothiazole

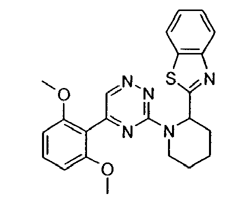

Under argon, a solution of 3-chloro-5-(2,6-dimethoxyphenyl)-1,2,4-triazine (15.4 mg, 0.061 mmol, prepared as in example 41), 2-piperidin-2-yl-1,3-benzothiazole (20 mg, 0.092 mmol) and DIPEA (11 µL, 0.063 mmol) in anhydrous acetonitrile (0.25 mL) was stirred at 85°C overnight. The solution was then concentrated and the residue was purified by preparative TLC (silica gel, dichloromethane/methanol 95/5) to give 2-{1-[5-(2,6-dimethoxyphenyl)-1,2,4-triazin-3-yl]piperidin-2-yl}-1,3-benzothiazole (9.8 mg, 37%) as a yellow oil.

ESI-MS m/z 434 (M+H)⁺.

¹H NMR (CDCl₃), δ (ppm): 8.59 (s, 1H), 7.99 (d, J = 7.8 Hz, 1H), 7.83 (d, J = 7.8 Hz, 1H), 7.45 (t, J = 8.4 Hz, 1H), 7.39-7.32 (m, 2H), 7.60 (d, J = 8.4 Hz, 2H), 6.55 (br s, 1H), 5.04-5.00 (m, 1H), 3.67 (s, 6H), 3.36-3.28 (m, 1H), 2.79-2.74 (m, 1H), 2.06-2.02 (m, 1H), 1.80-1.69 (m, 4H).

### Example 62:

### 2-{1-[5-(2,6-Dimethoxyphenyl)-1,2,4-triazin-3-yl]pyrrolidin-2-yl}-1,3-benzothiazole

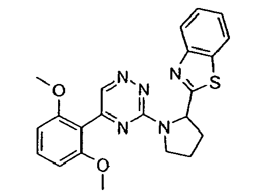

Under argon, a solution of 3-chloro-5-(2,6-dimethoxyphenyl)-1,2,4-triazine (16.4 mg, 0.065 mmol, prepared as in example 41), 2-pyrrolidin-2-yl-1,3-benzothiazole hydrochloride (23.5 mg, 0.098 mmol) and DIPEA (28 µL, 0.16 mmol) in anhydrous acetonitrile (0.25 mL) was stirred at 85°C overnight. The solution was then concentrated and the residue was purified by preparative TLC (silica gel, dichloromethane/methanol 95/5) to afford 2-{1-[5-(2,6-dimethoxyphenyl)-1,2,4-triazin-3-yl]pyrrolidin-2-yl}-1,3-benzothiazole (26.8 mg, 98%) as a pale yellow oil.

ESI-MS m/z 420 (M+H)⁺.

¹H NMR (CDCl₃), δ (ppm): 8.58 (s, 1H), 7.95 (d, J = 8.0 Hz, 1H), 7.80 (d, J = 8.0 Hz, 1H), 7.43 (t, J= 7.9 Hz, 1H), 7.32 (t, J = 8.0 Hz, 2H), 6.53 (d, J = 7.9 Hz, 2H), 5.75 (br s, 1H), 4.15 (br s, 1H), 3.89 (br s, 1H), 3.45 (br s, 6H), 2.54-2.41 (m, 2H), 2.30-2.01 (m, 2H).

### Example 63:

### N-[(4-Methoxy-1,3-benzothiazol-2-yl)methyl]-5-[2-(trifluoromethyl)phenyl]-1,2,4-triazin-3-amine

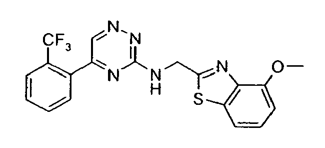

a)
   A solution of N-(2-methoxyphenyl)acetamide (869 mg, 5 mmol) and Lawesson's reagent (1.23 g, 3 mmol) in anhydrous toluene (50 mL) was stirred at 110°C under argon for 3 hours. The reaction mixture was concentrated. The crude product was purified by flash chromatography (silica gel, cyclohexane/ethyl acetate, 9/1 to 7/3) to afford N-(2-Methoxy-phenyl)-thioacetamide contaminated by traces Lawesson's reagent as a yellow oil. This oil was diluted with ethanol (0.5 mL), and mixed with a solution of sodium hydroxide (1.44 g, 36 mmol) in water (3.5 mL). The resulting solution was added dropwise (over a period of 20 minutes) to a solution of potassium ferricyanide (III) (5.3 g, 16 mmol) in water (1.5 mL) stirred at 90°C. The reaction mixture was kept stirring at 90°C for 50 minutes after completion of the addition. The mixture was cooled to room temperature and filtered. The solid was rinsed with water, then extracted with ethyl acetate. The aqueous layer was also extracted with ethyl acetate. Combined organic extracts were dried over sodium sulfate, filtered and evaporated. Purification by preparative TLC (silica gel, cyclohexane/ethyl acetate 7/3) afforded 4-methoxy-2-methyl-1,3-benzothiazole (89.3 mg, 10%) as a beige solid.
   ESI-MS m/z 180 (M+H)⁺.
b)
   A solution of 4-methoxy-2-methyl-1,3-benzothiazole (89.3 mg, 0.5 mmol) and selenium dioxide (111 mg, 1 mmol) in 1,4-dioxane (1.5 mL) was stirred under argon at 100°C for 3 hours. The reaction mixture was filtered and rinsed with hot 1,4-dioxane, and the filtrate was evaporated to give crude 4-methoxy-1,3-benzothiazole-2-carbaldehyde. Ethanol (1 mL) was added followed by hydroxylamine hydrochloride (69.5 mg, 1 mmol) and sodium methoxide (54 mg, I mmol), and the resulting mixture was stirred at room temperature under argon overnight. The solution was filtered and the solid rinsed with methanol, and the filtrate was concentrated. The obtained crude oxime was dissolved in acetic acid (3 mL), zinc dust (196 mg, 3 mmol) was added portionwise at room temperature and the reaction mixture was kept stirring at room temperature for 3 hours. The mixture was then filtered over a pad of celite, and rinsed with methanol. Toluene was added and the filtrate was concentrated. An aqueous solution of ammonium chloride was added, the solution was acidified to pH = 2 with hydrochloric acid 1N and extracted with diethyl ether and ethyl acetate. These organic layers were discarded. The aqueous layer was basified with aqueous sodium hydroxide to pH = 10, and then extracted with diethyl ether and ethyl acetate. Combined organic extracts were dried over sodium sulfate, filtered and evaporated to give crude (4-methoxy-1,3-benzothiazol-2-yl)methylamine (29.2 mg, 30%) as a brown solid which was used in the next steps without purification.
   ESI-MS m/z 195 (M+H)⁺.
c)
   Under argon, a solution of 3-chloro-5-[2-(trifluoromethyl)phenyl]-1,2,4-triazine (39 mg, 0.15 mmol, prepared as in example 13), (4-methoxy-1,3-benzothiazol-2-yl)methylamine (29.2 mg, 0.15 mmol) and DIPEA (26 µL, 0.15 mmol) in anhydrous acetonitrile (0.5 mL) was stirred at room temperature for 2.5 hours, then at 85°C overnight and at 50°C for 24 hours. The solution was then concentrated and the residue was purified by preparative TLC (silica gel, dichloromethane/methanol 95/5) to give N-[(4-methoxy-1,3-benzothiazol-2-yl)methyl]-5-[2-(trifluoromethyl)phenyl]-1,2,4-triazin-3-amine (51.1 mg, 81%) as a yellow solid.
   ESI-MS m/z 418 (M+H)⁺.
   ¹H NMR (CDCl₃), δ (ppm): 8.82 (s, 1H), 7.82-7.80 (m, 1H), 7.68-7.60 (m, 2H), 7.52-7.50 (m, 1H), 7.41 (dd, J = 8.0 Hz and 0.8 Hz, 1H), 7.33-7.29 (m, 1H), 6.90 (d, J = 7.6 Hz, 1H), 5.21 (br s, 2H), 4.04 (s, 3H).

### Example 64:

### 2-[({5-[2-(Trifluoromethyl)phenyl]-1,2,4-triazin-3-yl}amino)methyl]-1,3-benzothiazol-4-ol

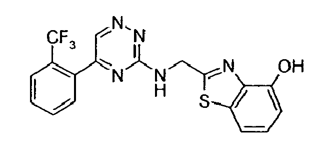

Under argon, a solution of boron tribromide in heptane (1M, 300 µL, 0.3 mmol) was slowly added to a solution of N-[(4-methoxy-1,3-benzothiazol-2-yl)methyl]-5-[2-(trifluoromethyl)phenyl]-1,2,4-triazin-3-amine (27.1 mg, 0.065 mmol, prepared as in example 63) in anhydrous dichloromethane (0.8 mL) at -78°C. The reaction mixture was stirred overnight allowing the temperature to raise to room temperature. The solution was then cooled to -50°C and methanol was added. The solution was kept stirring allowing the temperature to raise to room temperature, and then was evaporated. An aqueous solution of hydrochloric acid was added and the mixture was extracted with dichloromethane. The combined organic extracts were dried over sodium sulfate, filtered and evaporated. Purification by preparative TLC (silica gel, dichloromethane/methanol 95/5) afforded 2-[({5-[2-(trifluoromethyl)phenyl]-1,2,4-triazin-3-yl}amino)methyl]-1,3-benzothiazol-4-ol (10.9 mg, 42%) as an off-white solid.

ESI-MS m/z 404 (M+H)⁺.

¹H NMR (CDCl₃), δ (ppm): 8.83 (s, 1H), 7.84-7.82 (m, 1H), 7.70-7.63 (m, 2H), 7.53-7.51 (m, 1H), 7.33 (d, J = 7.8 Hz, 1H), 7.29-7.25 (m, 1H), 6.98 (dd, J = 7.8 Hz and 1.0 Hz, 1H), 5.17 (br s, 2H).

### Example 65:

### N-[(5,6-Difluoro-1,3-benzothiazol-2-yl)methyl]-5-(2,6-dimethoxyphenyl)-1,2,4-triazin-3-amine

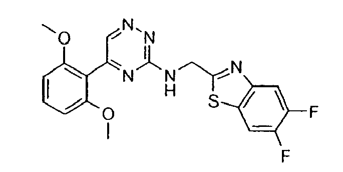

a)
   At room temperature, a solution of 3,4-difluoroaniline (100 µL, 1 mmol) and N-iodosuccinimide (225 mg, 1 mmol) in anhydrous dichloromethane (4 mL) was stirred overnight under argon atmosphere. Then an aqueous solution of sodium sulfite was added and the reaction mixture was extracted with dichloromethane. The combined organic extracts were dried over sodium sulfate, filtered and evaporated. The crude product was purified by flash chromatography (silica gel, cyclohexane/ethyl acetate, 1/0 to 8/2) to afford 4,5-difluoro-2-iodoaniline (175 mg, 69%) as a red oil.
   ESI-MS m/z 255.9 (M+H)⁺.
b) Representative procedure for the palladium catalyzed synthesis of (1,3-thiazol-2-yl)methylamine compounds
   A solution of 4,5-difluoro-2-iodoaniline (167 mg, 0.65 mmol) in anhydrous acetonitrile (0.7 mL) was added to the reaction mixture containing tris(dibenzylidene acetone)dipalladium(0) (13.8 mg, 0.015 mmol), dppf (33 mg, 0.06 mmol), calcium (II) oxide (56.1 mg, 1 mmol) and tert-butyl-2-amino-2-thioxoethylcarbamate (150 mg, 0.79 mmol) in degassed anhydrous acetonitrile (0.3 mL). Then the reaction mixture was degassed, placed under argon atmosphere and stirred at 60°C overnight. The solution was filtered over a pad of celite, rinsed with dichloromethane, methanol, ethyl acetate, and evaporated. Purification by preparative TLC (silica gel, dichloromethane/methanol 95/5) led to tert-butyl (5,6-difluoro-1,3-benzothiazol-2-yl)methylcarbamate (19.2 mg). The latter compound was dissolved in ethyl acetate (0.9 mL) and hydrochloric acid (37%, 0.3 mL) was slowly added at 0°C. The reaction mixture was vigorously stirred for 20 minutes at 0°C and then 15 minutes at room temperature. Water and ethyl acetate were added, extraction with diethyl ether and ethyl acetate gave organic layers which were discarded. The aqueous layer was basified with aqueous sodium hydroxide to pH = 10, then extracted with diethyl ether and ethyl acetate. Combined organic extracts were dried over sodium sulfate, filtered and evaporated to give crude (5,6-difluoro-1,3-benzothiazol-2-yl)methylamine (8.5 mg, 6%) as a red solid. ESI-MS m/z 201 (M+H)⁺.
c)
   Under argon, a solution of 3-chloro-5-(2,6-dimethoxyphenyl)-1 ,2,4-triazine (13 mg, 0.052 mmol, prepared as in example 41), (5,6-difluoro-1,3-benzothiazol-2-yl)methylamine (8.5 mg, 0.043 mmol) and DIPEA (7 µL, 0.04 mmol) in anhydrous acetonitrile (0.2 mL) was stirred at 85°C overnight. The solution was then concentrated and the residue was purified by preparative TLC (silica gel, dichloromethane/methanol 94/6) to give N-[(5,6-difluoro-1 ,3-benzothiazol-2-yl)methyl]-5-(2,6-dimethoxyphenyl)-1,2,4-triazin-3-amine (9.0 mg, 51 %) as a yellow solid.
   ESI-MS m/z 416 (M+H)⁺.
   ¹H NMR (CDCl₃), δ (ppm): 8.67 (s, 1H), 7.76 (dd, J = 10.4 Hz and 7.2 Hz, 1H), 7.61 (dd, J = 9.2 Hz and 7.6 Hz, 1H), 7.37 (t, J = 8.4 Hz, 1H), 6.63 (d, J = 8.4 Hz, 2H), 5.15 (d, J = 5.6 Hz, 2H), 3.72 (s, 6H).

### Example 66:

### Methyl 2-({[5-(2,6-dimethoxyphenyl)-1,2,4-triazin-3-yl]amino}methyl)-1,3-benzothiazole-6-carboxylate

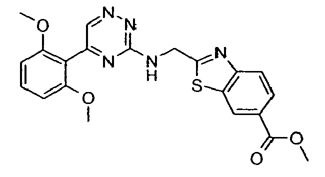

a)
   Methyl 2-(aminomethyl)-1,3-benzothiazole-6-carboxylate was prepared from methyl 4-amino-3-iodobenzoate according to the representative procedure for the palladium catalyzed synthesis of (1,3-thiazol-2-yl)methylamine compounds described in example 65. ESI-MS m/z 223 (M+H)⁺.
b)
   Under argon, a solution of 3-chloro-5-(2,6-dimethoxyphenyl)-1,2,4-triazine (21.5 mg, 0.085 mmol, prepared as in example 41), methyl 2-(aminomethyl)-1,3-benzothiazole-6-carboxylate (17.8 mg, 0.080 mmol) and DIPEA (14 µL, 0.08 mmol) in anhydrous acetonitrile (0.5 mL) was stirred at room temperature for 1 hour, then at 85°C overnight. The solution was then concentrated and the residue was purified by preparative TLC (silica gel, dichloromethane/methanol 94/6) to give methyl 2-({[5-(2,6-dimethoxyphenyl)-1,2,4-triazin-3-yl]amino} methyl)-1,3-benzothiazole-6-carboxylate (15 mg, 43%) as a yellow oil.
   ESI-MS m/z 438 (M+H)⁺.
   ¹H NMR (CDCl₃), δ (ppm): 8.66 (s, 1H), 8.57 (d, J = 1.6 Hz, 1H), 8.14 (dd, J = 8.6 Hz and 1.6 Hz, 1H), 8.00 (d, J = 8.6 Hz, 1H), 7.36 (t, J = 8.6 Hz, 1H), 6.61 (d, J = 8.6 Hz, 2H), 5.19 (d, J = 5.6 Hz, 2H), 3.95 (s, 3H), 3.70 (s, 6H).

### Example 67:

### N-[(6-Chloro-1,3-benzothiazol-2-yl)methyl]-5-(2,6-dimethoxyphenyl)-1,2,4-triazin-3-amine

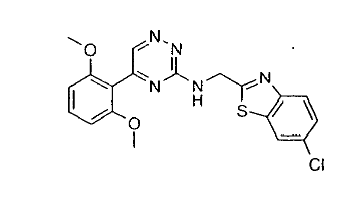

a)
   (6-Chloro-1,3-benzothiazol-2-yl)methylamine was prepared from 4-chloro-2-iodoaniline according to the representative procedure for the palladium catalyzed synthesis of (1,3-thiazol-2-yl)methylamine compounds described in example 65.
   ESI-MS m/z 199 and 201 (M+H)⁺.
b)
   Under argon, a solution of 3-chloro-5-(2,6-dimethoxyphenyl)-1,2,4-triazine (51 mg, 0.20 mmol, prepared as in example 41), (6-chloro-1,3-benzothiazol-2-yl)methylamine (60 mg, 0.30 mmol) and DIPEA (70 µL, 0.4 mmol) in anhydrous acetonitrile (0.4 mL) was stirred at 85°C overnight. The solution was then concentrated and the residue was purified by preparative TLC (silica gel, dichloromethane/methanol 95/5) to give N-[(6-chloro-1,3-benzothiazol-2-yl)metbyl]-5-(2,6-dimethoxyphenyl)-1,2,4-triazin-3-amine (23 mg, 27%) as a yellow solid.
   ESI-MS m/z 414 and 416 (M+H)⁺.
   ¹H NMR (CDCl₃), δ (ppm): 8.66 (s, 1H), 7.87 (d, J = 8.6 Hz, 1H), 7.79 (d, J = 2.0 Hz, 1H), 7.42 (dd, J = 8.6 Hz and J = 2.0 Hz, 1H), 7.34 (t, J = 8.6 Hz, 1H), 6.65 (br s, 1H), 6.59 (d, J = 8.6 Hz, 2H), 5.10 (d, J = 5.6 Hz, 2H), 3.60 (s, 6H).

### Example 68:

### N-[(6-Chloro-4-fluoro-1,3-benzothiazol-2-yl)methyl]-5-(2,6-dimethoxyphenyl)-1,2,4-triazin-3-amine

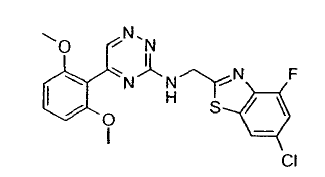

a)
   (6-chloro-4-fluoro-1,3-benzothiazol-2-yl)methylamine was prepared from 4-chloro-2-fluoro-6-iodoaniline according to the representative procedure for the palladium catalyzed synthesis of (1,3-thiazol-2-yl)methylamine compounds described in example 65. ESI-MS m/z 217 and 219 (M+H)⁺.
b)
   Under argon, a solution of 3-chloro-5-(2,6-dimethoxyphenyl)-1,2,4-triazine (93 mg, 0.37 mmol, prepared as in example 41), (6-chloro-4-fluoro-1,3-benzothiazol-2-yl)methylamine (80 mg, 0.37 mmol) and DIPEA (65 µL, 0.37 mmol) in anhydrous acetonitrile (0.7 mL) was stirred at 85°C overnight. The solution was then concentrated and the residue was purified by preparative TLC (silica gel, dichloromethane/methanol 95/5) to give N-[(6-chloro-4-fluoro-1,3-benzothiazol-2-yl)methyl]-5-(2,6-dimethoxypheny)-1,2,4-triazin-3-amine (60.5 mg, 38%) as a yellow solid.
   ESI-MS m/z 432 and 434 (M+H)⁺,
   ¹H NMR (CDCl₃), δ (ppm): 8.65 (s, 1H), 7.58 (d, J = 1.9 Hz 1H), 7.35 (t, J = 8.3 Hz, 1H), 7.19 (dd, J = 9.9 Hz and J = 1.9 Hz, 1H), 6.67 (br s, 1H), 6.60 (d, J = 8.3 Hz, 2H), 5.12 (d, J = 5.9 Hz, 2H), 3.69 (s, 6H).

### Example 69:

### 5-(2-Methoxy-6-methylphenyl)-N-([1,3]thiazolo[4,5-c]pyridin-2-ylmethyl)-1,2,4-triazin-3-amine

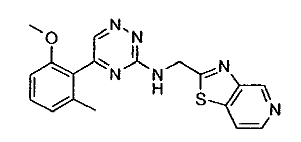

a)
   1-[1,3]Thiazolo[4,5-c]pyridin-2-ylmethanamine was prepared from 3-amino-4-iodopyridine according to the representative procedure for the palladium catalyzed synthesis of (1,3-thiazol-2-yl)methylamine compounds described in example 65.
   ESI-MS m/z 166 (M+H)⁺.
b)
   Under argon, a solution of 3-chloro-5-(2-methoxy-6-methylphenyl)-1,2,4-triazine (28.5 mg, 0.12 mmol, prepared as in example 25), 1-[1,3]thiazolo[4,5-c]pyridin-2-ylmethanamine (20 mg, 0.12 mmol) and triethylamine (17 µL, 0.12 mmol) in anhydrous acetonitrile (0.15 mL) was stirred at 85°C for 3 days. The solution was then concentrated and the residue was purified by preparative TLC (silica gel, dichloromethane/methanol 95/5) to give 5-(2-methoxy-6-methylphenyl)-N-([1,3]thiazolo[4,5-c]pyridin-2-ylmethyl)-1,2,4-triazin-3-amine (9.2 mg, 21%) as a yellow oil.
   ESI-MS m/z 365 (M+H)⁺.
   ¹H NMR (CDCl₃), δ (ppm): 9.29 (s, 1H), 8.72 (s, 1H), 8.52 (d, J = 5.3 Hz, 1H), 7.80 (d, J = 5.3 Hz, 1H), 7.31 (t, J = 7.8 Hz, 1H), 6.87 (d, J = 7.8 Hz, 1H), 6.82 (d, J = 7.8 Hz, 1H), 6.30 (br s, 1H), 5.20 (d, J = 5.3 Hz, 2H), 3.74 (s, 3H), 2.11 (br s, 3H).

### Example 70:

### 5-(2-Methoxy-6-methylphenyl)-N-([1,3]thiazolo[5,4-b]pyridin-2-ylmethyl)-1,2,4-triazin-3-amine

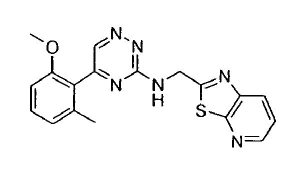

a)
   1-[1,3]Thiazolo[5,4-b]pyridin-2-ylmethanamine was prepared from 3-amino-2-bromopyridine according to the representative procedure for the palladium catalyzed synthesis of (1,3-thiazol-2-yl)methylamine compounds described in example 65.
   ESI-MS m/z 166 (M+H)⁺.
b)
   Under argon, a solution of 3-chloro-5-(2-methoxy-6-methylphenyl)-1,2,4-triazine (43 mg, 0.18 mmol, prepared as in example 25), 1-[1,3]thiazolo[5,4-b]pyridin-2-ylmethanamine (60 mg, 0.36 mmol) and triethylamine (25 µL, 0.18 mmol) in anhydrous acetonitrile (0.3 mL) was stirred at 85°C for 3 days. The solution was then concentrated and the residue was purified by preparative TLC (silica gel, dichloromethane/methanol 95/5) to give 5-(2-methoxy-6-methylphenyl)-N-([1,3]thiazolo[5,4-b]pyridin-2-ylmethyl)-1,2,4-triazin-3-amine (57.8 mg, 87%) as a yellow oil.
   ESI-MS m/z 365 (M+H)⁺.
   ¹H NMR (CDCl₃), δ (ppm): 8.68 (s, 1H), 8.55 (d, J = 4.5 Hz, 1H), 8.19 (d, J = 8.0 Hz, 1H), 7.40 (dd, J = 8.0 Hz and J = 4.5 Hz, 1H), 7.29-7.25 (m, 1H), 6.84 (d, J = 8.0 Hz, 1H), 6.79 (d, J = 8.0 Hz, 1H), 5.10 (br s, 2H), 3.71 (s, 3H), 2.10 (br s, 3H).

### Example 71:

### [2-({[5-(2,6-Dimethoxyphenyl)-1,2,4-triazin-3-yl]amino}methyl)-1,3-benzothiazol-6-yl]methanol

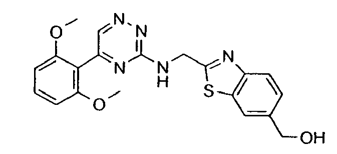

A solution of Dibal-H in toluene (1M, 110µL, 0.11 mmol) was added to a solution of methyl 2-({[5-(2,6-dimethoxyphenyl)-1,2,4-triazin-3-yl]amino}methyl)-1,3-benzothiazole-6-carboxylate (11.8 mg, 0.027 mmol, prepared as in example 66) in anhydrous dichloromethane (0.6 mL) stirred at -78°C under argon atmosphere. The reaction mixture was stirred for 2 hours allowing the temperature to raise slowly to -45°C. A drop of methanol was added followed by a 1M aqueous solution of sodium potassium tartrate. The temperature was allowed to raise to room temperature, then the reaction mixture was extracted with dichloromethane and ethyl acetate. Combined organic extracts were dried over sodium sulfate, filtered and evaporated. Purification by preparative TLC (silica gel, dichloromethane/methanol 95/5) afforded [2-({[5-(2,6-dimethoxyphenyl)-1,2,4-triazin-3-yl]amino}methyl)-1,3-benzothiazol-6-yl]methanol (5.3 mg, 48%) as a beige solid.

ESI-MS m/z 410 (M+H)⁺.

¹H NMR (CDCl₃), δ (ppm): 8.66 (s, 1H), 7.95 (d, J = 8.4 Hz, 1H), 7.87 (d, J = 1.6 Hz, 1H), 7.45 (dd, J = 8.4 Hz and 1.6 Hz, 1H), 7.37 (t, J = 8.4 Hz, 1H), 6.63 (d, J = 8.4 Hz, 2H), 5.19 (d, J = 5.6 Hz, 2H), 4.82 (s, 2H), 3.72 (s, 6H).

### Example 72:

### 5-(2-Methoxy-6-methylphenyl)-N-(4,5,6,7-tetrahydro[1,3]thazolo[4,5-c]pyridin-2-ylmethyl)-1,2,4-triazin-3-amine

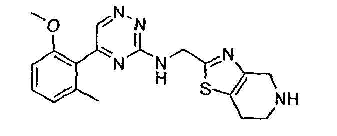

a)
   A solution of 3-amino-4-iodopyridine (500 mg, 2.27 mmol), tris(dibenzylideneacetone)dipalladium(0) (63.6 mg, 0.07 mmol), dppf (155 mg, 0.28 mmol), calcium (II) oxide (255 mg, 4.55 mmol) and tert-butyl-2-amino-2-thioxoethylcarbamate (600 mg, 3.15 mmol) in anhydrous acetonitrile (4.5 mL) was degassed and stirred under argon atmosphere at 60°C overnight. The solution was filtered over a pad of celite, rinsed with dichloromethane, methanol, ethyl acetate, and evaporated. Purification by flash chromatography (silica gel, dichloromethane/methanol 1/0 to 95/5) led to tert-butyl [1,3]thiazolo[4,5-c]pyridin-2-ylmethylcarbamate (500 mg, 83%) as a yellow oil.
   ESI-MS m/z 266 (M+H)⁺.
b)
   Under argon, methyl iodide (100 µL, 1.6 mmol) was added to a solution of tert-butyl [1,3]thiazolo[4,5-c]pyridin-2-ylmethylcarbamate (200 mg, 0.75 mmol) in anhydrous DMF (12 mL). The reaction mixture was stirred at 80°C for 4 hours. The solution was concentrated. Water (10 mL) was added to the residue, and sodium borohydride (82 mg, 2.17 mmol) was added portionwise at 0°C. The resulting mixture was stirred at room temperature for 1 hour. Aqueous sodium hydroxide 1N was added and the solution was extracted with ethyl acetate. The combined organic extracts were dried over sodium sulfate, filtered and evaporated. Purification by preparative TLC (silica gel, dichloromethane/methanol, 95/5) afforded tert-butyl (5-methyl-4,5,6,7-tetrahydro[1,3]thiazolo[4,5-c]pyridin-2-yl)methylcarbamate (160 mg, 75%) as a yellow oil.
   ESI-MS m/z 284 (M+H)⁺.
c)
   Tert-butyl (5-methyl-4,5,6,7-tetrahydro[1,3]thiazolo[4,5-c]pyridin-2-yl)methylcarbamate (160 mg, 0.57 mmol) was dissolved in ethyl acetate (2 mL) and hydrochloric acid (37%, 1.1 mL) was slowly added at 0°C. The reaction mixture was vigorously stirred for 20 minutes at 0°C and then 15 minutes at room temperature. Water and ethyl acetate were added, extraction with diethyl ether and ethyl acetate gave organic layers which were discarded. The aqueous layer was basified with aqueous sodium hydroxide to pH = 10, then extracted with diethyl ether and ethyl acetate. Combined organic extracts were dried over sodium sulfate, filtered and evaporated. The residue was purified by preparative TLC (silica gel, dichloromethane/methanol/aqueous ammonium hydroxide 33%, 95/5/1) to give (5-methyl-4,5,6,7-tetrahydro[1,3]thiazolo[4,5-c]pyridin-2-yl)methylamine (30 mg, 29%) as a yellow oil.
   ESI-MS m/z 184 (M+H)⁺.
d)
   Under argon, a solution of 3-chloro-5-(2-methoxy-6-methylphenyl)-1,2,4-triazine (30 mg, 0.127 mmol, prepared as in example 25), (5-methyl-4,5,6,7-tetrahydro[1,3]thiazolo[4,5-c]pyridin-2-yl)methylamine (30 mg, 0.164 mmol) and triethylamine (18 µL, 0.130 mmol) in anhydrous acetonitrile (0.6 mL) was stirred at 85°C for 3 days. The solution was then concentrated and the residue was purified by preparative TLC (silica gel, dichloromethane/methanol 95/5) to afford 5-(2-methoxy-6-methylphenyl)-N-(4,5,6,7-tetrahydro[1,3]thiazolo[4,5-c]pyridin-2-ylmethyl)-1,2,4-triazin-3-amine (6.4 mg, 14%) as a yellow oil.
   ESI-MS m/z 369 (M+H)⁺.
   ¹H NMR (CDCl₃), δ (ppm): 8.60 (s, 1H), 7.32 (t, J = 8.0 Hz, 1H), 6.91 (d, J = 8.0 Hz, 1H), 6.84 (d, J = 8.0 Hz, 1H), 5.02 (br s, 2H), 4.32-4.29 (m, 2H), 4.13 (br s, 2H), 3.76 (s, 3H), 2.98-2.95 (m, 2H), 2.24 (s, 3H).

### Example 73:

### (4-{3-[(5-Methoxy-benzothiazol-2-ylmethyl)-amino]-[1,2,4]triazin-5-yl}-3,5-dimethyl-phenyl)-carbamic acid tert-butyl ester

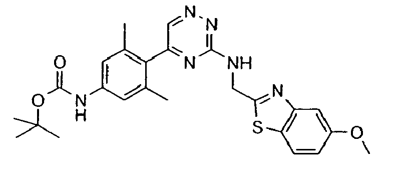

a)
   Under argon, to a solution of 1-(4-amino-2,6-dimethyl-phenyl)-ethanone (2.95 g, 18 mmol, prepared as in *J. Org. Chem*. **1953,** *18*, 496) in THF (36 mL) cooled to 0°C, were successively added triethylamine (2.5 mL, 18 mmol), and di(tert-butyl) dicarbonate (3.95 g, 18 mmol). The reaction mixture was stirred overnight allowing the temperature to raise to room temperature, then heated to 50°C for 2 h. The solvent was evaporated, and an aqueous solution of ammonium chloride was added. The resulting solution was extracted with diethyl ether and ethyl acetate. The combined organic extracts were dried over sodium sulfate, filtered and evaporated. The crude product was purified by flash chromatography (silica gel, cyclohexane/ethyl acetate 1/0 to 1/1) to afford (4-acetyl-3,5-dimethyl-phenyl)-carbamic acid tert-butyl ester (3.538 g, 74%) as a white solid
   ESI-MS m/z 286 (M+Na)⁺.
b) Representative procedure for the conversion of aryl methyl ketones into 3-amino-5-aryl-[1,2,4]-triazine derivatives:
   A solution of (4-acetyl-3,5-dimethyl-phenyl)-carbamic acid tert-butyl ester (1.317 g, 5 mmol), selenium dioxide (777 mg, 7 mmol) in dioxane (4 mL) and water (0.8 mL) was heated to 105°C for 6 h. The reaction mixture was cooled to room temperature, filtered over a pad of celite and evaporated. The resulting oil was dissolved in ethanol (5 mL) and water (0.8 mL). Aminoguanidine hydrochloride (480 mg, 4.3 mmol) and potassium carbonate (828 mg, 6 mmol) were added, and the solution was stirred at room temperature for 1 h, then at 95°C overnight. The reaction mixture was filtered on a pad of celite while hot, the reaction vessel being rinsed with hot ethanol. The resulting solution was evaporated. Purification by flash chromatography (silica gel, dichloromethane/methanol 1/0 to 95/5) led to [4-(3-amino-[1,2,4]triazin-5-yl)-3,5-dimethyl-phenyl]-carbamic acid tert-butyl ester (541 mg, 34%) as an orange solid.
   ESI-MS m/z 316 (M+H)⁺.
c) Representative procedure for the conversion of 3-amino-[1,2,4]-triazine derivatives into 3-chloro-[1,2,4]-triazine derivatives:
   Under argon, tert-butyl nitrite (90%, 365 µL, 2.76 mmol) was added to a solution of [4-(3-amino-[1,2,4]triazin-5-yl)-3,5-dimethyl-phenyl]-carbamic acid tert-butyl ester (541 mg, 1.72 mmol) and copper(II) chloride (300 mg, 2.23 mmol) in anhydrous acetonitrile (5 mL). The resulting mixture was heated to 70°C for 1 h. After cooling to room temperature, cold 3N aqueous hydrochloric acid (5 mL) was added and the mixture was extracted with diethyl ether and ethyl acetate. The combined organic extracts were dried over sodium sulfate, filtered and evaporated. The crude product was purified by flash chromatography (silica gel, cyclohexane/dichloromethane 1/0 to 1/1, then cyclohexane/ethyl acetate 7/3) to give [4-(3-chloro-[1,2,4]triazin-5-yl)-3,5-dimethyl-phenyl]-carbamic acid tert-butyl ester (173 mg, 30%) as a yellow solid.
   ESI-MS m/z 335 and 337 (M+H)⁺.
d)
   According to the experimental procedure used in example 22 for the synthesis of N-substituted-5-aryl-1,2,4-triazin-3-amine compounds by reaction of 5-aryl-3-chloro-[1,2,4]-triazine derivatives with amines, reaction of [4-(3-chloro-[1,2,4]triazin-5-yl)-3,5-dimethyl-phenyl]-carbamic acid tert-butyl ester (173 mg, 0.52 mmol) with (5-methoxy-benzothiazol-2-yl)-methylamine (152 mg, 0.78 mmol, prepared as in example 15) afforded (4-{3-[(5-methoxy-benzothiazol-2-ylmethyl)-amino]-[1,2,4]triazin-5-yl}-3,5-dimethyl-phenyl)-carbamic acid tert-butyl ester (207 mg, 81 %) as a beige solid.
   ESI-MS m/z 493 (M+H)⁺.
   ¹H NMR (CDCl₃), δ (ppm): 8.59 (s, 1H), 7.68 (d, J = 8.8 Hz, 1H), 7.47 (d, J = 2.4 Hz, 1H), 7.14 (s, 2H), 7.02 (dd, J = 8.8 Hz and 2.4 Hz, 1H), 5.14 (br s, 2H), 3.88 (s, 3H), 2.08 (s, 6H), 1.52 (s, 9H).

### Example 74:

### [5-(4-Amino-2,6-dimethyl-phenyl)-[1,2,4]triazin-3-yl]-(5-methoxy-benzothiazol-2-ylmethyl)-amine

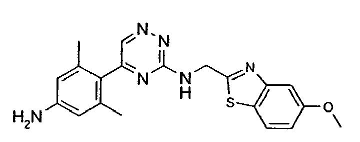

Under argon, to 4-{3-[(5-methoxy-benzothiazol-2-ylmethyl)-amino]-[1,2,4]triazin-5-yl}-3,5-dimethyl-phenyl)-carbamic acid tert-butyl ester (200 mg, 0.4 mmol, prepared as in example 73) cooled to 0°C was added a mixture of 37% aqueous hydrochloric acid (0.75 mL) and ethyl acetate (2.25 mL). The reaction mixture was stirred for 1 h allowing the temperature to raise to room temperature. A dilute aqueous solution of sodium hydroxyde was added until neutral or slightly basic pH was reached, then the reaction mixture was extracted with ethyl acetate. The combined organic extracts were dried over sodium sulfate, filtered and evaporated. The crude product was purified by preparative TLC (silica gel, dichloromethane/methanol 94/6 ) to afford [5-(4-amino-2,6-dimethyl-phenyl)-[1,2,4]triazin-3-yl]-(5-methoxy-benzothiazol-2-ylmethyl)-amine (138.4 mg, 87%) as a yellow solid.

ESI-MS m/z 393 (M+H)⁺.

¹H NMR (DMSO-d₆), δ (ppm): 8.63 (s, 1H), 7.88 (d, J = 8.8 Hz, 1H), 7.47 (s, 1H), 7.04 (d, J = 8.8 Hz, 1H), 6.28 (br s, 2H), 4.90 (br s, 2H), 3.83 (s, 3H), 1.94 (br s, 6H).

### Example 75:

### 2-Dimethylamino-N-(4-{3-[(5-methoxy-benzothiazol-2-ylmethyl)-amino]-[1,2,4]triazin-5-yl}-3,5-dimethyl-phenyl)-acetamide

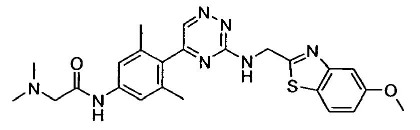

Under argon, triethylamine (70 µL, 0.5 mmol) was added to a solution of [5-(4-amino-2,6-dimethyl-phenyl)-[1,2,4]triazin-3-yl]-(5-methoxy-benzothiazol-2-ylmethyl)-amine (40 mg, 0.1 mmol, prepared as in example 74), 2-chloro-1-methylpyridinium iodide (77 mg, 0.3 mmol) and dimethylamino-acetic acid (16 mg, 0.15 mmol) in anhydrous dichloromethane (0.7 mL). The reaction mixture was stirred at room temperature for 2 days. An aqueous solution of sodium hydroxide and ammonia was then added and the solution was extracted with dichloromethane. The combined organic extracts were dried over sodium sulfate, filtered and evaporated. The crude product was purified by preparative TLC (silica gel, dichloromethane/methanol/aqueous ammonium hydroxide 33%, 94/6/1) to afford 2-dimethylamino-N-(4-{3-[(5-methoxy-benzothiazol-2-ylmethyl)-amino]-[1,2,4]triazin-5-yl}-3,5-dimethyl-phenyl)-acetamide (7.4 mg, 15%).

ESI-MS m/z 478 (M+H)⁺.

¹H NMR (CDCl₃), δ (ppm): 8.60 (s, 1H), 7.68 (d, J = 8.8 Hz, 1H), 7.47 (d, J = 2.4 Hz, 1H), 7.38 (s, 2H), 7.02 (dd, J = 8.8 Hz and 2.4 Hz, 1H), 5.14 (br s, 2H), 3.88 (s, 3H), 3.08 (s, 2H), 2.38 (s, 6H), 2.10 (s, 6H).

### Example 76:

### 2-Amino-N-[3,5-dimethyl-4-(3-{[(5-methoxy-1,3-benzothiazol-2-yl)methyl]amino}-1,2,4-triazin-5-yl)phenyl]acetamide

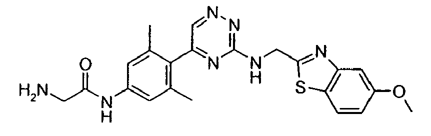

a)
   Under argon, triethylamine (70 µL, 0.5 mmol) was added to a solution of [5-(4-amino-2,6-dimethyl-phenyl)-[1,2,4]triazin-3-yl]-(5-methoxy-benzothiazol-2-ylmethyl)-amine (40 mg, 0.1 mmol, prepared as in example 74), 2-chloro-1-methyl-pyridinium iodide (77 mg, 0.3 mmol) and [(tert-butoxycarbonyl)amino]acetic acid (26.8 mg, 0.15 mmol) in anhydrous dichloromethane (0.7 mL). The reaction mixture was stirred at room temperature for 2 days. An aqueous solution of sodium hydroxide was then added and the solution was extracted with dichloromethane. The combined organic extracts were dried over sodium sulfate, filtered and evaporated. The crude product was purified by preparative TLC (silica gel, dichloromethane/methanol 95/5) to afford tert-butyl [(4-{3-[(5-methoxy-benzothiazol-2-ylmethyl)-amino]-[1,2,4]triazin-5-yl}-3,5-dimethyl-phenylcarbamoyl)-methyl]-carbamate (17 mg, 30%).
   ESI-MS m/z 550 (M+H)⁺.
b)
   Under argon, to tert-butyl [(4-{3-[(5-methoxy-benzothiazol-2-ylmethyl)-amino]-[1,2,4]triazin-5-yl}-3,5-dimethyl-phenylcarbamoyl)-methyl]-carbamate (17 mg, 0.031 mmol), was added a mixture of ethyl acetate (185 µL) and aqueous hydrochloric acid (37%, 65 µL). The resulting mixture was stirred at room temperature for 1 hour. Then an aqueous solution of sodium hydroxide was added to basify until pH = 10. The mixture was then extracted with dichloromethane, combined organic extracts were dried over sodium sulfate, filtered and evaporated. Purification by preparative TLC (silica gel, dichloromethane/methanol/ ammonium hydroxide 33% 95/5/1) led to 2-amino-N-[3,5-dimethyl-4-(3-{[(5-methoxy-1,3-benzothiazol-2-yl)methyl]amino}-1,2,4-triazin-5-yl)phenyl]acetamide (0.7 mg, 5%) as a pale yellow oil.
   ESI-MS m/z 450 (M+H)⁺.
   ¹H NMR (CDCl₃), δ (ppm): 9.41 (s, 1H), 8.61 (s, 1H), 7.68 (d, J = 8.6 Hz, 1H), 7.47 (s, 1H), 7.39 (s, 2H), 7.02 (d, J = 8.6 Hz, 1H), 5.15 (s, 2H), 3.88 (s, 3H), 3.48 (s, 2H), 2.10 (s, 6H).

### Example 77:

### N-[4-(3-{[(5-Methoxy-1,3-benzothiazol-2-yl)methyl]amino}-1,2,4-triazin-5-yl)-3,5-dimethylphenyl]methanesulfonamide

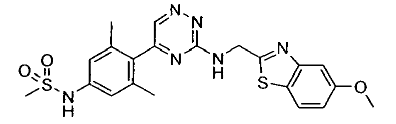

Under argon, a solution of [5-(4-amino-2,6-dimethyl-phenyl)-[1,2,4]triazin-3-yl]-(5-methoxy-benzothiazol-2-ylmethyl)-amine (23 mg, 0.059 mmol, prepared as in example 74) and pyridine (4 µL, 0.046 mmol) in anhydrous dichloromethane (0.1 mL) was cooled to 0°C. Methanesulfonyl chloride (3.5 µL, 0.045 mmol) was added and the reaction mixture was stirred overnight allowing the temperature to raise to room temperature. The solution was concentrated, then ethyl acetate and water were added. The mixture was extracted with ethyl acetate, combined organic extracts were dried over sodium sulfate, filtered and evaporated. Purification by preparative TLC (silica gel, dichloromethane/methanol, 97/3) led N-[4-(3-{[(5-methoxy-1,3-benzothiazol-2-yl)methyl]amino}-1,2,4-triazin-5-yl)-3,5-dimethylphenyl]-methanesulfonamide (4.2 mg, 15%) as a yellow solid.

ESI-MS m/z 471 (M+H)⁺.

¹H NMR (CDCl₃), δ (ppm): 8.56 (s, 1H), 7.68 (d, J = 8.7 Hz, 1H), 7.46 (d, J = 1.9 Hz, 1H), 7.03 (dd, J = 8.7 Hz and J = 1.9 Hz, 1H), 6.98 (s, 2H), 5.16 (s, 2H), 3.87 (s, 3H), 3.06 (s, 3H), 2.06 (s, 6H).

### Example 78:

### N,N-Dimethyl-N'-[4-(3-{[(5-methoxy-1,3-benzothiazol-2-yl)methyl]amino}-1,2,4-triazin-5-yl)phenyl]ethane-1,2-diamine

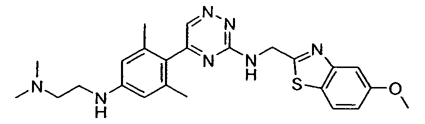

Under argon, 2-chloro-N,N-dimethylethanamine hydrochloride (17.6 mg, 0.14 mmol), sodium hydrogencarbonate (19.7 mg, 0.23 mmol) and a few milligrams of sodium iodide were successively added to a solution of [5-(4-amino-2,6-dimethylphenyl)-[1,2,4]triazin-3-yl]-(5-methoxy-benzothiazol-2-ylmethyl)-amine (40 mg, 0.1 mmol, prepared as in example 74) in ethanol (0.5 mL). The reaction mixture was stirred at 80°C overnight. The solution was then dried over potassium carbonate, filtered and concentrated. Purification by preparative TLC (silica gel, dichloromethane/methanol 95/5) led to N,N-dimethyl-N'-[4-(3-{[(5-methoxy-1,3-benzothiazol-2-yl)methyl]amino}-1,2,4-triazin-5-yl)phenyl]ethane-1,2-diamine (1.7 mg, 4%) as a yellow solid.

ESI-MS m/z 464 (M+H)⁺.

¹H NMR (CD₃OD), δ (ppm): 8.57 (s, 1H), 7.79 (d, J = 8.9 Hz, 1H), 7.43 (d , J = 2.2 Hz, 1H), 7.07 (dd, J = 8.9 Hz and J = 2.2 Hz, 1H), 6.49 (s, 2H), 5.10 (s, 2H), 3.87 (s, 3H), 3.57 (t, J = 5.9 Hz, 2H), 3.37 (t, J = 5.9 Hz, 2H), 2.94 (s, 6H), 2.06 (br s, 6H).

### Example 79:

[5-(4-Bromo-2,6-dimethyl-phenyl)-[1,2,4]triazin-3-yl]-(5-methox-benzothiazol-2-ylmethyl)-amine 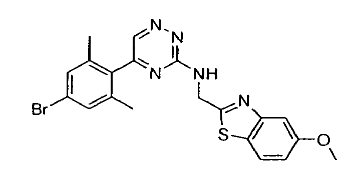
a)
   According to the experimental procedure used in example 22 for the conversion of aryl methyl ketones into 5-aryl-3-chloro-[1,2,4]-triazine derivatives, 1-(4-bromo-2,6-dimethyl-phenyl)-ethanone (2.27 g, 10 mmol, prepared as in J. Chem.. Soc. Perkin Trans. 2 1988, 943) led to 5-(4-bromo-2,6-dimethyl-phenyl)-3-chloro-[1,2,4]triazine (647.5 mg, 22%) as a yellow solid.
   ESI-MS m/z 298, 300 and 302 (M+H)⁺.
b)
   According to the experimental procedure used in example 22 for the reaction of 3-chloro-[1,2,4]-triazine derivatives with amines, reaction of 5-(4-bromo-2,6-dimethyl-phenyl)-3-chloro-[1,2,4]triazine (647.5 mg, 2.17 mmol) with (5-methoxy-benzothiazol-2-yl)-methylamine (464 mg, 2.39 mmol, prepared as ni example 15) afforded [5-(4-bromo-2,6-dimethyl-phenyl)-[1,2,4]triazin-3-yl]-(5-methoxy-benzothiazol-2-ylmethyl)-amine (898 mg, 91 %) as a yellow solid.
   ESI-MS m/z 456 and 458 (M+H)⁺.
   ¹H NMR (CDCl₃), δ (ppm): 8.59 (s, 1H), 7.68 (d, J = 8.8 Hz, 1H), 7.46 (d, J = 2.4 Hz, 1H), 7.25 (s, 2H), 7.02 (dd, J = 8.8 Hz and 2.4 Hz, 1H), 5.15 (br s, 2H), 3.88 (s, 3H), 2.06 (s, 6H).

### Example 80:

### [5-(2,6-Dimethyl-phenyl)-[1,2,4]triazin-3-yl]-(5-methoxy-benzothiazol-2-ylmethyl)-amine

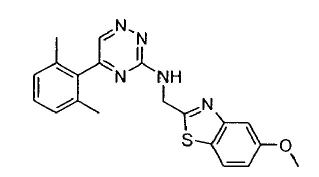

Under argon, a solution of [5-(4-bromo-2,6-dimethyl-phenyl)-[1,2,4]triazin-3-yl]-(5-methoxy-benzothiazol-2-ylmethyl)-amine (91.9 mg, 0.2 mmol, prepared as in example 79), triethylsilane (100 µL, 0.6 mmoL), cesium fluoride (60 mg, 0.4 mmol) and [1,1'-bis(diphenylphosphino)ferrocene]dichloropalladium(II) complex with dichloromethane (8.2 mg, 0.01 mmol) in degassed anhydrous 1,4-dioxane (1 mL) was stirred at 50°C for 3 days. The reaction mixture was filtered over a pad of celite, rinsed with dichloromethane, methanol, ethyl acetate, and evaporated. The crude product was purified by preparative TLC (silica gel, dichloromethane/methanol 94/6) to afford [5-(2,6-dimethyl-phenyl)-[1,2,4]triazin-3-yl]-(5-methoxy-benzothiazol-2-ylmethyl)-amine (72 mg, 95%) as a beige solid.

ESI-MS m/z 378 (M+H)⁺.

¹H NMR (CDCl₃), δ (ppm): 8.62 (s, 1H), 7.68 (d, J = 8.8 Hz, 1H), 7.47 (d, J = 2.4 Hz, 1H), 7.25 (t, J = 8.0 Hz, 1H), 7.11 Hz (d, J = 8.0 Hz, 2H), 7.02 (dd, J = 8.8 Hz and 2.4 Hz, 1H), 5.13 (br s, 2H), 3.88 (s, 3H), 2.09 (s, 6H).

### Example 81:

### N-(4-{3-[(5-Methoxy-benzothiazol-2-ylmethyl)-amino]-[1,2,4]triazin-5-yl}-3,5-dimethyl-phenyl)-acetamide

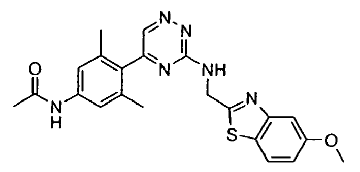

Under argon, a solution of [5-(4-bromo-2,6-dimethyl-phenyl)-[1,2,4]triazin-3-yl]-(5-methoxy-benzothiazol-2-ylmethyl)-amine (91.2 mg, 0.2 mmol, prepared as in example 79), acetamide (15.4 mg, 0.26 mmol), cesium carbonate (91.2 mg, 0.28 mmol), Xantphos (3.5 mg, 0.006 mmol), tris(dibenzylideneacetone)dipalladium(0) (1.8 mg, 0.002 mmol) in degassed anhydrous 1,4-dioxane (0.8 mL) was stirred at 100°C for 3 days. The reaction mixture was filtered over a pad of celite, rinsed with dichloromethane, methanol, ethyl acetate, and evaporated. The crude product was purified by preparative TLC (silica gel, dichloromethane/methanol 94/6) to afford N-(4-{3-[(5-methoxy-benzothiazol-2-ylmethyl)-amino]-[1,2,4]triazin-5-yl}-3,5-dimethyl-phenyl)-acetamide (20.4 mg, 23%) as a brown solid.

ESI-MS m/z 435 (M+H)⁺.

¹H NMR (CDCl₃), δ (ppm): 8.58 (s, 1H), 7.67 (d, J = 8.8 Hz, 1H), 7.46 (d, J = 2.4 Hz, 1H), 7.25 (s, 2H), 7.01 (dd, J = 8.8 Hz and 2.4 Hz, 1H), 5.12 (br s, 2H), 3.87 (s, 3H), 2.17 (s, 3H), 2.07 (s, 6H).

### Example 82:

### (E)-{5-[4-(3-Amino-propenyl)-2,6-dimethyl-phenyl]-[1,2,4]triazin-3-yl}-(5-methoxy-benzothiazol-2-ylmethyl)-amine

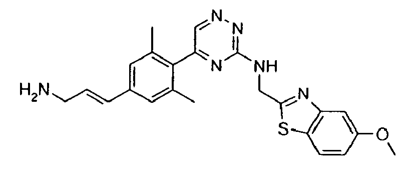

a)
   Under argon, a solution of [5-(4-bromo-2,6-dimethyl-phenyl)-[1,2,4]triazin-3-yl]-(5-methoxy-benzothiazol-2-ylmethyl)-amine (46 mg, 0.1 mmol, prepared as in example 79), *N*-allyl phtalimide (22.5 mg, 0.12 mmol, prepared as in Tetrahedron 2006, 62, 12247), triethylamine (28 µL, 0.2 mmol), tri-o-tolylphosphine (3.6 mg, 0.012 mmol), palladium(II) acetate (1.4 mg, 0.006 mmol) in degassed anhydrous acetonitrile (0.2 mL) was stirred at 100°C overnight. The reaction mixture was filtered over a pad of celite, rinsed with dichloromethane, methanol, ethyl acetate, and evaporated. The crude product was purified by preparative TLC (silica gel, dichloromethane/methanol 95/5) to afford 2-[3-(4-{3-[(5-methoxy-benzothiazol-2-ylmethyl)-amino]-[1,2,4]triazin-5-yl}-3,5-dimethyl-phenyl)-allyl]-isoindole-1,3-dione (43 mg, 76%) as a beige solid.
   ESI-MS m/z 563 (M+H)⁺.
b)
   Under argon, a solution of 2-[3-(4-{3-[(5-methoxy-benzothiazol-2-ylmethyl)-amino]-[1,2,4]triazin-5-yl}-3,5-dimethyl-phenyl)-allyl]-isoindole-1,3-dione (43 mg, 0,076), hydrazine hydrate (6 µL, 0.12 mmol) in ethanol (0.6 mL) was stirred a screwed-cap tube for 2 h. Solvent was evaporated, and the crude product was purified by preparative TLC (silica gel, dichloromethane/methanol/33% aqueous ammonia 95/5/1) to afford (*E*)-{5-[4-(3-Amino-propenyl)-2,6-dimethyl-phenyl]-[1,2,4]triazin-3-yl}-(5-methoxy-benzothiazol-2-ylmethyl)-amine (6.2 mg, 19%) as a beige solid.
   ESI-MS m/z 433 (M+H)⁺.
   ¹H NMR (CDCl₃), δ (ppm): 8.59 (s, 1H), 7.68 (d, J = 8.8 Hz, 1H), 7.46 (d, J = 2.4 Hz, 1H), 7.10 (s, 2H), 7.02 (dd, J = 8.8 Hz and 2.4 Hz, 1H), 6.49 (d, J = 16 Hz, 1H), 6.35 (dt, J = 16 Hz and 6 Hz, 1H), 5.13 (br s, 2H), 3.87 (s, 3H), 3.54 (d, J = 6 Hz, 2H), 2.07 (s, 6H).

### Example 83:

### (5-Metboxy-benzothiazol-2-ylmethyl)-{5-[4-(6-methoxy-pyridin-3-yl)-2,6-dimethylphenyl]-[1,2,4]triazin-3-yl}-amine

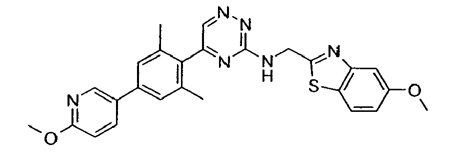

Under argon, a solution of [5-(4-bromo-2,6-dimethyl-phenyl)-[1,2,4]triazin-3-yl]-(5-methoxy-benzothiazol-2-ylmethyl)-amine (45.6 mg, 0.1 mmol, prepared as in example 79), 6-methoxy-3-pyridinylboronic acid (23 mg, 0.15 mmol), cesium carbonate (65 mg, 0.2 mmol) and [1,1'-bis(diphenylphosphino) ferrocene]dichloropalladium(II) adduct with dichloromethane (4 mg, 0.005 mmol) in degassed water (0.3 mL) and 1,2-dimethoxyethane (0.3 mL) was stirred at 85°C for 3 days. The reaction mixture was filtered over a pad of celite, rinsed with dichloromethane, methanol, and evaporated. The crude product was purified by preparative TLC (silica gel, dichloromethane/methanol 94/6) to afford (5-Methoxy-benzothiazol-2-ylmethyl)-{5-[4-(6-methoxy-pyridin-3-yl)-2,6-dimethyl-phenyl]-[1,2,4]triazin-3-yl}-amine (41.5 mg, 85%) as a white solid.

ESI-MS m/z 485 (M+H)⁺.

¹H NMR (CDCl₃), δ (ppm): 8.65 (s, 1H), 8.37 (d, J = 2.4 Hz, 1H), 7.76 (dd, J = 8.8 Hz and 2.4 Hz, 1H), 7.67 (d, J = 8.8 Hz, 1H), 7.45 (d, J = 2.0 Hz, 1H), 7.24 (s, 2H), 7.01 (dd, J = 8.8 Hz and 2.0 Hz, 1H), 6.81 (d, 8.4 Hz, 1H), 5.12 (br s, 2H), 3.98 (s, 3H), 3.87 (s, 3H), 2.16 (s, 6H).

### Example 84:

### 5-{4-[(2-Aminoethyl)thio]-2,6-dimethylphenyl}-N-[(5-methoxy-1,3-benzothiazol-2-yl)methyl]-1,2,4-triazin-3-amine

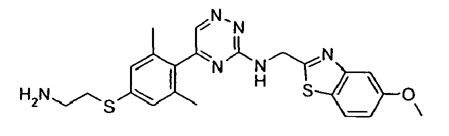

a)
   Under argon, to a solution of [5-(4-bromo-2,6-dimethyl-phenyl)-[1,2,4]triazin-3-yl]-(5-methoxy-benzothiazol-2-ylmethyl)-amine (50 mg, 0.11 mmol, prepared as in example 79), Xantphos (3.2 mg, 0.006 mmol), tris(dibenzylideneacetone)-dipalladium(0) (2.5 mg, 0.003 mmol) in degassed anhydrous 1,4-dioxane (0.25 mL) were successively added tert-butyl *N*-(2-mercaptoethyl)carbamate (24 µL, 0.14 mmol) and diisopropylethylamine (39 µL, 0.22 mmol). Then the reaction mixture was degassed, placed under argon atmosphere and stirred at 100°C overnight. The solution was then concentrated and purified by preparative TLC (silica gel, dichloromethane/methanol 94/6) to afford tert-butyl [2-(4-{3-[(5-methoxy-benzothiazol-2-ylmethyl)-amino]-[1,2,4]triazin-5-yl}-3,5-dimethyl-phenylsulfanyl)-ethyl]-carbamate (24 mg, 40%) as a yellow oil.
   ESI-MS m/z 553 (M+H)⁺.
b)
   Under argon, to tert-butyl [2-(4-{3-[(5-methoxy-benzothiazol-2-ylmethyl)-amino]-[1,2,4]triazin-5-yl}-3,5-dimethyl-phenylsulfanyl)-ethyl]-carbamate (24 mg, 0.043 mmol), was added a mixture of ethyl acetate (225 µL) and aqueous hydrochloric acid (37%, 75 µL). The resulting mixture was stirred at room temperature for 1 hour. Then an aqueous solution of sodium hydroxide was added to basify until pH = 10. The mixture was then extracted with dichloromethane, combined organic extracts were dried over sodium sulfate, filtered and evaporated. Purification by preparative TLC (silica gel, dichloromethane/methanol/ ammonium hydroxide 33% 95/5/1) led to 5-{4-[(2-aminoethyl)thio]-2,6-dimethylphenyl}-N-[(5-methoxy-1,3-benzothiazol-2-yl)methyl]-1,2,4-triazin-3-amine (17.5 mg, 89%) as a yellow solid.
   ESI-MS m/z 453 (M+H)⁺.
   ¹H NMR (CD₃OD), δ (ppm): 8.58 (s, 1H), 7.75 (d, J = 8.8 Hz, 1H), 7.40 (s, 1H), 7.08 (s, 2H), 7.03 (dd, J = 8.8 Hz and J = 2.2 Hz, 1H), 5.02 (s, 2H), 3.86 (s, 3H), 3.04 (t, J = 6.6 Hz, 2H), 2.81 (t, J = 6.6 Hz, 2H), 1.97 (s, 6H).

### Example 85:

### 4-(3-{[(5-Methoxy-1,3-benzothiazol-2-yl)methyl]amino}-1,2,4-triazin-5-yl)-3,5-dimethylbenzonitrile

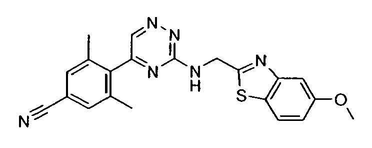

a)
   Under argon, a solution of 1-(4-bromo-2,6-dimethylphenyl)ethanone (2.749 g, 12.1 mmol, prepared as in J. Chem.. Soc. Perkin Trans. 2 1988, 943), copper cyanide (1.2 g, 13.4 mmol), and copper iodide (4.6 g, 24.2 mmol) in anhydrous DMF (12 mL) was stirred at 155°C overnight. The solution was cooled to room temperature, brine and ethyl acetate were added and the mixture was filtered over a pad of celite. The filtrate was extracted with ethyl acetate, combined organic extracts were dried over sodium sulfate, filtered and evaporated. Purification by flash chromatography (silica gel, cyclohexane/ethyl acetate, 95/5 to 7/3) afforded 4-acetyl-3,5-dimethyl-benzonitrile (800 mg, 38%) as a white solid.
   ESI-MS m/z 174 (M+H)⁺.
b)
   According to the experimental procedure used in example 22 for the conversion of aryl methyl ketones into 5-aryl-3-chloro-[1,2,4]-triazine derivatives, 4-acetyl-3,5-dimethylbenzonitrile (800 mg, 4.62 mmol) led to 4-(3-chloro-1,2,4-triazin-5-yl)-3,5-dimethylbenzonitrile (238.7 mg, 21%) as a white solid.
   ESI-MS m/z 245 and 247 (M+H)⁺.
c)
   According to the experimental procedure used in example 22 for the reaction of 3-chloro-[1,2,4]-triazine derivatives with amines, reaction of 4-(3-chloro-1,2,4-triazin-5-yl)-3,5-dimethylbenzonitrile (238.7 mg, 0.98 mmol) with (5-methoxy-benzothiazol-2-yl)-methylamine (464 mg, 2.39 mmol, prepared as in example 15) afforded [4-(3-{[(5-methoxy-1,3-benzothiazol-2-yl)methyl]amino}-1,2,4-triazin-5-yl)-3,5-dimethylbenzonitrile (322.2 mg, 82%) as a beige solid.
   ESI-MS m/z 403 (M+H)⁺.
   ¹H NMR (CDCl₃), δ (ppm): 8.60 (s, 1H), 7.69 (d, J = 8.8 Hz, 1H), 7.47 (s, 1H), 7.41 (s, 2H), 7.02 (d, J = 8.8 Hz, 1H), 5.14 (br s, 2H), 3.88 (s, 3H), 2.11 (s, 6H).

### Example 86:

### 5-[4-(Aminomethyl)-2,6-dimethylphenyll-N-F(5-methoxy-1,3-benzothiazol-2-yl)methyl]-1,2,4-triazin-3-amine

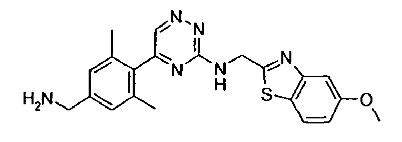

a)
   Under argon, a solution of diisobutylaluminium hydride in toluene (1M, 1 mL, 1 mmol) was slowly added to a solution of 4-(3-{[(5-methoxy-1,3-benzothiazol-2-yl)methyl]amino}-1,2,4-triazin-5-yl)-3,5-dimethylbenzonitrile (100 mg, 0.25 mmol, prepared as in example 85) in anhydrous dichloromethane (2.5 mL) stirred at -78°C. The reaction mixture was stirred for 3.5 hours at -78°C, then an aqueous solution of sodium potassium tartrate was added and the temperature let to rise. The mixture was extracted with dichloromethane, combined organic extracts were dried over sodium sulfate, filtered and evaporated. Purification by flash chromatography (silica gel, dichloromethane/methanol, 1/0 to 9/1) and preparative TLC (silica gel, dichloromethane/methanol 93/7) afforded 4-(3-{[(5-methoxy-1,3-benzothiazol-2-yl)methyl]amino}-1,2,4-triazin-5-yl)-3,5-dimethylbenzaldehyde (62.7 mg, 62%) as a yellow oil.
   ESI-MS m/z 406 (M+H)⁺.
b)
   Under argon, a solution of 4-(3-{[(5-methoxy-1,3-benzothiazol-2-yl)methyl]amino}-1,2,4-triazin-5-yl)-3,5-dimethylbenzaldehyde (60 mg, 0.15 mmol), ammonium acetate (171 mg, 2.22 mol) in anhydrous methanol (1.2 mL) with 4Å molecular sieves (100 mg) was stirred at room temperature for 5 minutes. Then a solution of sodium cyanoborohydride in THF (1M, 300 µL, 0.3 mmol) was added and the reaction mixture kept stirring overnight. An aqueous solution of potassium hydroxide was added (until pH = 10), and the mixture was extracted with dicloromethane. The combined organic extracts were dried over sodium sulfate, filtered and evaporated. Purication by preparative TLC (silica gel, dichloromethane/methanol/33% ammonium hydroxide 90/10/1) afforded 5-[4-(aminomethyl)-2,6-dimethylphenyl]-N-[(5-methoxy-1,3-benzothiazol-2-yl)methyl]-1,2,4-triazin-3-amine (7.0 mg, 12 %) as a white solid.
   ESI-MS m/z 407 (M+H)⁺.
   ¹H NMR (CDCl₃), δ (ppm): 8.61 (s, 1H), 7.68 (d, J = 8.8 Hz, 1H), 7.47 (d, J = 2.4 Hz, 1H), 7.06 (s, 2H), 7.02 (dd, J = 8.8 Hz and 2.4 Hz, 1H), 5.12 (br s, 2H), 3.88 (s, 3H), 3.84 (s, 2H), 2.10 (s, 6H).

### Example 87:

### 5-(2,6-Dimethoxyphenyl)-N-[(4,5-dimethyl-1,3-thiazol-2-yl)methyl]-N-methyl-1,2,4-triazin-3-amine

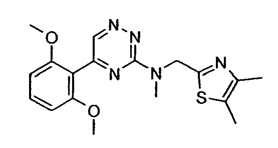

a)
   Under argon, to a solution of 4,5-dimethyl-1,3-thiazole-2-carbaldehyde (69 mg, 0.49 mmol, prepared as in example 1) and methylamine hydrochloride (165 mg, 2.44 mmol) in anhydrous dichloromethane (2 mL) were successively added triethylamine (82 µL, 0.59 mmol) and a solution of sodium cyanoborohydride in THF (1M, 1 mL, 1 mmol). The reaction mixture was stirred overnight at room temperature. An aqueous solution of potassium hydroxide 1N was added and the solution was extracted with dichloromethane. The combined organic extracts were dried over sodium sulfate, filtered and evaporated. Purification by preparative TLC (silica gel, dichloromethane/methanol 9/1) afforded N-[(4,5-dimethyl-1,3-thiazol-2-yl)methyl]-N-methylamine (5.6 mg, 7%) as a yellow oil.
   ESI-MS m/z 157 (M+H)⁺.
b)
   Under argon, a solution of 3-chloro-5-(2,6-dimethoxyphenyl)-1,2,4-triazine (6.85 mg, 0.027 mmol, prepared as in example 41), N-[(4,5-dimethyl-1,3-thiazol-2-yl)methyl]-N-methylamine (5.6 mg, 0.036 mmol) and potassium carbonate (3.8 mg, 0.027 mmol) in anhydrous acetonitrile (0.7 mL) was stirred at 85°C overnight. The solution was evaporated. The residue was purified by preparative TLC (silica gel, dichloromethane/methanol 95/5) to afford 5-(2,6-dimethoxyphenyl)-N-[(4,5-dimethyl-1,3-thiazol-2-yl)methyl]-N-methyl-1,2,4-triazin-3-amine (5.1 mg, 50%) as an off-white solid.
   ESI-MS m/z 372 (M+H)⁺.
   ¹H NMR (CDCl₃), δ (ppm): 8.59 (s, 1H), 7.36 (t, J = 8.4 Hz, 1H), 6.64 (d, J = 8.4 Hz, 2H), 5.18 (br s, 2H), 3.74 (s, 6H), 3.35 (s, 3H), 2.35 (s, 3H), 2.30 (s, 3H).

### Example 88:

### N-(1,3-Benzothiazol-2-ylmethyl)-5-(2,6-dimethoxyphenyl)-N-methyl-1,2,4-triazin-3-amine

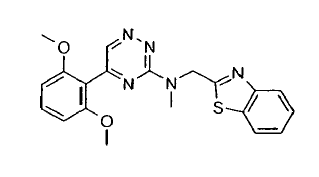

a)
   According to the same procedure used in example 87, reductive amination of 1,3-benzothiazole-2-carbaldehyde with methylamine hydrochloride afforded N-(1,3-benzothiazol-2-ylmethyl)-N-methylamine.
b)
   Under argon, a solution of 3-chloro-5-(2,6-dimethoxyphenyl)-1,2,4-triazine (8 mg, 0.032 mmol, prepared as in example 41), N-(1,3-benzothiazol-2-ylmethyl)-N-methylamine (8 mg, 0.045 mmol) and DIPEA (8 µL, 0.046 mmol) in anhydrous acetonitrile (0.2 mL) was stirred at 85°C overnight. The solution was evaporated. The residue was purified by preparative TLC (silica gel, dichloromethane/methanol 95/5) to afford N-(1,3-benzothiazol-2-ylmethyl)-5-(2,6-dimethoxyphenyl)-N-methyl-1,2,4-triazin-3-amine (6.5 mg, 52%) as an off-white solid.
   ESI-MS m/z 394 (M+H)⁺.
   ¹H NMR (CDCl₃), δ (ppm): 8.62 (s, 1H), 8.02 (d, J = 8.0 Hz, 1H), 7.84 (d, 8.0 Hz, 1H), 7.50-7.46 (m, 1H), 7.41-7.36 (m, 2H), 6.63 (d, J = 8.8 Hz, 2H), 5.37 (br s, 2H), 3.70 (s, 6H), 3.50 (br s, 3H).

### Example 89:

### 3-{(1,3-Benzothiazol-2-ylmethyl)[5-(2,6-dimethoxyphenyl)-1,2,4-triazin-3-yl]amino}propan-1-ol

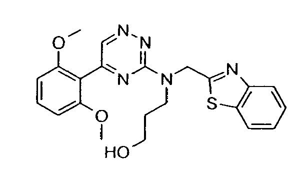

a)
   Under argon, a solution of 1,3-benzothiazole-2-carbaldehyde (31.4 mg, 0.19 mmol) and 3-aminopropan-1-ol (16 µL, 0.21 mmol) in methanol (1 mL) was stirred at room temperature overnight. Sodium borohydride (7 mg, 0.19 mmol) was added and the reaction mixture was stirred at room temperature for 1.5 hours. A few drops of aqueous ammonium chloride were added and the mixture was filtered over a pad of celite and concentrated. The residue was purified by preparative TLC (silica gel, dichloromethane/methanol 9/1) to furnish 3-[(1,3-benzothiazol-2-ylmethyl)amino]propan-1-ol (19.1 mg, 45%) as a pale yellow oil.
   ESI-MS m/z 223 (M+H)⁺.
b)
   Under argon, a solution of 3-chloro-5-(2,6-dimethoxyphenyl)-1,2,4-triazine (6.9 mg, 0.027 mmol, prepared as in example 41), 3-[(1,3-benzothiazol-2-ylmethyl)amino]propan-1-ol (19.1 mg, 0.086 mmol) in anhydrous acetonitrile (0.5 mL) was stirred at 85°C overnight. The solution was evaporated. The residue was purified by preparative TLC (silica gel, dichloromethane/methanol 9/1) to afford 3-{(1,3-benzothiazol-2-ylmethyl)[5-(2,6-dimethoxyphenyl)-1,2,4-triazin-3-yl]amino}propan-1-ol (4.7 mg, 39%) as a pale yellow oil.
   ESI-MS m/z 438 (M+H)⁺.
   ¹H NMR (CDCl₃), δ (ppm): 8.65 (s, 1H), 8.01 (d, J = 8.0 Hz, 1H), 7.83 (d, J = 8.0 Hz, 1H), 7.50-7.46 (m, 1H), 7.38 (t, J = 8.4 Hz, 2H), 6.64 (d, J = 8.4 Hz, 2H), 5.33 (br s, 2H), 3.80-3.63 (m, 4H), 3.71 (br s, 6H), 2.45-2.1 (m, 2H).

### Example 90:

### {(1,3-Benzothiazol-2-ylmethyl)[5-(2,6-dimethoxyphenyl)-1,2,4-triazin-3-yl]amino}acetic acid

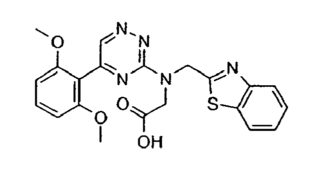

a)
   Under argon, a solution of 1,3-benzothiazol-2-ylmethylamine hydrochloride (600 mg, 3 mmol), ethyl chloroacetate (324 µL, 3 mmol) and triethylamine (916 µL, 6.6 mmol) in anhydrous acetonitrile (6 mL) was stirred at room temperature for 1.5 hours then at 50°C overnight. The solution was concentrated. The crude product was purified by flash chromatography (silical gel, dichloromethane/methanol, 1/0 to 95/5) to give ethyl [(1,3-benzothiazol-2-ylmethyl)amino]acetate (436 mg, 58%) as an orange oil.
   ESI-MS m/z 251 (M+H)⁺.
b)
   Under argon, a solution of 3-chloro-5-(2,6-dimethoxyphenyl)-1,2,4-triazine (50 mg, 0.20 mmol, prepared as in example 41), ethyl [(1,3-benzothiazol-2-ylmethyl)amino]acetate (50 mg, 0.20 mmol) and DIPEA (35 µL, 0.20 mmol) in anhydrous acetonitrile (0.4 mL) was stirred at 50°C overnight, and then at 80°C for 3 days. The solution was evaporated. The residue was purified by preparative TLC (silica gel, dichloromethane/methanol 97/3) to afford ethyl {(1,3-benzothiazol-2-ylmethyl)[5-(2,6-dimethoxyphenyl)-1,2,4-triazin-3-yl]amino} acetate (7 mg, 8%) as a yellow solid.
   ESI-MS m/z 466 (M+H)⁺.
c)
   Under argon, lithium hydroxide (0.4 mg, 0.017 mmol) was added to a solution of ethyl {(1,3-benzothiazol-2-ylmethyl)[5-(2,6-dimethoxyphenyl)-1,2,4-triazin-3-yl]amino}acetate (7 mg, 0.015 mmol) in THF (0.5 mL) and water (0.5 mL). The reaction mixture was stirred overnight at room temperature. A few drops of aqueous hydrochloric acid 1N were added, then the solvents were evaporated. Water was added and a solid was filtered. This solid was dissolved in dichloromethane, precipitation by addition of cyclohexane gave {(1,3-benzothiazol-2-ylmethyl)[5-(2,6-dimethoxyphenyl)-1,2,4-triazin-3-yl]amino}acetic acid (2.9 mg, 44%) as a white solid.
   ESI-MS m/z 438 (M+H)⁺.
   ¹H NMR (CDCl₃), δ (ppm): 8.75 (s, 1H), 7.94 (d, J = 7.8 Hz, 1H), 7.87 (d, J = 7.8 Hz, 1H), 7.49 (t, J = 7.8 Hz, 1H), 7.44-7.39 (m, 2H), 6.61 (br s, 2H), 5.53 (br s, 2H), 4.49 (br s, 2H), 3.80 (br s, 6H).

### Example 91:

### 3-{(1,3-Benzothiazol-2-ylmethyl)[5-(2,6-dimethoxyphenyl)-1,2,4-triazin-3-yl]amino}propanoic acid

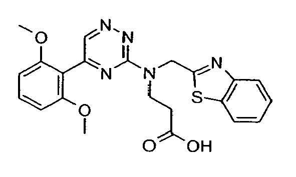

a)
   Under argon, a solution of 1,3-benzothiazol-2-ylmethylamine hydrochloride (201 mg, 1 mmol), methyl 3-bromopropanoate (113 µL, 1 mmol) and triethylamine (291 µL, 2.1 mmol) in anhydrous acetonitrile (2 mL) was stirred at room temperature for 30 miutes then at 45°C overnight. Water was added and the solution was extracted with ethyl acetate. The combined organic extracts were dried over sodium sulfate, filtered and evaporated. Purification by preparative TLC (silica gel, dichloromethane/methanol 94/6) furnished methyl 3-[(1,3-benzothiazol-2-ylmethyl)amino]propanoate (85.8 mg, 34%) as an off-white solid.
   ESI-MS m/z 251 (M+H)⁺.
b)
   Under argon, a solution of 3-chloro-5-(2,6-dimethoxyphenyl)-1,2,4-triazine (50 mg, 0.20 mmol, prepared as in example 41), methyl 3-[(1,3-benzothiazol-2-ylmethyl)amino]propanoate (50 mg, 0.20 mmol) and DIPEA (35 µL, 0.20 mmol) in anhydrous acetonitrile (0.4 mL) was stirred at 85°C for 3 days. The solution was evaporated. The residue was purified by preparative TLC (silica gel, dichloromethane/methanol 97/3) to afford methyl 3-{(1,3-benzothiazol-2-ylmethyl)[5-(2,6-dimethoxyphenyl)-1,2,4-triazin-3-yl]amino}propanoate (46.3 mg, 50%).
   ESI-MS m/z 466 (M+H)⁺.
c)
   Under argon, lithium hydroxide (3.6 mg, 0.15 mmol) was added to a solution of methyl 3-{(1,3-benzothiazol-2-ylmethyl)[5-(2,6-dimethoxyphenyl)-1,2,4-triazin-3-yl]amino}propanoate (46.3 mg, 0.10 mmol) in THF (0.5 mL) and water (0.5 mL). The reaction mixture was stirred overnight at room temperature. The solvents were evaporated and the residue was purified by preparative TLC (silica gel, dichloromethane/methanol 90/10) to give 3-{(1,3-benzothiazol-2-ylmethyl)[5-(2,6-dimethoxyphenyl)-1,2,4-triazin-3-yl]amino}propanoic acid (14.9 mg, 33%) as pale yellow oil.
   ESI-MS m/z 452 (M+H)⁺.
   ¹H NMR (CDCl₃), δ (ppm): 8.66 (s, 1H), 7.97 (br s, 1H), 7.78 (d, J = 7.8 Hz, 1H), 7.45-7.31 (m, 3H), 6.61 (d, J = 8.3 Hz, 2H), 5.42 (br s, 2H), 4.11 (br s, 2H), 3.68 (br s, 6H), 2.90 (br s, 2H).

### Example 92:

### Tert-butyl 2-{(13-benzothiazol-2-ylmethyl)[5-(2,6-dimethoxyphenyl)-1,2,4-triazin-3-yl]amino}ethylcarbamate

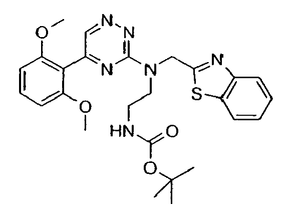

a)
   Under argon, a solution of 1,3-benzothiazole-2-carbaldehyde (49 mg, 0.30 mmol) and tert-butyl 2-aminoethylcarbamate (50 mg, 0.31 mmol) in methanol (1.2 mL) was stirred at room temperature overnight. Sodium borohydride (11.4 mg, 0.30 mmol) was added and the reaction mixture was stirred at room temperature for 2 hours. The mixture was concentrated and the residue was purified by preparative TLC (silica gel, dichloromethane/methanol 9/1) to furnish
   tert-butyl 2-[(1,3-benzothiazol-2-ylmethyl)amino]ethylcarbamate (57.1 mg, 62%) as a pale yellow oil.
   ESI-MS m/z 308 (M+H)⁺.
b)
   Under argon, a solution of 3-chloro-5-(2,6-dimethoxyphenyl)-1,2,4-triazine (40 mg, 0.16 mmol, prepared as in example 41), tert-butyl 2-[(1,3-benzothiazol-2-ylmethyl)amino]ethylcarbamate (57.1 mg, 0.19 mmol)and DIPEA (28 µL, 0.16 mmol) in anhydrous acetonitrile (0.7 mL) was stirred at 85°C for 2 days. The solution was evaporated. The residue was purified by preparative TLC (silica gel, dichloromethane/methanol 95/5) to afford tert-butyl 2-{(1,3-benzothiazol-2-ylmethyl)[5-(2,6-dimethoxyphenyl)-1,2,4-triazin-3-y!]amino}ethylcarbamate (35 mg, 42%) as a yellow solid.
   ESI-MS m/z 523 (M+H)⁺.
   ¹H NMR (CDCl₃), δ (ppm): 8.64 (s, 1H), 7.99 (d, J = 7.5 Hz, 1H), 7.80 (d, J = 7.5 Hz, 1H), 7.44 (t, J = 7.5 Hz, 1H), 7.37-7.33 (m, 2H), 6.62 (d, J = 8.6 Hz, 2H), 5.31 (br s, 2H), 3.98 (br s, 2H), 3.68 (br s, 6H), 3.55 (br s, 2H), 1.35 (s, 9H).

### Example 93:

### N-(1,3-Benzothiazol-2-ylmethyl)-N-[5-(2,6-dimethoxyphenyl)-1,2,4-triazin-3-yl]ethane-1,2-diamine

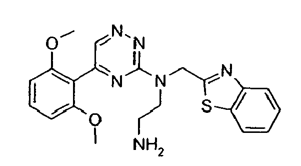

Under argon, trifluoroacetic acid (50µL) was added dropwise to a solution of tert-butyl 2-{(1,3-benzothiazol-2-ylmethyl)[5-(2,6-dimethoxyphenyl)-1,2,4-triazin-3-yl]amino}ethylcarbamate (15 mg, 0.029 mmol, prepared as in example 92) in dichloromethane (1 mL) cooled at 0°C. The reaction mixture was stirred at 0°C for 2 hours then at room temperature for 30 minutes. The solution was concentrated. Ethyl acetate and aqueous sodium hydroxide were added. The mixture was extracted with ethyl acetate, combined organic extracts were dried over sodium sulfate, filtered and evaporated. Purification by preparative TLC (silica gel, dichloromethane/methanol 95/5) led to N-(1,3-benzothiazol-2-ylmethyl)-N-[5-(2,6-dimethoxyphenyl)-1,2,4-triazin-3-yl]ethane-1,2-diamine (2.5 mg, 21%).

ESI-MS m/z 423 (M+H)⁺.

¹H NMR (CD₃OD), δ (ppm): 8.63 (s, 1H), 7.95 (d, J = 8.0 Hz, 2H), 7.51 (t, J = 8.0 Hz, 1H), 7.45-7.41 (m, 2H), 6.72 (br s, 2H), 5.34 (br s, 2H), 4.19 (br s, 2H), 3.59 (br s, 6H), 3.39 (br s, 2H).

### Example 94:

### N-({5-[(2-Aminoethyl)thio]-1,3-benzothiazol-2-yl}methyl)-5-(2,6-dichlorophenyl)-1,2,4-triazin-3-amine

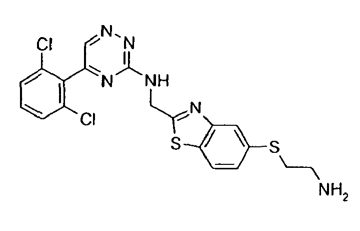

a)
   Under argon, a solution 3-chloro-5-(2,6-dichlorophenyl)-1,2,4-triazine (503 mg, 1.93 mmol, prepared as in example 15), (5-bromo-1,3-benzothiazol-2-yl)methylamine (516 mg, 2.12 mmol, prepared from 5-bromo-2-methyl-1,3-benzothiazole according to the representative procedure for the synthesis of (1,3-benzothiazol-2-yl)methylamine compounds from methyl-1,3-benzothiazole derivatives described in example 15) and DIPEA (500 µL, 2.87 mmol) in anhydrous acetonitrile (7 mL) was stirred at 85°C overnight. Water (10 mL) was added at room temperature, a precipitate was filtered and rinsed with water. The solid was dried to afford N-[(5-bromo-1,3-benzothiazol-2-yl)methyl]-5-(2,6-dichlorophenyl)-1,2,4-triazin-3-amine (833 mg, 92%) as a brown solid.
   ¹H NMR (CDCl₃, 300 MHz), δ (ppm): 8.72 (s, 1H), 8.14 (d, J = 1.8 Hz, 1H), 7.72 (d, J = 8.6 Hz, 1H), 7.50 (dd, J = 8.6 and 1.8 Hz, 1H), 7.45-7.34 (m, 3H), 5.21 (br, 2H).
b)
   Under argon, to a solution of N-[(5-bromo-1,3-benzothiazol-2-yl)methyl]-5-(2,6-dichlorophenyl)-1,2,4-triazin-3-amine (93.4 mg, 0.2 mmol), Xantphos (5.8 mg, 0.01 mmol), tris(dibenzylideneacetone)dipalladium(0) (4.6 mg, 0.005 mmol) in degassed anhydrous 1,4-dioxane (0.5 mL) were successively added tert-butyl N-(2-mercaptoethyl)carbamate (46 µL, 0.27 mmol) and diisopropylethylamine (70 µL, 0.4 mmol). Then the reaction mixture was degassed, placed under argon atmosphere and stirred at 100°C overnight. The solution was then concentrated and purified by preparative TLC (silica gel, dichloromethane/methanol 94/6) to afford tert-butyl {[2-({[5-(2,6-dichlorophenyl)-1,2,4-triazin-3-yl]amino}methyl)-1,3-benzothiazol-5-yl]thio}ethylcarbamate (65.1 mg, 57%) as a yellow solid.
   ESI-MS m/z 563 and 565 (M+H)⁺.
c)
   Under argon, to tert-butyl {[2-({[5-(2,6-dichlorophenyl)-1,2,4-triazin-3-yl]amino}methyl)-1,3-benzothiazol-5-yl]thio}ethylcarbamate (65.1 mg, 0.12 mmol), was added a mixture of ethyl acetate (0.75 mL) and aqueous hydrochloric acid (37%, 0.25 mL). The resulting mixture was stirred at room temperature for 1 hour. Then an aqueous solution of sodium hydroxide was added to basify until pH = 10. The mixture was then extracted with dichloromethane, combined organic extracts were dried over sodium sulfate, filtered and evaporated. Purification by preparative TLC (silica gel, dichloromethane/methanol/ ammonium hydroxide 33% 90/10/1) led to N-({5-[(2-aminoethyl)thio]-1,3-benzothiazol-2-yl}methyl)-5-(2,6-dichlorophenyl)-1,2,4-triazin-3-amine (18.5 mg, 35%) as a white solid.
   ESI-MS m/z 463 and 465 (M+H)⁺.
   ¹H NMR (CD₃OD), δ (ppm): 8.68 (s, 1H), 7.91 (br s, 1H), 7.83 (d, J = 8.4 Hz, 1H), 7.50-7.43 (m, 3H), 7.41 (dd, J = 8.4 Hz and 2.0 Hz, 1H), 5.06 (br s, 2H), 3.09 (t, J = 6.6 hz, 2H), 2.82 (t, J = 6.6 Hz, 2H).

### Example 95:

### N-[4-Chloro-3-(3-{[(5-methoxy-1,3-benzothiazol-2-yl)methyl]amino}-1,2,4-triazin-5-yl)phenyl]acetamide

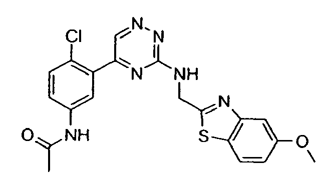

Under argon, a solution of 5-(5-bromo-2-chlorophenyl)-N-[(5-methoxy-1,3-benzothiazol-2-yl)methyl]-1,2,4-triazin-3-amine (40 mg, 0.086 mmol, prepared as in example 31), acetamide (6.6 mg, 0.11 mmol), cesium carbonate (39.4 mg, 0.12 mmol), Xantphos (1.5 mg, 2.6 µmol), tris(dibenzylideneacetone)dipalladium(0) (0.8 mg, 0.9 µmol) in degassed anhydrous 1,4-dioxane (0.5 mL) was stirred at 100°C overnight. Water was added and the resulting mixture was extracted with ethyl acetate. Combined organic extracts were dried over sodium sulfate, filtered and evaporated. The crude product was purified by preparative TLC (silica gel, dichloromethane/methanol 95/5) to afford N-[4-chloro-3-(3-{[(5-methoxy-1,3-benzothiazol-2-yl)methyl]amino}-1,2,4-triazin-5-yl)phenyl]acetamide (5.4 mg, 14%) as a brown solid.

ESI-MS m/z 441 and 443 (M+H)⁺.

¹H NMR (CD₃OD), δ (ppm): 8.97 (s, 1H), 7.94-7.92 (m, 1H), 7.77 (d, J = 8.8 Hz, 1H), 7.69 (dd, J = 8.8 Hz and 2.8 Hz, 1H), 7.47 (d, J = 8.8 Hz, 1H), 7.44 (d, J = 2.4 Hz, 1H), 7.04 (dd, J = 8.8 and 2.4 Hz, 1H), 5.09 (br s, 2H), 3.87 (s, 3H), 2.12 (s, 3H).

### Example 96:

### 3-[4-Chloro-3-(3-{[(5-methox-1,3-benzothiazol-2-yl)methyl]amino}-1,2,4-triazin-5-yl)phenyl]-1,3-oxazolidin-2-one

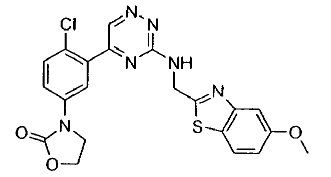

Under argon, a solution of 5-(5-bromo-2-chlorophenyl)-N-[(5-methoxy-1,3-benzothiazol-2-yl)methyl]-1,2,4-triazin-3-amine (50 mg, 0.108 mmol, prepared as in example 31), 1,3-oxazolidin-2-one (12.5 mg, 0.14 mmol), cesium carbonate (49.3 mg, 0.15 mmol), Xantphos (3.7 mg, 0.006 mmol), tris-(dibenzylideneacetone)-dipalladium(0) (2.0 mg, 0.002 mmol) in degassed anhydrous 1,4-dioxane (0.5 mL) was stirred at 100°C for 2 days. The reaction mixture was then filtered over a pad of celite, rinsed with methanol, dichloromethane and ethyl acetate. The filtrate was concentrated. The crude product was purified by preparative TLC (silica gel, dichloromethane/methanol 94/6) to give 3-[4-chloro-3-(3-{[(5-methoxy-1,3-benzothiazol-2-yl)methyl]amino}-1,2,4-triazin-5-yl)phenyl]-1,3-oxazolidin-2-one (14 mg, 27%) as a brown solid.

ESI-MS m/z 469 and 471 (M+H)⁺.

¹H NMR (CDCl₃), δ (ppm): 9.15 (s, 1H), 7.88-7.86 (m, 1H), 7.69 (d, J = 8.8 Hz, 1H), 7.49-7.47 (m, 3H), 7.02 (dd, J = 8.8 Hz and 2.4 Hz, 1H), 5.14 (br s, 2H), 4.38-4.36 (m, 2H), 3.88 (s, 3H), 3.88-3.86 (m, 2H).

### Example 97:

### 5-[2-Chloro-5-(1H-pyrazol-3-yl)phenyl]-N-[(5-methoxy-1,3-benzothiazol-2-yl)methyl]-1,2,4-triazin-3-amine

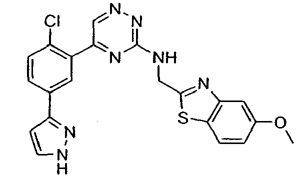

Under argon, a solution of 5-(5-bromo-2-chlorophenyl)-N-[(5-methoxy-1,3-benzothiazol-2-yl)methyl]-1,2,4-triazin-3-amine (50 mg, 0.11 mmol, prepared as in example 31), 1H-pyrazol-3-yl boronic acid (24.2 mg, 0.22 mmol), sodium carbonate (34.5 mg, 0.33 mmol) and palladium-tetrakis(triphenylphosphine) (37.5 mg, 0.032 mmol) in degassed DMF (0.75 mL) was stirred at 100°C overnight. An aqueous solution of ammonium chloride was added and the reaction mixture was extracted with ethyl acetate. The combined organic extracts were dried over sodium sulfate, filtered and evaporated. The crude product was purified by preparative TLC (silica gel, dichloromethane/methanol/aqueous ammonium hydroxide 33%, 95/5/1) to afford 5-[2-chloro-5-(1H-pyrazol-3-yl)phenyl]-N-[(5-methoxy-1,3-benzothiazol-2-yl)methyl]-1,2,4-triazin-3-amine (25 mg, 51%) as a yellow solid.

ESI-MS m/z 450 and 452 (M+H)⁺.

¹H NMR (CDCl₃), δ (ppm): 9.13 (s, 1H), 8.05 (br s, 1H), 7.81 (dd, J = 8.4 Hz and J = 1.6 Hz, 1H), 7.64 (d, J = 8.4 Hz, 1H), 7.59 (br s, 1H), 7.49-7.44 (m, 2H), 6.99 (dd, J = 8.4 Hz and J = 2.4 Hz, 1H), 6.49 (br s, 1H), 5.19 (br s, 2H), 3.83 (s, 3H).

### Example 98:

### 5-[2-Chloro-5-(1H-pyrazol-4-yl)phenyl]-N-[(5-methoxy-1,3-benzothiazol-2-yl)methyl]-1,2,4-triazin-3-amine

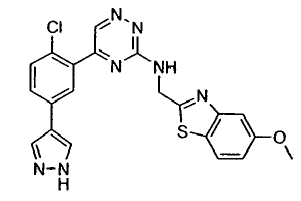

Under argon, a solution of 5-(5-bromo-2-chlorophenyl)-N-[(5-methoxy-1,3-benzothiazol-2-yl)methyl]-1,2,4-triazin-3-amine (50 mg, 0.11 mmol, prepared as in example 31), 1H-pyrazol-4-yl boronic acid (24.2 mg, 0.22 mmol), sodium carbonate (34.5 mg, 0.33 mmol) and palladium-tetrakis(triphenylphosphine) (37.5 mg, 0.032 mmol) in degassed DMF (0.75 mL) was stirred at 100°C overnight. An aqueous solution of ammonium chloride was added and the reaction mixture was extracted with ethyl acetate. The combined organic extracts were dried over sodium sulfate, filtered and evaporated. The crude product was purified by preparative TLC (silica gel, dichloromethane/methanol 95/5) to afford 5-[2-chloro-5-(1H-pyrazol-4-yl)phenyl]-N-[(5-methoxy-1,3-benzothiazol-2-yl)methyl]-1,2,4-triazin-3-amine (1.1 mg, 2%) as a yellow solid.

ESI-MS m/z 450 and 452 (M+H)⁺.

¹H NMR (CD₃OD), δ (ppm): 9.04 (s, 1H), 7.80-7.43 (m, 7H), 7.07 (dd, J = 8.9 Hz and J = 2.4 Hz, 1H), 5.09 (br s, 2H), 3.88 (br s, 3H).

### Example 99:

### 5-(2-Chloro-5-pyridin-3-ylphenyl)-N-[(5-methoxy-1,3-benzothiazol-2-yl)methyl]-1,2,4-triazin-3-amine

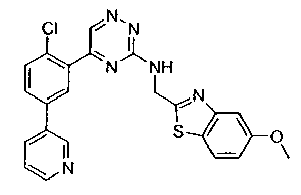

Under argon, a solution of 5-(5-bromo-2-chlorophenyl)-N-[(5-methoxy-1,3-benzothiazol-2-yl)methyl]-1,2,4-triazin-3-amine (30 mg, 0.065 mmol, prepared as in example 31), 3-pyridinyl boronic acid (9.5 mg, 0.078 mmol), cesium carbonate (63.5 mg, 0.19 mmol) and [1,1'-bis(diphenylphosphino)ferrocene]dichloropalladium(II) adduct with dichloromethane (4.8 mg, 0.006 mmol) in degassed water (0.1 mL) and 1,4-dioxane (0.3 mL) was stirred at 100°C overnight. An aqueous solution of ammonium chloride was added and the reaction mixture was extracted with ethyl acetate. The combined organic extracts were dried over sodium sulfate, filtered and evaporated. The crude product was purified by preparative TLC (silica gel, dichloromethane/methanol 95/5) to afford 5-(2-chloro-5-pyn'din-3-yiphenyl)-N-[(5-methoxy-1,3-benzothiazol-2-yl)methyl]-1,2,4-triazin-3-amine (10 mg, 33%) as a yellow solid.

ESI-MS m/z 461 and 463 (M+H)⁺.

¹H NMR (CDCl₃), δ (ppm): 9.17 (s, 1H), 8.83 (s, 1H), 8.63 (d, J = 5.0 Hz, 1H), 7.90-7.76 (m, 2H), 7.69-7.60 (m, 3H), 7.47 (d, J = 2.0 Hz, 1H), 7.35 (t, J = 5.0 Hz, 1H), 7.02 (dd, J = 8.9 Hz and J = 2.0 Hz, 1H), 5.18 (s, 2H), 3.87 (s, 3H).

### Example 100:

### 2-[4-(4-Chloro-3-{3-[(5-methoxy-benzothiazol-2-ylmethyl)-amino]-[1,2,4]triazin-5-yl}-phenyl)-[1,2,3]triazol-1-yl]-ethanol

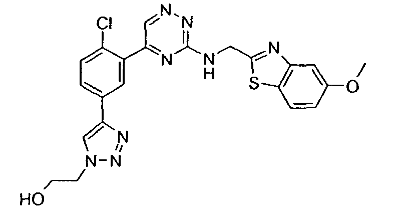

a)
   Under argon, triethylamine (280 µL, 2 mmol) and trimethylsilylacetylene (115 µL, 0.8 mmol) were successively added to a solution of 5-(5-bromo-2-chlorophenyl)-N-[(5-methoxy-1,3-benzothiazol-2-yl)methyl]-1,2,4-triazin-3-amine (185.1 mg, 0.4 mmol, prepared as in example 31), [1,1'-bis(diphenylphosphino)-ferrocene]-dichloropalladium(II) complex with dichloromethane (16.4 mg, 0.02 mmol) and copper(I) iodide in degassed anhydrous acetonitrile (1.6 mL). The resulting mixture was degassed and stirred under argon at 90°C for 4.5 hours. The solution was concentrated and the crude product was purified by preparative TLC (silica gel, dichloromethane/methanol 93/7) to afford [5-(2-chloro-5-trimethylsilanylethynylphenyl)-[1,2,4]triazin-3-yl]-(5-methoxy-benzothiazol-2-ylmethyl)-amine (139 mg, 72%) as a brown solid.
   ESI-MS m/z 480 and 482 (M+H)⁺.
b)
   Under argon, potassium carbonate (200 mg, 1.45 mmol) was added to a solution of [5-(2-chloro-5-trimethylsilanylethynyl-phenyl)-[1,2,4]triazin-3-yl]-(5-methoxy-benzothiazol-2-ylmethyl)-amine (139 mg, 0.29 mmol) in methanol (1 mL) and dichloromethane (1 mL). The resulting mixture was stirred at room temperature for 1.5 hours. The solution was filtered and the filtrate was concentrated. Purification by preparative TLC (silica gel, dichloromethane/methanol 95/5) led to [5-(2-chloro-5-ethynyl-phenyl)-[1,2,4]triazin-3-yl]-(5-methoxy-benzothiazol-2-ylmethyl)-amine (69.6 mg, 59%) as an off-white solid.
   ESI-MS m/z 408 and 410 (M+H)⁺.
c)
   Under argon, sodium azide (12.2 mg, 0.188 mmol) and ethyl bromoacetate were added to a solution of [5-(2-chloro-5-ethynyl-phenyl)-[1,2,4]triazin-3-yl]-(5-methoxy-benzothiazol-2-ylmethyl)-amine (69.6 mg, 0.17 mmol) in tert-butanol (0.35 mL) and water (0.35 mL) stirred at room temperature. Then copper(II) sulfate (1.4 mg, 0.009 mmol) and sodium ascorbate (3.4 mg, 0.017 mmol) were added. The reaction mixture was stirred overnight at room temperature. Sodium azide (12.2 mg, 0.188 mmol), ethyl bromoacetate, copper(II) sulfate (1.4 mg, 0.009 mmol) and sodium ascorbate (3.4 mg, 0.017 mmol) were added and the reaction was kept sirring at room temperature for 24 hours, then at 50°C for 24 hours. An aqueous solution of ammonium hydroxide was added, and the mixture was extracted with ethyl acetate and dichloromethane. Combined organic extracts were dried over sodium sulfate, filtered and evaporated. Purification by preparative TLC (silica gel, dichloromethane/methanol 95/5) gave ethyl [4-(4-chloro-3-{3-[(5-methoxy-benzothiazol-2-ylmethyl)-amino]-[1,2,4]triazin-5-yl}-phenyl)-[1,2,3]triazol-1-yl]-acetate (19.3 mg, 21%).
   ESI-MS m/z 537 and 539 (M+H)⁺.
d)
   Under argon, a solution of diisobutylaluminium hydride in toluene (1M, 0.14 mL, 0.14 mmol) was slowly added to a solution of [4-(4-chloro-3-{3-[(5-methoxybenzothiazol-2-ylmethyl)-amino]-[1,2,4]triazin-5-yl}-phenyl)-[1,2,3]triazol-1-yl]-acetate (19.3 mg, 0.036 mmol) in anhydrous dichloromethane (1 mL) stirred at -78°C. The reaction mixture was stirred for 1.5 hours allowing the temperature to raise to -10°C. Methanol (0.3 mL) was added and the solution was kept stirring for 30 minutes allowing the temperature to rise. Sodium borohydride (5 mg, 0.13 mmol) was next added and the resulting mixture was stirred at room temperature for 1.5 hours. Then an aqueous solution of sodium potassium tartrate was added and the resulting solution was stirred for 30 minutes. The mixture was extracted with ethyl acetate, combined organic extracts were dried over sodium sulfate, filtered and evaporated. Purification by preparative TLC (silica gel, dichloromethane/methanol 9/1) afforded 2-[4-(4-Chloro-3-{3-[(5-methoxy-benzothiazol-2-ylmethyl)-amino]-[1,2,4]triazin-5-yl}-phenyl)-[1,2,3]triazol-1-yl]-ethanol (9.4 mg, 53%) as a pale yellow oil.
   ESI-MS m/z 495 and 497 (M+H)⁺.
   ¹H NMR (CD₃OD), δ (ppm): 9.12 (s, 1H), 8.38 (br s, 1H), 8.25 (br s, 1H), 8.02 (dd, J = 8.1 Hz and 1.8 Hz, 1H), 7.86 (d, J = 8.9 Hz, 1H), 7.68 (d, J = 8.1 Hz, 1H), 7.46 (br s, 1H), 7.11 (dd, J = 8.9 Hz and 2.5 Hz, 1H), 5.23 (s, 2H), 4.56 (t, J = 5.2 Hz, 2H), 3.99 (t, J = 5.2 Hz, 2H), 3.88 (s, 3H).

### Example 101:

### 5-(2-Chlorophenyl)-N-[(5-methoxy-1,3-benzothiazol-2-yl)methyl]-1,2,4-triazin-3-amine

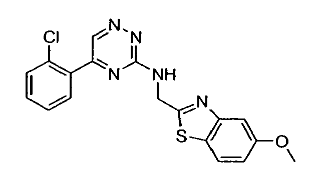

Under argon, a solution of 5-(5-bromo-2-chlorophenyl)-N-[(5-methoxy-1,3-benzothiazol-2-yl)methyl]-1,2,4-triazin-3-amine (50 mg, 0.11 mmol, prepared as in example 31), triethylsilane (52 µL, 0.33 mmoL), cesium fluoride (32.9 mg, 0.22 mmol) and [1,1'-bis(diphenylphosphino)ferrocene]dlchloropalladium(II) complex with dichloromethane (4.4 mg, 0.005 mmol) in degassed anhydrous 1,4-dioxane (0.5 mL) was stirred at 50°C for 3 days. The reaction mixture was filtered over a pad of celite, rinsed with dichloromethane, methanol, ethyl acetate, and evaporated. The crude product was purified by preparative TLC (silica gel, dichloromethane/methanol 95/5) to afford 5-(2-chlorophenyl)-N-[(5-methoxy-1,3-benzothiazol-2-yl)methyl]-1,2,4-triazin-3-amine (7.9 mg, 19%) as a yellow oil.

ESI-MS m/z 384 and 386(M+H)⁺.

¹H NMR (CDC13), δ (ppm): 9.11 (s, 1H), 7.70-7.63 (m, 2H), 7.52-7.37 (m, 4H), 7.03 (d, J = 8.8 Hz, 1H), 5.19 (s, 2H), 3.88 (s, 3H).

### Example 102:

### 5-(4-Fluoro-2-vinylphenyl)-N-[(5-methoxy-1,3-benzothiazol-2-yl)methyl]-1,2,4-triazin-3-amine

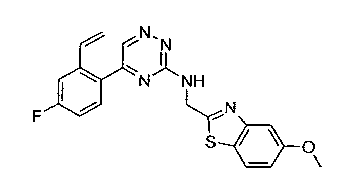

Under argon, 4,4,5,5-tetramethyl-2-vinyl-1,3,2-dioxaborolane (114 µL, 0.67 mmol) was added to a solution of 5-(2-bromo-4-fluorophenyl)-N-[(5-methoxy-1,3-benzothiazol-2-yl)methyl]-1,2,4-triazin-3-amine (150 mg, 0.336 mmol, prepared as in example 30), [1,1'-bis(diphenylphosphino)ferrocene]dichloropalladium(II) complex with dichloromethane (13.7 mg, 0.017 mmol), potassium carbonate (92.7 mg, 0.67 mmol), in acetonitrile (1.5 mL) and water (0.5 mL). The reaction mixture was degassed and stirred at 85°C under argon overnight. An aqueous solution of ammonium chloride was added and the solution was extracted with ethyl acetate. Combined organic extracts were dried over sodium sulfate, filtered and evaporated. The crude product was purified by preparative TLC (silica gel, dichloromethane/methanol 95/5) to afford 5-(4-fluoro-2-vinylphenyl)-N-[(5-methoxy-1,3-benzothiazol-2-yl)methyl]-1 ,2,4-triazin-3-amine (120.5 mg, 91%) as a yellow solid.

ESI-MS m/z 394 (M+H)⁺.

¹H NMR (CDCl₃), δ (ppm): 8.82 (s, 1H), 7.68 (d, J = 8.8 Hz, 1H), 7.58 (dd, J = 8.4 Hz and 5.6 Hz, 1H), 7.48 (d, J = 2.4 Hz, 1H), 7.30 (dd, J = 9.6 Hz and 2.4 Hz, 1H), 7.09 (td, J = 8.4 Hz and 2.4 Hz, 1H), 7.03 (dd, J = 8.8 Hz and 2.4 Hz, 1H), 6.85 (dd, J = 17.2 Hz and 11.2 Hz, 1H), 5.80 (d, J = 17.2 Hz, 1H), 5.32-5.25 (m, 1H), 5.16 (br s, 2H), 3.88 (s, 3H).

### Example 103:

### N-[(5-Methoxy-1,3-benzothiazol-2-yl)methyl]-5-(2-bromo-4-morpholin-4-ylphenyl)-1,2,4-triazin-3-amine

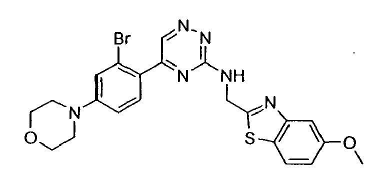

Under argon, a solution of 5-(2-bromo-4-fluorophenyl)-N-[(5-methoxy-1,3-benzothiazol-2-yl)methyl]-1,2,4-triazin-3-amine (30 mg, 0.067 mmol, prepared as in example 30) and morpholine (30 µL, 0.34 mmol) in n-butanol (0.5 mL) was stirred at 120°C for 3 days. The reaction mixture was concentrated. The crude product was purified by preparative TLC (silica gel, dichloromethane/methanol 95/5) to give N-[(5-methoxy-1,3-benzothiazol-2-yl)methyl]-5-(2-bromo-4-morpholin-4-ylphenyl)-1,2,4-triazin-3-amine (8.8 mg, 26%) as a yellow solid.

ESI-MS m/z 513 and 515 (M+H)⁺.

¹H NMR (CDCl₃), δ (ppm): 9.14 (s, 1H), 7.68 (d, J = 8.8 Hz, 1H), 7.57 (d, J = 8.8 Hz, 1H), 7.49 (d, J = 2.4 Hz, 1H), 7.13 (d, J = 2.4 Hz, 1H), 7.02 (dd, J = 8.8 Hz and 2.4 Hz, 1H), 6.89 (dd, J = 8.8 Hz and 2.4 Hz, 1H), 5.18 (d, J = 5.6 Hz, 2H), 3.89 (s, 3H), 3.89-3.84 (m, 4H), 3.27-3.25 (m, 4H).

### Example 104:

### N-[(5-Methoxy-1,3-benzothiazol-2-yl)methyl]-5-(4-fluoro-2-morpholin-4-ylphenyl)-1,2,4-triazin-3-amine

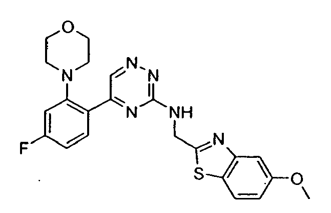

The title compound (8.9 mg, 29%) was also obtained as a product of the reaction described in example 103, as a yellow solid.

ESI-MS m/z 453 (M+H)⁺.

¹H NMR (CDCl₃), δ (ppm): 9.48 (s, 1H), 7.72-7.68 (m, 2H), 7.49 (d, J = 2.4 Hz, 1H), 7.03 (dd, J = 8.8 Hz and 2.4 Hz, 1H), 6.87-6.79 (m, 2H), 5.18 (d, J = 6.0 Hz, 2H), 3.89 (s, 3H), 3.78-3.76 (m, 4H), 2.92-2.90 (m, 4H).

### Example 105:

### 5-(4-Fluorophenyl)-N-[(5-methoxy-1,3-benzothiazol-2-yl)methyl]-1,2,4-triazin-3-amine

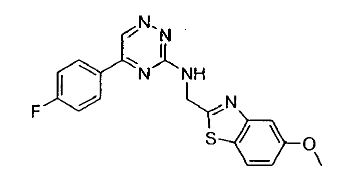

Under argon, to a solution of 5-(2-bromo-4-fluorophenyl)-N-[(5-methoxy-1,3-benzothiazol-2-yl)methyl]-1,2,4-triazin-3-amine (30 mg, 0.067 mmol, prepared as in example 30) and iron(III) acetylacetonate (1.2 mg, 0.003 mmol) in anhydrous THF (0.4 mL) and NMP (40 µL) was slowly added a solution ethyl magnesium bromide in THF (1M, 150 µL, 0.15 mmol). The reaction mixture was stirred at room temperature for 15 minutes, then diethyl ether and an aqueous solution of hydrochloric acid 1N were successively added at 0°C. The solution was extracted with ethyl acetate. Combined organic extracts were dried over sodium sulfate, filtered and evaporated. The crude product was purified by preparative TLC (silica gel, dichloromethane/methanol 96/4) to afford 5-(4-fluorophenyl)-N-[(5-methoxy-1,3-benzothiazol-2-yl)methyl]-1,2,4-triazin-3-amine (5.6 mg, 23%) as a yellow solid.

ESI-MS m/z 368 (M+H)⁺.

¹H NMR (CDCl₃), δ (ppm): 9.11 (s, 1H), 8.14-8.10 (m, 2H), 7.67 (d, J = 8.8 Hz, 1H), 7.49 (d, J = 2.4 Hz, 1H), 7.20 (t, J = 8.6 Hz, 2H), 7.02 (dd, J = 8.8 Hz and 2.4 Hz, 1H), 5.19 (br s, 2H), 3.88 (s, 3H).

### Example 106:

### 5-(2-Chloro-4-fluorophenyl)-N-[(5-methoxy-1,3-benzothiazol-2-yl)methyl]-1,2,4-triazin-3-amine

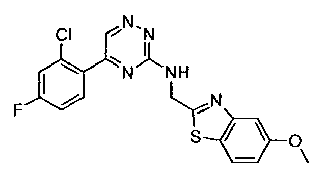

Under argon, a solution of isopropylmagnesium chloride in THF (2M, 112 µL, 0.224 mmol) and zinc chloride in THF (0.5M, 450 µL, 0.225 mmol) was stirred at 50°C for 2.5 hours. The reaction mixture was then added to a solution of 5-(2-bromo-4-fluorophenyl)-N-[(5-methoxy-1,3-benzothiazol-2-yl)methyl]-1,2,4-triazin-3-amine (31 mg, 0.069 mmol, prepared as in example 30), [1,1'-bis(diphenylphosphino)ferrocene]dichloropalladium(II) complex with dichloromethane (3.7 mg, 0.005 mmol) and copper (1) iodide (0.7 mg, 0.004 mmol), in anhydrous THF (0.4 mL) at room temperature. The reaction mixture was degassed and stirred under argon at room temperature for 24 hours, then at 65°C for 48 hours. The reaction mixture was then filtered over a pad of celite, rinsed with methanol, dichloromethane and ethyl acetate. The filtrate was concentrated. The crude product was purified by preparative TLC (silica gel, dichloromethane/methanol 95/5) to yield 5-(2-chloro-4-fluorophenyl)-N-[(5-methoxy-1,3-benzothiazol-2-yl)methyl]-1,2,4-triazin-3-amine (12.8 mg, 46%) as a brown solid.

ESI-MS m/z 402 and 404 (M+H)⁺.

¹H NMR (CDCl₃), δ (ppm): 9.11 (s, 1H), 7.69-7.65 (m, 1H), 7.68 (d, J = 8.8 Hz, 1H), 7.48 (d, J = 2.4 Hz, 1H), 7.25 (dd, J = 8.4 Hz and 2.4 Hz, 1H), 7.13-7.09 (m, 1H), 7.02 (dd, J = 8.8 Hz and 2.4 Hz, 1H), 5.17 (br s = 2H), 3.88 (s, 3H).

### Example 107:

### N-[(5-Methoxy-1,3-benzothiazol-2-yl)methyl]-5-(2-chloro-4-piperazin-1-ylphenyl)-1,2,4-triazin-3-amine

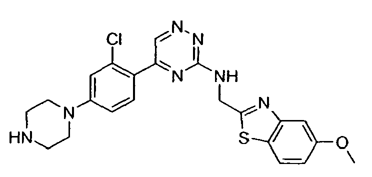

Under argon, a solution of 5-(2-chloro-4-fluorophenyl)-N-[(5-methoxy-1,3-benzothiazol-2-yl)methyl]-1,2,4-triazin-3-amine (21.8 mg, 0.054 mmol, prepared as in example 106) and piperazine (23.4 mg, 0.27 mmol) in n-butanol (0.5 mL) was stirred at 120°C for 3 days. The reaction mixture was concentrated. An aqueous solution of ammonium chloride was added and the solution was extracted with ethyl acetate. Combined organic extracts were dried over sodium sulfate, filtered and evaporated. The crude product was purified by preparative TLC (silica gel, dichloromethane/methanol/triethylamine 9/1/0.1) to give N-[(5-methoxy-1,3-benzothiazol-2-yl)methyl]-5-(2-chloro-4-piperazin-1-ylphenyl)-1,2,4-triazin-3-amine (5.1 mg, 20%) as a yellow solid.

ESI-MS m/z 468 and 470 (M+H)⁺.

¹H NMR (CDCl₃), δ (ppm): 9.20 (s, 1H), 7.69 (d, J = 8.8 Hz, 1H), 7.68 (d, J = 8.8 Hz, 1H), 7.49 (d, J = 2.4 Hz, 1H), 7.02 (dd, J = 8.8 Hz and 2.4 Hz, 1H), 6.92 (d, J = 2.0 Hz, 1H), 6.85 (dd, J = 8.8 Hz and 2.0 Hz, 1H), 5.17 (d, J = 5.2 Hz, 2H), 3.89 (s, 3H), 3.31-3.29 (m, 4H), 3.06-3.03 (m, 4H).

### Example 108:

### N-[4-(3-{[5-(2-Amino-ethoxy)-benzothiazol-2-ylmethyl]-amino}-[1,2,4]triazin-5-yl)-3,5-dimethyl-phenyl]-N',N'-dimethyl-ethane-1,2-diamine

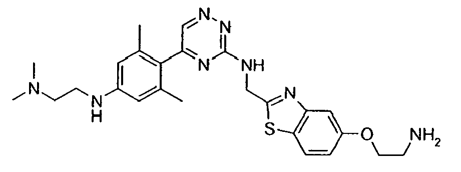

a)
   Under argon, to a solution of (5-methoxy-1,3-benzothiazol-2-yl)methylamine hydrochloride (1.38 g, 6 mmol, prepared as in example 15) and phtalic anhydride (1.01 g, 6.6 mmol) in anhydrous THF (30 mL) cooled in an ice bath, diisopropylethylamine (1.15 mL, 6.6 mmol) was added and the reaction mixture was stirred for 40 minutes, allowing the temperature to raise to room temperature. The solution was then heated at 70°C for 4 hours. The solvent was evaporated, then diethyl ether and aqueous hydrochloric acid 1N were added. The solid was filtered and rinsed with diethyl ether. The filtrate solution was extracted with ethyl acetate, combined organic extracts dried over sodium sulfate, filtered and evaporated. The residue obtained was combined with the solid filtered previously, toluene (40 mL) and a few milligrams of p-toluenesulfonic acid were added and the resulting mixture was stirred at 115°C for 3 hours. The solvent was evaporated, water and ethyl acetate were added. The mixture was extracted with ethyl acetate, combined organic extracts were dried over sodium sulfate, filtered and evaporated. Purification by recrystallsation of the crude product in a mixture of cyclohexane and dichloromethane, and preparative TLC (silica gel, dichloromethane/methanol 95/5) of the mother liquor after concentration afforded 2-[(5-methoxy-1,3-benzothiazol-2-yl)methyl]-1H-isoindole-1,3(2H)-dione (1.338 g, 69%) as a brown solid.
   ESI-MS m/z 325 (M+H)⁺.
b)
   To a solution of 2-[(5-methoxy-1,3-benzothiazol-2-yl)methyl]-1H-isoindole-1,3(2H)-dione (1.338 g, 4.12 mmol) in anhydrous dichloromethane (10 mL) stirred at -78°C under argon, a solution of boron tribromide in dichloromethane (1M, 41 mL, 41 mmol) was added dropwise. The reaction mixture was stirred overnight allowing the temperature to raise slowly to room temperature. The solution was then cooled to -78°C, methanol was added and the temperature let to rise. The mixture was concentrated, an aqueous ammonium chloride solution was added and the solution was extracted with dichloromethane. The combined organic extracts were dried over sodium sulfate, filtered and evaporated. Purification by flash chromatography (silica gel, dichloromethane/methanol, 1/0 to 95/5) afforded 2-[(5-hydroxy-1,3-benzothiazol-2-yl)methyl]-1H-isoindole-1,3(2H)-dione (1.119 g, 87%) as a yellow solid.
   ESI-MS m/z 311 (M+H)⁺.
c)
   Under argon, a solution of diethyl azodicarboxylate (730 µL, 4.69 mmol) in anhydrous THF (10 mL) was slowly added to a solution of 2-[(5-hydroxy-1,3-benzothiazol-2-yl)methyl]-1H-isoindole-1,3(2H)-dione (1.119 g, 3.6 mmol), triphenylphosphine (1.416 g, 3.82 mmol), and tert-butyl 2-hydroxyethylcarbamate (651 mg, 3.96 mmol) in anhydrous THF (20 mL) stirred at 0°C. The reaction was kept stirring for 3 days, allowing the temperature to rise. The solvent was evaporated. Purification by flash chromatography (silica gel, dichloromethane/methanol, 1/0 to 97/3) gave tert-butyl {2-[2-(1,3-dioxo-1,3-dihydro-isoindol-2-ylmethyl)-benzothiazol-5-yloxy]-ethyl}-carbamate contaminated by triphenylphosphine oxide as a brown oil. The latter was dissolved in ethanol (15 mL) in a screwed-cap tube, methyl hydrazine (580 mL, 10.8 mmol) was added and the solution was stirred at 80°C under argon for 1.5 hours. The solvent was evaporated and diethyl ether was added. A solid was filtered off, rinsed with diethyl ether and the filtrate was concentrated. Purification by flash chromatography (silica gel, dichloromethane/methanol, 1/0 to 9/1) afforded tert-butyl 2-{[2-(aminomethyl)-1,3-benzothiazol-5-yl]oxy}ethylcarbamate (720.8 mg, 62%) as an orange oil.
   ESI-MS m/z 324 (M+H)⁺.
   ¹H NMR (DMSO-d₆), δ (ppm): 7.90 (d, J = 8.6 Hz, 1H), 7.42 (d, J = 2.4 Hz, 1H), 7.00 (dd, J = 8.6 Hz and 2.4 Hz, 1H), 4.09 (s, 2H), 4.02 (t, J = 6.6 Hz, 2H), 3.34-3.30 (m, 2H), 1.39 (s, 9H).
d)
   A solution of [4-(3-chloro-[1,2,4]triazin-5-yl)-3,5-dimethyl-phenyl]-carbamic acid tert-butyl ester (160 mg, 0.48 mmol, prepared as in example 73), tert-butyl 2-{[2-(aminomethyl)-1,3-benzothiazol-5-yl]oxy}ethylcarbamate (180 mg, 0.56 mmol) and diisopropylethylamine (84 µL, 0.48 mmol) in anhydrous acetonitrile (2 mL) was stirred under argon at 85°C overnight. An aqueous solution of ammonium chloride was added and the reaction mixture was extracted with ethyl acetate. The combined organic extracts were dried over sodium sulfate, filtered and evaporated. Purification by preparative TLC (silica gel, dichloromethane/methanol 95/5) afforded tert-butyl [4-(3-{[5-(2-tert-butoxycarbonylamino-ethoxy)-benzothiazol-2-ylmethyl]-amino}-[1,2,4]triazin-5-yl)-3,5-dimethyl-phenyl]-carbamate (174.1 mg, 58%) as an orange solid.
   ESI-MS m/z 622 (M+H)⁺.
e)
   A mixture of 37% aqueous hydrochloric acid (1.25 mL) and ethyl acetate (3.75 mL) was added to tert-butyl [4-(3-{[5-(2-tert-butoxycarbonylamino-ethoxy)-benzothiazol-2-ylmethyl]-amino}-[1,2,4]triazin-5-yl)-3,5-dimethyl-phenyl]-carbamate (174.1 mg, 0.28 mmol) cooled to 0°C. The reaction mixture was stirred for 1 hour allowing the temperature to raise to room temperature. A dilute aqueous solution of potassium hydroxyde was added until basic pH was reached. The solution was extracted with ethyl acetate and dichloromethane, combined organic extracts were dried over sodium sulfate, filtered and evaporated. The crude product was dissolved in anhydrous THF (3 mL), triethylamine (60 µL, 0.43 mmol) and di(tert-butyl) dicarbonate (67.2 mg, 0.31 mmol) were added. The resulting mixture was stirred at room temperature overnight. An aqueous solution of ammonium chloride was added and the reaction mixture was extracted with diethyl ether and ethyl acetate. The combined organic extracts were dried over sodium sulfate, filtered and evaporated. Purification by preparative TLC (silica gel, dichloromethane/methanol 93/7) afforded tert-butyl [2-(2-{[5-(4-amino-2,6-dimethyl-phenyl)-[1,2,4]triazin-3-ylamino]-methyl}-benzothiazol-5-yloxy)-ethyl]-carbamate (107 mg, 73%) as a yellow solid.
   ESI-MS m/z 522 (M+H)⁺.
f)
   Under argon, 2-chloro-N,N-dimethylethanamine hydrochloride (59 mg, 0.41 mmol), sodium hydrogencarbonate (34.5 mg, 0.41 mmol) and a catalytic amount of sodium iodide were successively added to a solution of tert-butyl [2-(2-{[5-(4-amino-2,6-dimethyl-phenyl)-[1,2,4]triazin-3-ylamino]-methyl}-benzothiazol-5-yloxy)-ethyl]-carbamate (107 mg, 0.21 mmol) in ethanol (1.65 mL). The reaction mixture was stirred at room temperature for 1 hour, and then at 80°C overnight. The solution was then dried over potassium carbonate, filtered and concentrated. Purification by preparative TLC (silica gel, dichloromethane/methanol 85/15) led to {2-[2-({5-[4-(2-dimethylamino-ethylamino)-2,6-dimethyl-phenyl]-[1,2,4]triazin-3-ylamino}-methyl)-benzothiazol-5-yloxy]-ethyl}-carbamic acid tert-butyl ester. The latter compound was dissolved in methanol (125 µL) and a solution of hydrogen chloride in dioxane (4N, 375 µL, 1.5 mmol) was added. The resulting mixture was stirred at room temperature for 25 minutes. Methanol and dichloromethane were added and the solution was dried over potassium carbonate, filtered and concentrated. The crude product was purified by preparative TLC (silica gel, dichloromethane/methanol/aqueous ammonium hydroxide 33%, 90/10/2) to afford N-[4-(3-{[5-(2-aminoethoxy)-benzothiazol-2-ylmethyl]-amino}-[1,2,4]triazin-5-yl)-3,5-dimethyl-phenyl]-N',N'-dimethyl-ethane-1,2-diamine (13.6 mg, 13%) as a yellow solid.
   ESI-MS m/z 493 (M+H)⁺.
   ¹H NMR (DMSO-d₆), δ (ppm): 8.63 (s, 1H), 7.91 (d, J = 8.8 Hz, 1H), 7.51 (d, J = 2.0 Hz, 1H), 7.07 (dd, J = 8.8 Hz and 2.0 Hz, 1H), 6.32 (br s, 2H), 4.93 (br s, 2H), 4.16-4.13 (m, 2H), 3.12-3.10 (m, 4H), 2.44-2.40 (m, 2H), 2.18 (s, 6H), 1.91 (br s, 6H).

### Example 109: Pharmaceutical compositions

1) A pharmaceutical composition for injection has been prepared as follows:
   - compound of example 9 500 mg
   - aqueous sterile vehicle q.s.p. 10 mL
2) A lyophilized pharmaceutical composition for injection has been prepared as follows:
   - compound of example 3 500 mg
   - erythromycin 1g
   - aqueous sterile excipient q.s.p 5 mL

The two active principles can, if desired, be introduced in two distinct containers.

### Pharmacological study of the compounds of the invention.

### Inhibition of the enzymatic activity of RfaE

### HTS biochemical assays developed to assess RfaE enzymatic activity.

### Assays:

RfaE is a kinase sharing similarities with the ribokinases family. It catalyses an essential step of the biosynthesis of L-ADP-Heptose, namely the phosphorylation of β-heptose-7-phosphate (H7P) into β-heptose-1,7-bisphosphate (H1,7P). RfaE assays as described in the literature are essentially based on direct HLPC detection of the substrates H7P and ATP, and of the products H1,7P and ADP, raising obvious limitations for HTS applications. The assays described below are based either on luminescent ATP detection, or on fluorescent ADP detection. They are easily amenable to miniaturized formats and fast readouts as required by HTS.

### RfaE luminescent assay

The assay buffer "AB" contains 50 mM Hepes pH=7.5, 1 mM MnCl₂, 25 mM KCI, 0.012% Triton-X100 and 1 mM DTT (Dithiothreitol). The following components are added in a white polystyrene Costar plate up to a final volume of 31 µL: 3 µL DMSO, or inhibitor dissolved in DMSO and 28 µL RfaE in AB. After 30min of pre-incubation at room temperature, 29 µL of Substrates mix in AB are added in each well to a final volume of 60 µL. This reaction mixture is then composed of 3 nM RfaE (produced in house from *E.coli*), 0.2 µM β -heptose-7-phosphate (in house synthesis) and 0.2 µM ATP (Sigma) in assay buffer. After 40min of incubation at room temperature, 200 µL of the revelation mix are added to a final volume of 260 µL, including the following constituents at the respective final concentrations: 2 nM luciferase (Sigma), 30 µM D-luciferin (Sigma), 100 µM N-acetylcysteamine (Aldrich). Luminescence intensity is immediately measured on an Analyst-HT (Molecular Devices) and converted into inhibition percentages.

For IC50 determinations, the inhibitor is tested at 6 to 10 different concentrations, and the related inhibitions are fitted to a classical langmuir equilibrium model using XLFIT (IDBS).

### Example 110:

The IC50 values in µM of preferred compounds are given in Table 1 below:

**Table 1.**

| Example | IC50 RfaE (µM) | | Example | IC50 RfaE (µM) | | Example | IC50 RfaE (µM) |
|---|---|---|---|---|---|---|---|
| 9 | 4.3 | | 29 | 0.051 | | 78 | 0.082 |
| 15 | 0.13 | | 32 | 0.063 | | 90 | 2.0 |
| 16 | 0.018 | | 33 | 0.020 | | 94 | 0.25 |
| 17 | 0.019 | | 35 | 0.069 | | 97 | 0.053 |
| 18 | 0.047 | | 43 | 0.41 | | 98 | 0.049 |
| 19 | 0.165 | | 57 | 1.9 | | 99 | 0.15 |
| 20 | 0.077 | | 64 | 5.6 | | 100 | 0.255 |
| 21 | 0.067 | | 70 | 3.1 | | 107 | 0.67 |
| 22 | 0.13 | | 75 | 0.19 | | 108 | 1.4 |

### Bacterial sensitization to complement mediated killing

As outlined above, it has been demonstrated that the inner core LPS is necessary to protect *Escherichia coli* from complement killing in vitro. The inhibition of bacterial RfaE by some of the compounds described herein should therefore sensitize the treated bacteria to complement killing.

### Sensitization to complement killing assay

The Gram-negative bacteria *E. coli* K12 AcrAB-/TolC- (K12 strain for which efflux pump components AcrA, AcrB and TolC have been deleted) is grown on Müller Hinton agar (MHA) overnight at 35°C. Five well isolated colonies from overnight agar growth are then added to 10 mL of cation adjusted Müller-Hinton Broth (MH2) and incubated at 35°C with constant shaking for two hours to reach the exponential phase. The suspension is then diluted to obtain an inoculum of 1.5E+04 CFU/mL. Viable counts are obtained by plating 10 to 1000-fold dilutions of the suspension on MHA. A solution of decomplemented Foetal Bovine Serum (dFBS) is prepared by heating normal FBS at 56°C for 30min. Compound dilutions are prepared in DMSO/water 20/80 in polypropylene plates. The following components are added on a polystyrene sterile culture plate in a final volume of 100µL:
5µL of compound in 20% DMSO,
30µL of FBS (30% final) or dFBS (30% final) or MH2 (0% FBS),
65µL of bacterial suspension at 1.5e4 CFU/mL (final inoculum of 1e4 CFU/mL). After 18-22H at 35°C incubation, the MIC of the compound in the presence of FBS 30%, dFBS 30% or FBS 0% is determined by visual inspection of bacterial pellets.

### Example 111:

The MIC of preferred compound of example **18** has been measured in absence/presence of 30% decomplemented FBS, and in the presence of 30% normal (complemented) FBS. Values in µg/mL are given in Table 2 below:

| | MIC (µg/mL) for E. coli K12 AcrAB- TolC- | | |
|---|---|---|---|
| | FBS=0 | decomplemeted FBS=30% | normal FBS=30% |
| example 18 | >32 | >32 | 4 |

As awaited, this compound alone or in the presence of decomplemented serum is not inhibiting the growth of our efflux pump deleted E. coli strain. On the other hand, no bacterial growth is observed when bacteria are treated with 4µg/mL of compound 18 in the presence of 30% normal serum. Compound 18 at 4µg/mL has inhibited inner core LPS synthesis by inhibiting bacterial RfaE, thus inducing bacterial sensitization to complement killing.

## Claims

**1.** The compounds having the general formula (I) 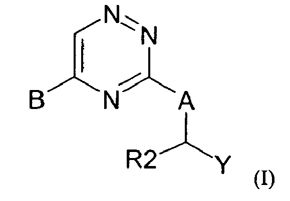 wherein,
- A is NR1, O or S;
R1 is H or (C₁-C₆) alkyl optionally substituted by one groups R'1, or R1 and R2 form with N a 4 to 6 membered saturated heterocycle;
R'1 is CH₂OH, CO₂H, CO₂ (C₁-C₄) alkyl, CH₂NH₂, SO₃H or PO₃H₂
- R2 is H or (C₁-C₆) alkyl optionally substituted by one or several identical or different R, or R1 and R2 form with N a 4 to 6 membered saturated heterocycle;
- Y is a 5-10 membered mono or bicyclic, unsaturated or aromatic heterocycle containing1-4 heteroatoms selected from N, O and S, optionally substituted by one or several identical or different groups R;
- B is (C₁-C₆) alkyl or mono or bicyclic aryl, all being optionally substituted by one or several identical or different groups R;
R is selected from the group consisting of (C₁-C₆) alkyl, (C₂-C₆) alkenyl, (C₂-C₆) alkynyl, phenyl, 4-6 membered monocyclic saturated or unsaturated heterocycle containing 1-3 heteroatoms selected from N, O and S, CO₂Rₐ, CORₐ, CONRₐR_{b}, OCORₐ, ORₐ, NRₐR_{b}, CRₐ=NOR_{b}, NRₐCOR_{b}, NRₐCOOR_{b}, OCONRₐR_{b}, NRₐCONR_{b}R_{c}, NRₐSO₂R_{b}, S(O)ₙRₐ, and SO₂NRₐR_{b}, all the above members of the group representing R being optionally substituted by one or several identical or different groups R', or R is halogen, CN or NO₂;
Rₐ, R_{b} and R_{c}, identical or different, are selected from the group consisting of H, (C₁-C₆) alkyl, (C₂-C₆) alkenyl, (C₂-C₆) alkynyl, phenyl and 4-6 membered monocyclic saturated or unsaturated heterocycle containing 1-3 heteroatoms selected from N, O and S, all the above members of the group representing Rₐ, R_{b} and R_{c} being optionally substituted by one or different groups R',
R' is selected from the group consisting of (C₁-C₆) alkyl, (C₁-C₆) alkyl substituted by hydroxy, 4-6 membered monocyclic saturated or unsaturated heterocycle containing 1-3 heteroatoms selected from N, O and S, CO₂R", COR", CONR"R"', OCOR", OR", NR"R"', NR"COR"', NR"COOR"', CONR"-(C₁-C₃)alkyl-OH, OCONR"R"', NR"CONR"'R"", NR"SO₂R"', S(O)ₙR", SO₂ NR"R"', halogen, CN, NR"-CO(C₁-C₃) alkyl-NR"'R"", NR"-CO(C₁-C₃) alkyl-heterocycle, NR"-CO(C₁-C₃) alkyl-OH, NR"-CO(C₁-C₃) alkyl-CONH₂, -CONR"-(C₁-C₃) alkyl-NR"'R"" -CONR"-(C₁-C₃) alkyl-CONH₂, -CO-NR"-(C₁-C₃) alkyl-heterocycle, the heterocyle being selected from the group consisting of piperidinyl, imidazolyl, morpholinyl, piperazinyl, pyrrolidinyl and azetidinyl;
R", R"' and R"" being identical or different are H or (C₁-C₅) alkyl or form together a 4 to 6 membered nitrogenous saturated heterocycle;
n is 0, 1 or 2;
their N-oxide derivatives,
in their racemic, enantiomeric or geometric forms,
and their addition salts thereof with acids and bases.

**2.** The compounds of general formula (I), according to claim 1, wherein B represents phenyl substituted at position 2 by a group R, or 1-naphthyl optionally substituted by R, R being as defined in claim 1.

**3.** The compounds of general formula (I), according to claim 1 or 2,
wherein B represents phenyl substituted at positions 2,5, 2,6 and 2,4,6 by identical or different groups R, R being as defined in claim 1.

**4.** The compounds of general formula (I), according to any of claims 1 to 3, wherein B represents phenyl substituted at positions 2,6 by identical or different groups R, R being selected from the group consisting of Me, halogen, NH₂, OH and OMe.

**5.** The compounds of general formula (I), according to any of claims 1 to 4, wherein the heterocycle represented by Y is selected from the group consisting of the compounds of general formula (I) wherein the heterocycle represented by Y is selected from the group consisting of oxazolyl, oxazolinyl, benzoxazolyl, tetrahydrobenzoxazolyl, oxazolopyridinyl, tetrahydrooxazolopyridinyl, oxazolopyrimidinyl, tetrahydrooxazolopyrimidinyl, oxazolopyrazinyl, oxazolopyridazinyl, oxazolotriazinyl, thiazolyl, thiazolinyl, benzothiazolyl, tetrahydrobenzothiazolyl, thiazolopyridinyl, tetrahydrothiazolopyridinyl, thiazolopyrimidinyl, tetrahydrothiazolopyrimidinyl, thiazolopyrazinyl, thiazolopyridazinyl, thiazolotriazinyl, pyrazolyl, pyrazolopyridinyl, tetrahydropyrazolopyridnyl, pyrazolopyrimidinyl, pyrazolopyrazinyl, pyrazolotriazinyl, triazolopyridinyl, tetrahydrotriazolopyridinyl, triazolopyrimidinyl, triazolopyrazinyl and triazolotriazinyl.

**6.** The compounds of general formula (I), according to any of claims 1 to 5, wherein Y represents a benzothiazole group, a tetrahydrobenzothiazole group, or a thiazolopyridine group, optionally substituted at position 5 by a group R, R being as defined in claim 1.

**7.** The compounds of general formula (I), according to any of claims I to 5, wherein Y represents a benzothiazole group, a tetrahydrobenzothiazole group, or a thiazolopyridine group, optionally substituted at position 5 by a group ORₐ, Rₐ being as defined in claim 1.

**8.** The compounds of general formula (I), according to any of claims 1 to 5, wherein Y represents a thiazole group, optionally substituted at position 4 and/or 5 by one or two groups R, R being as defined in claim 1.

**9.** The compounds of general formula (I), according to any of claims 1 to 8, wherein A is NH or N-CH₃.

**10.** The compounds of general formula (I), according to any of claims 1 to 9, wherein R2 is H.

**11.** The compounds of general formula (I), according to claim 1, the names of which follows:
-5-(2-Chlorophenyl)-N-[(4,5-dimethyl-1,3-thiazol-2-yl)methyl]-1,2,4-triazin-3-amine,
-5-(2,6-Dichlorophenyl)-N-[(5-methoxy-1,3-benzothiazol-2-yl)methyl]-1,2,4-triazin-3-amine,
-2-{[2-({[5-(2,6-Dichlorophenyl)-1,2,4-triazin-3-yl]amino}methyl)-1,3-benzothiazol-5-yl]oxy} ethanol,
-{[2-({[5-(2,6-dichlorophenyl)-1,2,4-triazin-3-yl]amino}methyl)-1,3-benzothiazol-5-yl]oxyl acetic acid,
-2-(2-{[5-(2,6-Dichloro-phenyl)-[1,2,4]triazin-3-ylamino]-methyl}benzothiazol-5-yloxy)-N-(2-hydroxy-ethyl)-acetamide,
-2-(2-{[5-(2,6-Dichloro-phenyl)-[1,2,4]triazin-3-ylamino]-methyl}benzothiazol-5-yloxy)-N-(2-dimethylamino-ethyl)-acetamide,
-1-{[2-({[5-(2,6-Dichlorophenyl)-1,2,4-triazin-3-yl]amino}methyl)-1,3-benzothiazol-5-yl]oxy} acetone,
-2-{[2-({[5-(2,6-Dichlorophenyl)-1,2,4-triazin-3-yl]amino}methyl)-1,3-benzothiazol-5-yl]oxy}acetamide,
-Benzothiazol-2-ylmethyl-[5-(2-methoxy-naphthalen-1-yl)-[1,2,4]triazin-3-yl]-amine,
-N-[(5-Methoxy-1,3-benzothiazol-2-yl)methyl]-5-(2-methoxy-1-naphthyl)-1,2,4-triazin-3-amine,
-1-{3-[(Benzothiazol-2-ylmethyl)-amino]-[1,2,4]triazin-5-yl}-naphthalen-2-ol,
-2-{[5-(2-Hydroxy-naphthalen-1-yl)-[1,2,4]triazin-3-ylamino]-methyl}benzothiazol-5-ol,
-2-(3-{[(5-Methoxy-1,3-benzothiazol-2-yl)methyl]amino}-1,2,4-triazin-5-yl)-3-methylphenol,
-3-Methyl-2-{3-[({5-[(1,3-thiazol-2-ylmethyl)amino]-1,3-benzothiazol-2-yl}methyl)amino]-1,2,4-triazin-5-yl}phenol,
-5-(2,6-Dimethoxyphenyl)-N-(4,5,6,7-tetrahydro-1,3-benzothiazol-2-ylmethyl)-1,2,4-triazin-3-amine,
-2-[({5-[2-(Trifluoromethyl)phenyl]-1,2,4-triazin-3-yl}amino)methyl]-1,3-benzothiazol-4-ol,
-5-(2-Methoxy-6-methylphenyl)-N-([1,3]thiazolo[5,4-b]pyridin-2-ylmethyl)-1,2,4-triazin-3-amine,
-2-Dimethylamino-N-(4-{3-[(5-methoxy-benzothiazol-2-ylmethyl)-amino]-[1,2,4]triazin-5-yl}-3,5-dimethyl-phenyl)-acetamide,
-N,N-Dimethyl-N'-[4-(3-{[(5-methoxy-1,3-benzothiazol-2-yl)methyl]amino}-1,2,4-triazin-5-yl)phenyl]ethane-1,2-diamine,
-{(1,3-Benzothiazol-2-ylmethyl)[5-(2,6-dimethoxyphenyl)-1,2,4-triazin-3-yl]amino}acetic acid,
-N-({5-[(2-Aminoethyl)thio]-1,3-benzothiazol-2-yl}methyl)-5-(2,6-dichlorophenyl)-1,2,4-triazin-3-amine,
-5-[2-Chloro-5-(1H-pyrazol-3-yl)phenyl]-N-[(5-methoxy-1,3-benzothiazol-2-yl)methyl]-1,2,4-triazin-3-amine,
-5-[2-Chloro-5-(1H-pyrazol-4-yl)phenyl]-N-[(5-methoxy-1,3-benzothiazol-2-yl)methyl]-1,2,4-triazin-3-amine,
-5-(2-Chloro-5-pyridin-3-ylphenyl)-N-[(5-methoxy-1,3-benzothiazol-2-yl)methyl]-1,2,4-triazin-3-amine,
-2-[4-(4-Chloro-3- {3-[(5-methoxy-benzothiazol-2-ylmethyl)-amino]-[1,2,4]triazin-5-yl}-phenyl)-[1,2,3]triazol-1-yl]-ethanol,
-N-[(5-Methoxy-1,3-benzothiazol-2-yl)methyl]-5-(2-chloro-4-piperazin-1-ylphenyl)-1,2,4-triazin-3-amine, and
-N-[4-(3-{[5-(2-Amino-ethoxy)-benzothiazol-2-ylmethyl]-amino}-[1,2,4]triazin-5-yl)-3,5-dimethyl-phenyl]-N',N'-dimethyl-ethane-1,2-diamine, and their salts.

**12.** Process for preparing the compounds of formula (I) as defined in claim 1, which comprises:
a) reacting a compound of formula (II): 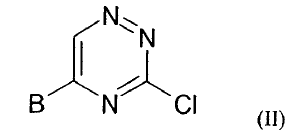 wherein B has values defined in claim 1, with a compound of formula (III):
H-A-CH(R2)-Y (III)
wherein A, R2 and Y have the values defined in claim 1, in the presence of a base,
b) if appropriate, the reaction being followed or preceded by one or more of the following reactions, performed in an appropriate order, to achieve the substitutions on B and/or Y and/or R1 and/or R2 defined above:
protection of reactive functions,
deprotection of reactive functions,
halogenation,
dehalogenation,
dealkylation,
alkylation of amine, aniline, alcohol and phenol,
reduction of nitro, esters, cyano, aldehydes,
transition metal-catalyzed reactions,
acylation,
sulfonylation/introduction of sulfonyl groups,
saponification/hydrolysis of esters groups,
halogen exchange,
nucleophilic substitution with amine, thiol or alcohol,
reductive amination,
fixation of R groups on B and/or Y,
N-oxidation,
salification.

**13.** Process for preparing the compounds of formula (I) as defined in claim 1, which comprises:
a) reacting a compound of formula (IV): 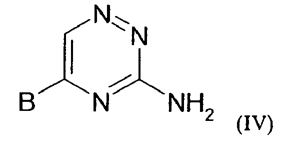 wherein B has values defined in claim 1, with a compound of formula (V) :
R2-C(O)-Y (V)
wherein Y and R2 have the values defined in claim 1, in the presence of a reducing agent,
b) if appropriate, the reaction being followed or preceded by one or more of the reactions defined in claim 12, performed in an appropriate order, to achieve the substitutions on B and/or Y and/or R1 and/or R2 defined in claim 1.

**14.** Process for preparing the compounds of formula (I) as defined in claim 1, wherein A represents NH, which comprises:
a) reacting a compound of formula (VI): 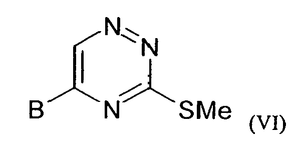
wherein B has values defined in claim 1, with a compound of formula (VII):
H₂N-CH(R2)-Y (VII)
wherein R2 and Y have the values defined in claim 1, in the presence of an oxidizing agent and a base,
if appropriate, the reaction being followed or preceded by one or more of the reactions defined in claim 12, performed in an appropriate order, to achieve the substitutions on B and/or Y and/or R1 and/or R2 defined in claim 1.

**15.** Process for preparing the compounds of formula (II) as defined in claim 12, which comprises reacting a compound of formula (IV) as defined in claim 13 with a chlorination agent.

**16.** Process for preparing the compounds of formula (II) as defined in claim 12, which comprises reacting a compound of formula (VIII):
B-CO-CHO (VIII)
wherein B has values defined in claim 1, with semicarbazide of formula (IX):
H₂N-CO-NH-NH₂ (IX)
to obtain a compound of formula (X): 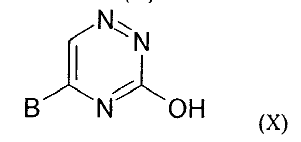 wherein B has values defined in claim 1, which is submitted to the action of a chlorinating agent.

**17.** Process for preparing the compounds of formulae (II) and (IV) as defined in claim 12 and 13, which comprises reacting a compound of formula (VIII):
B-CO-CHO (VIII)
wherein B has values defined in claim 1, with aminoguanidine of formula (XI):
H₂N-C(NH)-NH-NH₂ (XI)
to obtain a compound of formula (IV), which is, if desired, either submitted to the action of an alkali metal hydroxide to obtain a compound of formula (X), as defined in claim 16, which is submitted to the action of a chlorination agent, or directly submitted to the action of a chlorination agent.

**18.** Process for preparing the compounds of formula (IV) as defined in claim 13, which comprises reacting a compound of formula (XIII):
B-CO-CBr₂ (XIII)
with morpholine to obtain a compound of formula (XII):
B-CO-C(morpholine)₂ (XII)
wherein B has values defined in claim 1, in an acidic medium, and reacting the compound of formula (XII) with aminoguanidine of formula (XI) as defined in claim 17.

**19.** The compounds of formulae (II), (IV), (VI), (X), (VIII), (XII) and (XIII) as defined respectively in claims 12, 13, 14, 16 and 18.

**20.** The compounds of formula (III) as defined in claim 12, wherein Y is substituted by one or several identical or different groups R, Y and R being as defined in claim 1.

**21.** Use as drugs of the compounds of formula (I) as defined in claim 1, as well as their salts with pharmaceutically acceptable acids and bases.

**22.** Use as drugs of the compounds of formula (I) as defined in any of claims 2 to 11, as well as their salts with pharmaceutically acceptable acids and bases.

**23.** Pharmaceutical compositions comprising an effective amount of at least one drug according to claim 21 or 22, and, if desired, an appropriate pharmaceutically acceptable carrier.

**23.** Associations of the drugs according to claim 21 or 22, with antimicrobial peptides or natural, hemisynthetic or synthetic antibacterial molecules.

**24.** Pharmaceutical compositions comprising an effective amount of at least one association of drugs according to claim 24, and, if desired, an appropriate pharmaceutically acceptable carrier.
